# EUROPEAN PATENT APPLICATION

(11) **EP 3 819 312 A1**
(43) Date of publication of application: **12.05.2021**
(21) Application number: 19208214.7
(22) Date of filing: 10.11.2019
(51) Int. Cl.: C07K 16/28, C07K 16/30, A61P 35/00

(54) **DOSING REGIMEN FOR ANTI-DLL3 AGENTS**

(71) Applicant: Amgen, Inc, Thousand Oaks, CA 91320-1799 (US)
(72) Inventor: SMIT, Marie-Anne Damiette, Thousand Oaks, CA 91320-1799 (US); SOMAN, Neelesh, Thousand Oaks, CA 91320-1799 (US); UPRETI, Vijay Vishesh, Thousand Oaks, CA 91320-1799 (US); MINOCHA, Mukul, Thousand Oaks, CA 91320-1799 (US); YAGO, Marc Anthony, San Francisco, CA 94112 (US); LIAO, Michael, Thousand Oaks, CA 91320-1799 (US)
(74) Representative: Schiweck Weinzierl Koch Patentanwälte Partnerschaft mbB

(57) **Abstract**

The present invention provides a method for the treatment of DLL3-positive cancer or SCLC, comprising administering to a subject in need thereof an anti-DLL3 agent at a dose of between about 0.3 mg to about 100 mg once every two weeks. Step dosing of the anti-DLL3 agent for the treatment of SCLC is also disclosed.

## Description

### FIELD OF THE INVENTION

The present application relates to dosage and administration of anti-DLL3 agents for the treatment of cancer.

### BACKGROUND OF THE INVENTION

Small cell lung cancer (SCLC) is an aggressive form of lung cancer with a poor prognosis and limited therapeutic options and represents about 10-15% of lung cancers. Survival rates have remained low for several decades, with only 5% of SCLC patients surviving five years, in a large part due to the lack of new therapies to combat this form of lung cancer. About a third of patients present with limited stage disease. Most patients present with extensive-stage disease, defined by the presence of tumors in only one side of the chest and that fit in a single radiation field. These stages impact available therapeutic regiments, with limited stage disease treated with chemotherapy and radiation and extensive stage disease treated with chemotherapy alone. Disseminated, metastatic tumors with lymphoma-like characteristics are a hallmark of SCLC.

Patients typically respond well to the current front-line therapy, which includes etoposide and cisplatin, but invariably quickly relapse with chemoresistant disease, for which no therapeutic options are currently available. Prognosis in the relapsed refractory setting is extremely poor, with rapid disease progression and short median survival of less than six months. Furthermore, SCLC patients have high rates of comorbidities, including hypertension, cardiac disease, diabetes and paraneoplastic syndromes. These, coupled with the typically advanced age of SCLC patients, impact the ability of patients to endure harsh chemo regimens, further limiting treatment options.

Delta-like 3 (DLL3) is a type 1 transmembrane protein and noncanonical Notch ligand that is differentially expressed in SCLC. Using immunohistochemistry (IHC), 85% of SCLC tumors stained positive for DLL3 in a pattern consistent with both membranous and cytoplasmic expression. In contrast, low levels of DLL3 protein expression were detected in normal brain, pancreatic islets, and pituitary gland with a cytoplasmic staining pattern (Saunders et al, Sci Transl Med. 7:302ra136 (2015)). DLL3 is a novel and promising target for the development of T-cell-targeted therapies for SCLC.

There is an unmet medical need for the development of therapies for the treatment of SCLC.

### SUMMARY OF THE INVENTION

Based on the disclosure provided herein, those skilled in the art will recognize, or be able to ascertain using no more than routine experimentation, many equivalents to the specific embodiments of the invention described herein. Such equivalents are intended to be encompassed by the following embodiments (E).
E1: A method of treating DLL3-positive cancer, comprising administering to a subject in need thereof an anti-DLL3 agent, wherein the anti-DLL3 agent is administered at a dose of from 0.3 mg to 100 mg once every two weeks.
E2: A method of treating DLL3-positive cancer, comprising administering to a subject in need thereof an anti-DLL3 agent, wherein said anti-DLL3 agent is administered according to the following schedule: a) a first dose of from 0.3 mg to 100 mg on day 1, b) a second dose of from 0.3 mg to 100 mg on day 8, and c) one or more subsequence doses of from 0.3 mg to 100 mg, starting on day 15 and once every two weeks thereafter, and wherein the second and subsequent doses are the same, and are higher than the first dose.
E3: The method of E1 or E2, wherein the anti-DLL3 positive cancer is SCLC.
E4: The method of any one of E1-E3, wherein the anti-DLL3 positive cancer is RR SCLC or ED SCLC.
E5: The method of any one of E1-E4, wherein the anti-DLL3 agent is a bispecific antibody construct comprising two binding domains: the first domain binds to human DLL3, and the second domain binds to human CD3.
E6: The method of E5, wherein the DLL3-binding domain binds to an epitope of human DLL3 comprised within the amino acid sequence of SEQ ID NO: 285.
E7: The method of E5 or E6, wherein the DLL3-binding domain comprises (a) a heavy chain variable region (VH) that comprises: (i) a VH complementarity determining region one (CDR-H1) comprising the amino acid sequence of SEQ ID NO:31; (ii) a CDR-H2 comprising the amino acid sequence of SEQ ID NO:32; and (iii) a CDR-H3 comprising the amino acid sequence of SEQ ID NO:33; and (b) a light chain variable region (VL) that comprises: (i) a VL complementarity determining region one (CDR-L1) comprising the amino acid sequence of SEQ ID NO:34; (ii) a CDR-L2 comprising the amino acid sequence of SEQ ID NO:35; and (iii) a CDR-L3 comprising the amino acid sequence of SEQ ID NO:36.
E8: The method of any one of E5-E7, wherein the DLL3-binding domain comprises: (1) a VH that comprises the amino acid sequence of SEQ ID NO:37, and a VL that comprises the amino acid sequence of SEQ ID NO:38, or (2) a VH that comprises the amino acid sequence of SEQ ID NO:435, and a VL that comprises the amino acid sequence of SEQ ID NO:436.
E9: The method of any one of E5-E8, wherein the VH and VL of the DLL3-binding domain are joined by a linker to form a single chain Fv (scFv).
E10: The method of E9, wherein the linker comprises a sequence selected from any one of SEQ ID NOs: 285-293.
E11: The method of E9 or E10, wherein the linker comprises (Gly4Ser)x, where x is an integer of 1 or greater (e.g. 1, 2, 3 or 4).
E12: The method of any one of E5-E11, wherein the DLL3-binding domain comprises the amino acid sequence of SEQ ID NO: 39 or SEQ ID NO: 347.
E13: The method of any one of E5-E12, wherein the CD3-binding domain comprises: (a) a VH that comprises: a CDR-H1 comprising the amino acid sequence of SEQ ID NO:426, a CDR-H2 comprising the amino acid sequence of SEQ ID NO:427, and a CDR-H3 comprising the amino acid sequence of SEQ ID NO:428; and a VL that comprises: a CDR-L1 comprising the amino acid sequence of SEQ ID NO:423, a CDR-L2 comprising the amino acid sequence of SEQ ID NO:424, and a CDR-L3 comprising the amino acid sequence of SEQ ID NO:425.
E14: The method of any one of E4-E13, wherein the CD3-binding domain comprises: a VH that comprises the amino acid sequence of SEQ ID NO:429, and a VL that comprises the amino acid sequence of SEQ ID NO:430.
E15: The method of E13 or E14, wherein the VH and VL of the CD3-binding domain are joined by a linker to form a single chain Fv (scFv).
E16: The method of E15, wherein the linker comprises a sequence selected from any one of SEQ ID NOs: 285-293.
E17: The method of E15 or E16, wherein the linker comprises (Gly4Ser)x, where x is an integer of 1 or greater (e.g. 1, 2, 3 or 4).
E18: The method of any one of E13-E17, wherein the CD3-binding domain comprises the amino acid sequence of SEQ ID NO: 431.
E19: The method of any one of E5-E18, wherein the DLL3-binding domain and the CD3-binding domain are joined by a linker.
E20: The method of E19, wherein the linker is a peptide linker comprising a sequence selected from any one of SEQ ID NOs: 285-293.
E21: The method of E19 or E20, wherein the linker is a peptide linker comprises (Gly4Ser)x, where x is an integer of 1 or greater (e.g., 1, 2, 3 or 4).
E22: The method of any one of E5-E21, the anti-DLL3 agent comprises a DLL3-binding domain and a CD3-binding domain. The DLL3-binding domain comprises (a) a heavy chain variable region (VH) that comprises: (i) a VH complementarity determining region one (CDR-H1) comprising the amino acid sequence of SEQ ID NO:31; (ii) a CDR-H2 comprising the amino acid sequence of SEQ ID NO:32; and (iii) a CDR-H3 comprising the amino acid sequence of SEQ ID NO:33; and (b) a light chain variable region (VL) that comprises: (i) a VL complementarity determining region one (CDR-L1) comprising the amino acid sequence of SEQ ID NO:34; (ii) a CDR-L2 comprising the amino acid sequence of SEQ ID NO:35; and (iii) a CDR-L3 comprising the amino acid sequence of SEQ ID NO:36. The CD3-binding domain comprises (a) a VH that comprises: (i) a CDR-H1 comprising the amino acid sequence of SEQ ID NO:426, (ii) a CDR-H2 comprising the amino acid sequence of SEQ ID NO:427, and (iii) a CDR-H3 comprising the amino acid sequence of SEQ ID NO:428; and (b) a VL that comprises: (i) a CDR-L1 comprising the amino acid sequence of SEQ ID NO:423, (ii) a CDR-L2 comprising the amino acid sequence of SEQ ID NO:424, and (iii) a CDR-L3 comprising the amino acid sequence of SEQ ID NO:425.
E23: The method of any one of E5-E22, the DLL3-binding domain comprises a VH that comprises the amino acid sequence of SEQ ID NO:37, and a VL that comprises the amino acid sequence of SEQ ID NO:38, and the CD3-binding domain comprises a VH that comprises the amino acid sequence of SEQ ID NO:429, and a VL that comprises the amino acid sequence of SEQ ID NO:430.
E24: The method of any one of E5-E22, the DLL3-binding domain comprises a VH that comprises the amino acid sequence of SEQ ID NO:435, and a VL that comprises the amino acid sequence of SEQ ID NO:436, and the CD3-binding domain comprises a VH that comprises the amino acid sequence of SEQ ID NO:429, and a VL that comprises the amino acid sequence of SEQ ID NO:430.
E25: The method of any one of E5-E23, wherein the DLL3-binding domain comprises the amino acid of SEQ ID NO: 39 and the CD3-binding domain comprises the amino acid of SEQ ID NO: 431.
E26: The method of any one of E5-E22 or E24, the DLL3-binding domain comprises the amino acid of SEQ ID NO: 437 and the CD3-binding domain comprises the amino acid of SEQ ID NO: 431.
E27: The method of E25, wherein the anti-DLL3 agent comprises the amino acid sequence of SEQ ID NO: 40.
E28: The method of E26, wherein the anti-DLL3 agent comprises the amino acid sequence of SEQ ID NO: 438.
E29: The method of any one of E5-E28, wherein the anti-DLL3 agent further comprises a third domain that extends or enhance the serum half-life of the anti-DLL3 agent.
E30: The method of E29, wherein the third domain comprises the amino acid sequence selected from any one of SEQ ID NOs: 541-548.
E31: The method of any one of E5-E22, E24, E26, E28, E29 or E30, wherein the anti-DLL3 agent comprises the amino acid of SEQ ID NO: 520.
E32: The method of any one of E1 or E3-E31, wherein the anti-DLL3 agent is administered once every two weeks at a dose of: from about 0.3 mg to about 90 mg, from about 0.3 mg to about 80 mg, from about 0.3 mg to about 70 mg, from about 0.3 mg to about 60 mg, from about 0.3 mg to about 50 mg, from about 0.3 mg to about 40 mg, from about 0.3 mg to about 30 mg, from about 0.3 mg to about 20 mg, from about 0.3 mg to about 10 mg, from about 0.3 mg to about 3 mg, from about 0.3 mg to about 1 mg, from about 1 mg to about 100 mg, from about 1 mg to about 90 mg, from about 1 mg to about 80 mg, from about 1 mg to about 70 mg, from about 1 mg to about 60 mg, from about 1 mg to about 50 mg, from about 1 mg to about 40 mg, from about 1 mg to about 30 mg, from about 1 mg to about 20 mg, from about 1 mg to about 10 mg, from about 1 mg to about 3 mg, from about 3 mg to about 100 mg, from about 3 mg to about 90 mg, from about 3 mg to about 80 mg, from about 3 mg to about 70 mg, from about 3 mg to about 60 mg, from about 3 mg to about 50 mg, from about 3 mg to about 40 mg, from about 3 mg to about 30 mg, from about 3 mg to about 20 mg, from about 3 mg to about 10 mg, from about 3 mg to about 12 mg, from about 3 mg to about 15 mg, from about 10 mg to about 100 mg, from about 10 mg to about 90 mg, from about 10 mg to about 80 mg, from about 10 mg to about 70 mg, from about 10 mg to about 60 mg, from about 10 mg to about 50 mg, from about 10 mg to about 40 mg, from about 10 mg to about 30 mg, from about 10 mg to about 20 mg, from about 10 mg to about 15 mg, from about 20 mg to about 100 mg, from about 20 mg to about 90 mg, from about 20 mg to about 80 mg, from about 20 mg to about 70 mg, from about 20 mg to about 60 mg, from about 20 mg to about 50 mg, from about 20 mg to about 40 mg, from about 20 mg to about 30 mg, from about 30 mg to about 100 mg, from about 30 mg to about 90 mg, from about 30 mg to about 80 mg, from about 30 mg to about 70 mg, from about 30 mg to about 60 mg, from about 30 mg to about 50 mg, or from about 30 mg to about 40 mg.
E33: The method of any one of E1 or E3-E32, wherein the anti-DLL3 agent is administered on day 1 and day 15 of a 28-day cycle.
E34: The method of any one of E2-E31, wherein the second and the one or more subsequent doses are the same and are at least 1.5-fold, at least 2-fold, at least 3-fold, at least 4-fold, at least 5-fold, at least 6-fold, at least 7-fold, at least 8-fold, at least 9-fold, at least 10-fold, at least 20-fold, at least 25-fold, at least 30-fold, at least 35-fold, at least 40-fold, at least 45-fold, at least 50-fold, at least 55-fold, at least 60-fold, at least 65-fold, at least 70-fold, at least 75-fold, at least 80-fold, at least 85-fold, at least 90-fold, at least 95-fold, or at least 100-fold higher than the first dose.
E35: The method of any one of E2-E31 or E34, wherein each of the first, second and subsequent doses of the anti-DLL3 agent can be any one of the following: from about 0.3 mg to about 90 mg, from about 0.3 mg to about 80 mg, from about 0.3 mg to about 70 mg, from about 0.3 mg to about 60 mg, from about 0.3 mg to about 50 mg, from about 0.3 mg to about 40 mg, from about 0.3 mg to about 30 mg, from about 0.3 mg to about 20 mg, from about 0.3 mg to about 10 mg, from about 0.3 mg to about 3 mg, from about 0.3 mg to about 1 mg, from about 1 mg to about 100 mg, from about 1 mg to about 90 mg, from about 1 mg to about 80 mg, from about 1 mg to about 70 mg, from about 1 mg to about 60 mg, from about 1 mg to about 50 mg, from about 1 mg to about 40 mg, from about 1 mg to about 30 mg, from about 1 mg to about 20 mg, from about 1 mg to about 10 mg, from about 1 mg to about 3 mg, from about 3 mg to about 100 mg, from about 3 mg to about 90 mg, from about 3 mg to about 80 mg, from about 3 mg to about 70 mg, from about 3 mg to about 60 mg, from about 3 mg to about 50 mg, from about 3 mg to about 40 mg, from about 3 mg to about 30 mg, from about 3 mg to about 20 mg, from about 3 mg to about 10 mg, from about 3 mg to about 12 mg, from about 3 mg to about 15 mg, from about 10 mg to about 100 mg, from about 10 mg to about 90 mg, from about 10 mg to about 80 mg, from about 10 mg to about 70 mg, from about 10 mg to about 60 mg, from about 10 mg to about 50 mg, from about 10 mg to about 40 mg, from about 10 mg to about 30 mg, from about 10 mg to about 20 mg, from about 10 mg to about 15 mg, from about 20 mg to about 100 mg, from about 20 mg to about 90 mg, from about 20 mg to about 80 mg, from about 20 mg to about 70 mg, from about 20 mg to about 60 mg, from about 20 mg to about 50 mg, from about 20 mg to about 40 mg, from about 20 mg to about 30 mg, from about 30 mg to about 100 mg, from about 30 mg to about 90 mg, from about 30 mg to about 80 mg, from about 30 mg to about 70 mg, from about 30 mg to about 60 mg, from about 30 mg to about 50 mg, and from about 30 mg to about 40 mg.
E36: The method of any one of E1-E35, wherein the method comprises administering an anti-inflammatory agent to the subject.
E37: The method of E36, wherein the one or more additional therapeutic agents is a steroid.
E38: The method of any one of E36 or E37, wherein the additional therapeutic agent is dexamethasone.
E39: The method of any one of E36-E38, wherein the anti-inflammatory agent is administered prior to the treatment with the anti-DLL3 agent.
E40: The method of any one of E36-E38, wherein the anti-inflammatory agent is administered concurrently with the anti-DLL3 agent.
E41: The method of any one of E1-E40, wherein the subject is a human.
E42: An anti-DLL3 agent for use in a method as set forth in any one of embodiments E1-E41.
E43: Use of an anti-DLL3 agent as set forth in E42 in the preparation of a medicament for the treatment of SCLC.
E44: Use of an anti-DLL3 agent as set forth in E42 in the preparation of a medicament for the treatment of an DLL3-positive cancer.

### BRIEF DESCRIPTION OF THE DRAWINGS

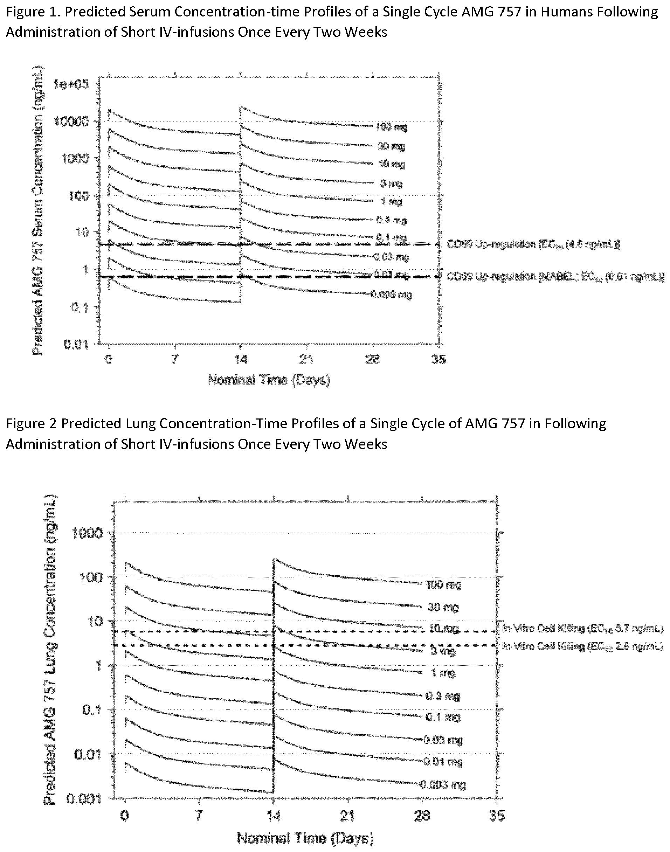

Figure 1 shows predicted serum concentration-time profiles of a single cycle of AMG 757 in humans after administration of short IV-infusions once every two weeks. The dashed lines represent the concentrations required for 50% and 90% maximal effect (EC50 and EC90 values of 0.61 ng/mL and 4.6 ng/mL, respectively) of AMG 757-mediated CD69 upregulation, which was identified as the most sensitive marker of AMG 757 activity (Study 123564).
Figure 2 shows predicted lung concentration-time profiles of a single cycle of AMG 757 in humans after administration of short IV-infusions once every two weeks. The dashed lines represent the concentrations required for 50% and 90% maximal effect (EC50 and EC90 values of 2.8 ng/mL and 5.7 ng/mL, respectively) of AMG 757-mediated cell killing in SHP-77 cells.

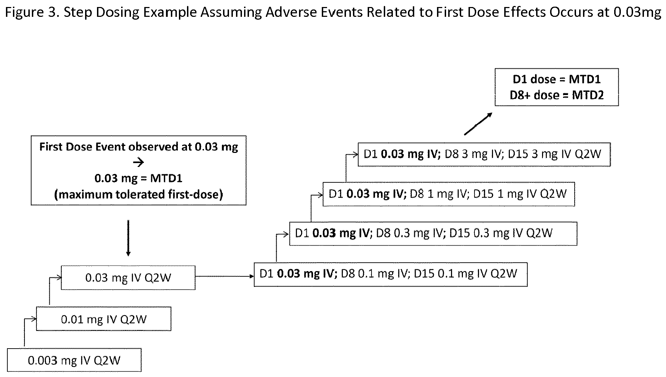

Figure 3 shows a step dosing example assuming adverse events related to first dose effects occurs at 0.03 mg.

### DETAILED DESCRIPTION

As disclosed and exemplified herein, a Phase 1 clinical study was conducted for the treatment of SCLC, using a bispecific protein (AMG 757) that targets DLL3 and CD3.

AMG 757 is a half-life-extended BiTE® (bispecific T cell engager) molecule developed for the treatment of SCLC. The activity of AMG 757 requires the simultaneous binding to both target cells (DLL3⁺ cells) and T cells. The pharmacological effect of AMG 757 is mediated by specific redirection of previously primed cytotoxic CD8⁺ or CD4⁺ T lymphocytes to kill DLL3⁺ cells. The selection of the starting dose for the First in Human (FIH) study was based on the Minimum Anticipated Biological Effect Level (MABEL), which was identified as the EC₅₀ of AMG 757-mediated CD69 upregulation in SHP-77 cells (Study 123564). A starting dose of 0.003 mg once every two weeks (Q2W) was selected based on human PK predictions and is predicted to generate maximum serum concentrations equivalent to the MABEL (0.61 ng/mL). This regimen is expected to achieve adequate exposures in target tissues (e.g., lung) throughout the entire dosing interval, while minimizing peak-to-trough ratios after multiple treatment cycles of AMG 757. Based on clinical experience in the FIH study, a dose of at least 0.3 mg Q2W is desirable.

### 1. DEFINITION

Some of exemplary bispecific anti-GLL3 agents disclosed herein (such as BiTE® molecules) are recombinant protein constructs comprising two binding domains, each domain derived from an antigen-binding fragment of a full-length antibody. Such antigen-binding fragment retains the ability to specifically bind to an antigen (preferably with substantially the same binding affinity). Examples of an antigen-binding fragment includes (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, and (v) a dAb fragment (Ward et al., 1989 Nature 341:544-546), which consists of a VH domain. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. Science 242:423- 426 (1988) and Huston et al., 1988, Proc. Natl. Acad. Sci. USA 85:5879-5883.

A "variable domain" refers to the variable region of the antibody light chain (VL) or the variable region of the antibody heavy chain (VH), either alone or in combination. As known in the art, the variable regions of the heavy and light chains each consist of four framework regions (FR) connected by three complementarity determining regions (CDRs), and contribute to the formation of the antigen-binding site of antibodies.

The "Complementarity Determining Regions" (CDRs) of exemplary DLL3-binding domains and CD3-binding domains are provided in the Sequence Table. The CDRs can be defined according to Kabat, Chothia, the accumulation of both Kabat and Chothia, AbM, contact, North, and/or conformational definitions or any method of CDR determination well known in the art. See, e.g., Kabat et al., 1991, Sequences of Proteins of Immunological Interest, 5th ed. (hypervariable regions); Chothia et al., 1989, Nature 342:877-883 (structural loop structures). AbM definition of CDRs is a compromise between Kabat and Chothia and uses Oxford Molecular's AbM antibody modeling software (Accelrys®). The identity of the amino acid residues in a particular antibody that make up a CDR can be determined using methods well known in the art.

The term "treatment" includes prophylactic and/or therapeutic treatments. If it is administered prior to clinical manifestation of a condition, the treatment is considered prophylactic. Therapeutic treatment includes, e.g., ameliorating or reducing the severity of a disease, or shortening the length of the disease.

"About" or "approximately," when used in connection with a measurable numerical variable, refers to the indicated value of the variable and to all values of the variable that are within the experimental error of the indicated value (e.g. within the 95% confidence interval for the mean) or ± 10% of the indicated value, whichever is greater. Numeric ranges are inclusive of the numbers defining the range.

### 2. ANTI-DLL3 AGENTS

DLL3 is a non-canonical Notch ligand expressed primarily during embryonic development that functions during somitogenesis. In contrast to other Notch ligands that are expressed on the surface of cells, DLL3 accumulate in the Golgi in normal tissues (Geffers et al, J Cell Biol.178:465-476 (2007)). DLL3 was identified as a tumor-associated antigen and a compelling target for T cell-based therapies by analyzing the differential expression of this target in 28 SCLC tumors and a large panel of normal tissues (Study 123658).

The human DLL3 protein comprises eight extracellular domains: signal peptide, N-terminus, DSL, EGF1, EGF2, EGF3, EGF4, EGF5 and EGF6. The amino acid sequence of human DLL3, the EGF3 domain, the EGF4 domain, and the combined EGF3 and EGF4 domains are shown in the sequence table as SEQ ID NOs: 252, 258, 259 and 260, respectively.

An exemplary anti-DLL3 agent is a bispecific molecule that binds DLL3 and CD3, such as a BiTE® (bispecific T cell engager) molecule. BiTE® molecules are recombinant protein constructs made from two flexibly linked binding domains, each domain derived from antibodies. One binding domain of BiTE® molecule is specific for a tumor-associated surface antigen (such as DLL3); the second binding domain is specific for CD3, a subunit of the T cell receptor complex on T cells. By their design, BiTE® molecules are uniquely suited to transiently connect T cells with target cells and, at the same time, potently activate the inherent cytolytic potential of T cells against target cells. See e.g., WO 99/54440, WO 2005/040220, and WO 2008/119567.

Accordingly, in some embodiments, the anti-DLL3 agent described comprises two binding domains: the first domain binds DLL3 (preferably human DLL3), and the second domain binds CD3 (preferably human CD3). Preferably, the first domain binds to an epitope of DLL3 comprised within the amino acid sequence of SEQ ID NO: 260. More preferably, the first domain binds to an epitope of DLL3 comprised within the amino acid sequence of SEQ ID NO: 258.

In certain embodiments, the DLL3-binding domain comprises (a) a heavy chain variable region (VH) that comprises: (i) a VH complementarity determining region one (CDR-H1) comprising the amino acid sequence of SEQ ID NO:31; (ii) a CDR-H2 comprising the amino acid sequence of SEQ ID NO:32; and (iii) a CDR-H3 comprising the amino acid sequence of SEQ ID NO:33; and (b) a light chain variable region (VL) that comprises: (i) a VL complementarity determining region one (CDR-L1) comprising the amino acid sequence of SEQ ID NO:34; (ii) a CDR-L2 comprising the amino acid sequence of SEQ ID NO:35; and (iii) a CDR-L3 comprising the amino acid sequence of SEQ ID NO:36.

In certain embodiments, the DLL3-binding domain comprises: a VH that comprises the amino acid sequence of SEQ ID NO:37, and a VL that comprises the amino acid sequence of SEQ ID NO:38. In certain preferred embodiments, the DLL3-binding domain comprises: a VH that comprises the amino acid sequence of SEQ ID NO:435, and a VL that comprises the amino acid sequence of SEQ ID NO:436.

In some embodiments, the VH and VL are joined by a linker to form a single chain Fv (scFv). In some embodiments, the linker is a peptide linker comprising a sequence selected from any one of SEQ ID NOs: 285-293. In some embodiments, the linker is a GS liker, such as Gly-Gly-Gly-Gly-Ser (G4S, SEQ ID NO: 286), or polymers thereof, i.e. (Gly4Ser)x, where x is an integer of 1 or greater (e.g. 2 or 3) (e.g., SEQ ID NOs: 292, 293).

In certain embodiments, the DLL3-binding domain comprises the amino acid sequence of SEQ ID NO: 39. In certain preferred embodiments, the DLL3-binding domain comprises the amino acid sequence of SEQ ID NO: 347.

In certain embodiments, the CD3-binding domain comprises: (a) a VH that comprises: a CDR-H1 comprising the amino acid sequence of SEQ ID NO:426, a CDR-H2 comprising the amino acid sequence of SEQ ID NO:427, and a CDR-H3 comprising the amino acid sequence of SEQ ID NO:428; and a VL that comprises: a CDR-L1 comprising the amino acid sequence of SEQ ID NO:423, a CDR-L2 comprising the amino acid sequence of SEQ ID NO:424, and a CDR-L3 comprising the amino acid sequence of SEQ ID NO:425.

In certain embodiments, the CD3-binding domain comprises: a VH that comprises the amino acid sequence of SEQ ID NO:429, and a VL that comprises the amino acid sequence of SEQ ID NO:430. In some embodiments, the VH and VL are joined by a linker to form a single chain Fv (scFv). In some embodiments, the linker is a peptide linker comprising a sequence selected from any one of SEQ ID NOs: 285-293. In some embodiments, the linker is a GS liker, such as Gly-Gly-Gly-Gly-Ser (G4S, SEQ ID NO: 286), or polymers thereof, i.e. (Gly4Ser)x, where x is an integer of 1 or greater (e.g. 2 or 3).

In certain embodiments, the CD3-binding domain comprises the amino acid sequence of SEQ ID NO: 431.

In certain embodiments, the DLL3-binding domain and the CD3-binding domain are joined by a linker. In some embodiments, the linker is a peptide linker comprising a sequence selected from any one of SEQ ID NOs: 285-293. In some embodiments, the linker is a GS liker, such as Gly-Gly-Gly-Gly-Ser (G4S, SEQ ID NO: 286), or polymers thereof, i.e. (Gly4Ser)x, where x is an integer of 1 or greater (e.g., 2 or 3).

In certain embodiments, the anti-DLL3 agent disclosed herein comprises two domains. The first domain binds to DLL3 (preferably human DLL3) and comprises (a) a heavy chain variable region (VH) that comprises: (i) a VH complementarity determining region one (CDR-H1) comprising the amino acid sequence of SEQ ID NO:31; (ii) a CDR-H2 comprising the amino acid sequence of SEQ ID NO:32; and (iii) a CDR-H3 comprising the amino acid sequence of SEQ ID NO:33; and (b) a light chain variable region (VL) that comprises: (i) a VL complementarity determining region one (CDR-L1) comprising the amino acid sequence of SEQ ID NO:34; (ii) a CDR-L2 comprising the amino acid sequence of SEQ ID NO:35; and (iii) a CDR-L3 comprising the amino acid sequence of SEQ ID NO:36. The second domain binds to CD3 (preferably human CD3), and comprises (a) a VH that comprises: (i) a CDR-H1 comprising the amino acid sequence of SEQ ID NO:426, (ii) a CDR-H2 comprising the amino acid sequence of SEQ ID NO:427, and (iii) a CDR-H3 comprising the amino acid sequence of SEQ ID NO:428; and (b) a VL that comprises: (i) a CDR-L1 comprising the amino acid sequence of SEQ ID NO:423, (ii) a CDR-L2 comprising the amino acid sequence of SEQ ID NO:424, and (iii) a CDR-L3 comprising the amino acid sequence of SEQ ID NO:425.

In certain embodiments, the anti-DLL3 agent described herein comprises two domains: (a) the first domain binds DLL3 (preferably human DLL3) and comprises: a VH that comprises the amino acid sequence of SEQ ID NO:37, and a VL that comprises the amino acid sequence of SEQ ID NO:38; and (b) the second domain binds CD3 (preferably human CD3) and comprises: a VH that comprises the amino acid sequence of SEQ ID NO:429, and a VL that comprises the amino acid sequence of SEQ ID NO:430. In certain preferred embodiments, In certain embodiments, the anti-DLL3 agent described herein comprises two domains: (a) the first domain binds DLL3 (preferably human DLL3) and comprises: a VH that comprises the amino acid sequence of SEQ ID NO:435, and a VL that comprises the amino acid sequence of SEQ ID NO: 436; and (b) the second domain binds CD3 (preferably human CD3) and comprises: a VH that comprises the amino acid sequence of SEQ ID NO:429, and a VL that comprises the amino acid sequence of SEQ ID NO:430.

In certain embodiments, the anti-DLL3 agent described herein comprises two domains: (a) the first domain binds DLL3 (preferably human DLL3) and comprises the amino acid sequence of SEQ ID NO: 39, (b) the second domain binds CD3 (preferably human CD3) and comprises the amino acid of SEQ ID NO: 431. In certain embodiments, the anti-DLL3 agent described herein comprises two domains: (a) the first domain binds DLL3 (preferably human DLL3) and comprises the amino acid sequence of SEQ ID NO: 437, (b) the second domain binds CD3 (preferably human CD3) and comprises the amino acid of SEQ ID NO: 431.

In certain embodiments, the anti-DLL3 agent described herein comprises the amino acid sequence of SEQ ID NO: 40. In certain embodiments, the anti-DLL3 agent described herein comprises the amino acid sequence of SEQ ID NO: 438.

In certain embodiments, anti-DLL3 agent described herein further comprises a third domain that extends or enhance the serum half-life of the anti-DLL3 agent. In certain embodiments, the third domain comprises two polypeptides joined by a linker, each peptide comprising a hinge, a CH2 and a CH3 domain of human IgG. In certain embodiments, the third domain comprises, in an N- to C-terminal order: hinge-CH2-CH3-linker-hinge-CH2-CH3. In some embodiments, the linker is a GS liker, such as Gly-Gly-Gly-Gly-Ser (G4S, SEQ ID NO: 286), or polymers thereof, i.e. (Gly4Ser)x, where x is an integer of 1 or greater (e.g., 6). In certain embodiments, the third domain comprises the amino acid sequence selected from any one of SEQ ID NOs: 541-548.

In certain embodiments, the DLL3-binding domain and the CD3-binding domain are joined by a first linker to form a peptide, which is joined to the third domain by a second linker. In certain embodiments, the first linker is a peptide linker comprising a sequence selected from any one of SEQ ID NOs: 285-293, and the second linker comprises a sequence selected from any one of SEQ ID NO: 285, 286, 288, 289, 290, 292 and 293. In some embodiments, the first linker is a GS liker, such as Gly-Gly-Gly-Gly-Ser (G4S, SEQ ID NO: 286), or polymers thereof, i.e. (Gly4Ser)x, where x is an integer of 1 or greater (e.g. 2 or 3), and the second linker comprises a sequence selected from any one of SEQ ID NO: 285, 286, 288, 289, 290, 292 and 293.

In certain embodiments, the anti-DLL3 agent described herein comprises three domains: (a) the first domain binds DLL3 (preferably human DLL3) and comprises the amino acid sequence of SEQ ID NO: 39, (b) the second domain binds CD3 (preferably human CD3) and comprises the amino acid of SEQ ID NO: 431, and (c) the third domain comprises an amino acid sequence selected from any one of SEQ ID NOs: 541-548. In certain embodiments, the anti-DLL3 agent described herein comprises three domains: (a) the first domain binds DLL3 (preferably human DLL3) and comprises the amino acid sequence of SEQ ID NO: 437, (b) the second domain binds CD3 (preferably human CD3) and comprises the amino acid of SEQ ID NO: 431, and (c) the third domain comprises any one of the amino acid sequence selected from SEQ ID NOs: 541-548.

In certain embodiments, the anti-DLL3 agent described herein comprises the amino acid sequence of SEQ ID NO: 520.

### 3. DOSING OF ANTI-DLL3 AGENTS

Disclosed herein are methods of treating DLL3-positive cancer comprising administering to a subject in need thereof an anti-DLL3 agent, at a dose of from about 0.3 mg to about 100 mg once every two weeks. In certain embodiments, the DLL3-positive cancer is small cell lung cancer (SCLC). In certain embodiments, the SCLC is relapsed/refractory SCLC (RR SCLC) or extensive disease SCLC (ED SCLC). In certain embodiments, the subject is a human having SCLC, e.g., RR SCLC or ED SCLC.

In certain embodiments, the anti-DLL3 agent is administered once every two weeks at a dose of: from about 0.3 mg to about 100 mg, from about 0.3 mg to about 90 mg, from about 0.3 mg to about 80 mg, from about 0.3 mg to about 70 mg, from about 0.3 mg to about 60 mg, from about 0.3 mg to about 50 mg, from about 0.3 mg to about 40 mg, from about 0.3 mg to about 30 mg, from about 0.3 mg to about 20 mg, from about 0.3 mg to about 10 mg, from about 0.3 mg to about 3 mg, from about 0.3 mg to about 1 mg, from about 1 mg to about 100 mg, from about 1 mg to about 90 mg, from about 1 mg to about 80 mg, from about 1 mg to about 70 mg, from about 1 mg to about 60 mg, from about 1 mg to about 50 mg, from about 1 mg to about 40 mg, from about 1 mg to about 30 mg, from about 1 mg to about 20 mg, from about 1 mg to about 10 mg, from about 1 mg to about 3 mg, from about 3 mg to about 100 mg, from about 3 mg to about 90 mg, from about 3 mg to about 80 mg, from about 3 mg to about 70 mg, from about 3 mg to about 60 mg, from about 3 mg to about 50 mg, from about 3 mg to about 40 mg, from about 3 mg to about 30 mg, from about 3 mg to about 20 mg, from about 3 mg to about 10 mg, from about 3 mg to about 12 mg, from about 3 mg to about 15 mg, from about 10 mg to about 100 mg, from about 10 mg to about 90 mg, from about 10 mg to about 80 mg, from about 10 mg to about 70 mg, from about 10 mg to about 60 mg, from about 10 mg to about 50 mg, from about 10 mg to about 40 mg, from about 10 mg to about 30 mg, from about 10 mg to about 20 mg, from about 10 mg to about 15 mg, from about 20 mg to about 100 mg, from about 20 mg to about 90 mg, from about 20 mg to about 80 mg, from about 20 mg to about 70 mg, from about 20 mg to about 60 mg, from about 20 mg to about 50 mg, from about 20 mg to about 40 mg, from about 20 mg to about 30 mg, from about 30 mg to about 100 mg, from about 30 mg to about 90 mg, from about 30 mg to about 80 mg, from about 30 mg to about 70 mg, from about 30 mg to about 60 mg, from about 30 mg to about 50 mg, or from about 30 mg to about 40 mg.

In certain embodiments, the anti-DLL3 agent is administered on day 1 and day 15 of a 28-day cycle.

As described Example 2, the starting dose for the FIH study was based on the Minimum Anticipated Biological Effect Level (MABEL), which was identified as the EC50 of AMG 757-mediated CD69 upregulation in SHP-77 cells (Study 123564). A starting dose of 0.003 mg was selected based on human PK predictions and is predicted to generate maximum serum concentrations equivalent to the MABEL (0.61 ng/mL). The dose of AMG 757 in the Phase 1 clinical study is 0.003 mg and higher and is administered as intravenous (IV) infusions once every two weeks (Q2W) in patients with SCLC. This regimen is believed to achieve adequate exposures in target tissues (e.g., lung) throughout the entire dosing interval, while minimizing peak-to-trough ratios after multiple treatment cycles of AMG 757.

There was one confirmed response at 0.3 mg administered as IV infusion once every two weeks in the clinical study. It is believed that efficacious dose of AMG 757 can be at least 0.3 mg (e.g., from about 0.3 mg to about 100 mg) administered once every two weeks.

The anti-DLL3 agent can be administered by any suitable means, including parenteral, subcutaneous, intraperitoneal, intrapulmonary, intranasal, and/or intralesional administration. Parenteral administration includes intramuscular, intravenous, intraarterial, intraperitoneal, or subcutaneous administration. In some embodiments, the anti-DLL3 agent is administered by intravenous (IV) infusion, such as a short IV infusion (approximately 60 minutes), once every two weeks.

### 3.1 STEP DOSING

Due to its mechanism of action, subjects may be at an increased risk for first dose effects (e.g., cytokine release syndrome) following initial infusion of AMG 757. It is believed that an optimal maximal tolerated dose (MTD) may require a step dosing approach (e.g., initial dose on day 1, step dose on Day 8, and subsequent dose starting on day 15 and thereafter). Two MTDs may be estimated, one for the initial dosing (MTD1) and one for the subsequent dosing (MTD2).

If a first dose effect (e.g., cytokine release syndrome (CRS)) is experienced by a subject, an appropriate first dose (MTD1) not exceeding the dose at which a CRS event is observed can be determined and implemented. A second dose and a subsequent dose can also be determined and implemented. In certain embodiments, the second dose and the subsequent dose are the same, and are higher than the first dose. These doses and dosing schedules can be guided by modeling and simulation of clinical data (e.g., pharmacokinetics, safety data, etc.) to ensure that systemic exposures of AMG 757 may not exceed those associated with doses at which first dose effects were seen (e.g., CRS) due to the increased dosing frequency of a step dosing regimen and potential for drug accumulation.

Accordingly, disclosed herein are methods of treating DLL3-positive cancer comprising administering to a subject in need thereof an anti-DLL3 agent, wherein the anti-DLL3 agent is administered according to the following schedule: a) a first dose of from 0.3 mg to 100 mg on day 1, b) a second dose of from 0.3 mg to 100 mg on day 8, and c) one or more subsequence doses of from 0.3 mg to 100 mg, starting on day 15 and once every two weeks thereafter, and wherein the second and subsequent doses are the same, and are higher than the first dose.

In certain embodiments, the second and the subsequent doses are the same and are at least 1.5-fold, at least 2-fold, at least 3-fold, at least 4-fold, at least 5-fold, at least 6-fold, at least 7-fold, at least 8-fold, at least 9-fold, at least 10-fold, at least 20-fold, at least 25-fold, at least 30-fold, at least 35-fold, at least 40-fold, at least 45-fold, at least 50-fold, at least 55-fold, at least 60-fold, at least 65-fold, at least 70-fold, at least 75-fold, at least 80-fold, at least 85-fold, at least 90-fold, at least 95-fold, or at least 100-fold higher than the first dose.

In certain embodiments, the second and the subsequent doses are the same and are at least 1.5-fold, at least 2-fold, at least 3-fold, at least 4-fold, at least 5-fold, at least 6-fold, at least 7-fold, at least 8-fold, at least 9-fold, at least 10-fold, at least 20-fold, at least 25-fold, at least 30-fold, at least 35-fold, at least 40-fold, at least 45-fold, at least 50-fold, at least 55-fold, at least 60-fold, at least 65-fold, at least 70-fold, at least 75-fold, at least 80-fold, at least 85-fold, at least 90-fold, at least 95-fold, or at least 100-fold higher than the first dose, and each of the first, second and subsequent doses of the anti-DLL3 agent can be any one of the following: from about 0.3 mg to about 100 mg, from about 0.3 mg to about 90 mg, from about 0.3 mg to about 80 mg, from about 0.3 mg to about 70 mg, from about 0.3 mg to about 60 mg, from about 0.3 mg to about 50 mg, from about 0.3 mg to about 40 mg, from about 0.3 mg to about 30 mg, from about 0.3 mg to about 20 mg, from about 0.3 mg to about 10 mg, from about 0.3 mg to about 3 mg, from about 0.3 mg to about 1 mg, from about 1 mg to about 100 mg, from about 1 mg to about 90 mg, from about 1 mg to about 80 mg, from about 1 mg to about 70 mg, from about 1 mg to about 60 mg, from about 1 mg to about 50 mg, from about 1 mg to about 40 mg, from about 1 mg to about 30 mg, from about 1 mg to about 20 mg, from about 1 mg to about 10 mg, from about 1 mg to about 3 mg, from about 3 mg to about 100 mg, from about 3 mg to about 90 mg, from about 3 mg to about 80 mg, from about 3 mg to about 70 mg, from about 3 mg to about 60 mg, from about 3 mg to about 50 mg, from about 3 mg to about 40 mg, from about 3 mg to about 30 mg, from about 3 mg to about 20 mg, from about 3 mg to about 10 mg, from about 3 mg to about 12 mg, from about 3 mg to about 15 mg, from about 10 mg to about 100 mg, from about 10 mg to about 90 mg, from about 10 mg to about 80 mg, from about 10 mg to about 70 mg, from about 10 mg to about 60 mg, from about 10 mg to about 50 mg, from about 10 mg to about 40 mg, from about 10 mg to about 30 mg, from about 10 mg to about 20 mg, from about 10 mg to about 15 mg, from about 20 mg to about 100 mg, from about 20 mg to about 90 mg, from about 20 mg to about 80 mg, from about 20 mg to about 70 mg, from about 20 mg to about 60 mg, from about 20 mg to about 50 mg, from about 20 mg to about 40 mg, from about 20 mg to about 30 mg, from about 30 mg to about 100 mg, from about 30 mg to about 90 mg, from about 30 mg to about 80 mg, from about 30 mg to about 70 mg, from about 30 mg to about 60 mg, from about 30 mg to about 50 mg, and from about 30 mg to about 40 mg.

The anti-DLL3 agent can be administered by any suitable means, including parenteral, subcutaneous, intraperitoneal, intrapulmonary, intranasal, and/or intralesional administration. Parenteral administration includes intramuscular, intravenous, intraarterial, intraperitoneal, or subcutaneous administration. In some embodiments, the anti-DLL3 agent is administered by intravenous (IV) infusion.

In certain embodiments, the DLL3-positive cancer is small cell lung cancer (SCLC). In certain embodiments, the SCLC is relapsed/refractory SCLC (RR SCLC) or extensive disease SCLC (ED SCLC). In certain embodiments, the subject is a human having SCLC, e.g., RR SCLC or ED SCLC.

In some embodiments, the compositions and methods of the invention provide for the use of an anti-DLL3 agent in combination with one or more additional therapeutic agents.

In some embodiments, the one or more additional therapeutic agents may be an anti-inflammatory agent (for example, to prophylactically treat CRS). The anti-inflammatory agent may be administered prior to, concurrently, or after the administration of the anti-DLL3 agent. Exemplary anti-inflammatory agent includes acetaminophen, naproxen sodium, ibuprofen, tramadol, aspirin, celecoxib, valdecoxib, indomethacin, or other NSAIDs. Other anti-inflammatory agent includes, e.g., beclomethasone, hydroxycortisone, betamethasone, methylprednisolone, budesonide, prednisolone, cortisone, prednisone, dexamethasone, and triamcinolone, or other glucocorticoids.

When a steroid (such as dexamethasone) is used, higher doses of anti-DLL3 agent may be needed. Accordingly, in some aspect, the invention provides a method of treating SCLC, or an DLL3-positive cancer, comprising administering to a subject in need thereof a steroid (such as a corticosteroid, e.g., dexamethasone), and an anti-DLL3 agent, wherein the anti-DLL3 agent is administered at a dose of from about 0.3 mg to 100 mg once every two weeks (such as from about 0.3 mg to about 90 mg, from about 0.3 mg to about 80 mg, from about 0.3 mg to about 70 mg, from about 0.3 mg to about 60 mg, from about 0.3 mg to about 50 mg, from about 0.3 mg to about 40 mg, from about 0.3 mg to about 30 mg, from about 0.3 mg to about 20 mg, from about 0.3 mg to about 10 mg, from about 0.3 mg to about 3 mg, from about 0.3 mg to about 1 mg, from about 1 mg to about 30 mg, from about 1 mg to about 20 mg, from about 3 mg to about 15 mg, or from about 3 mg to about 12 mg, once every two weeks).

In some aspect, the invention provides a method of treating SCLC, or an DLL3-positive cancer, comprising administering to a subject in need thereof a steroid (such as a corticosteroid, e.g., dexamethasone), and an anti-DLL3 agent, wherein the anti-DLL3 agent is administered according to the following schedule: (a) a first dose of from 0.3 mg to 100 mg on day 1 (such as from about 0.3 mg to about 90 mg, from about 0.3 mg to about 80 mg, from about 0.3 mg to about 70 mg, from about 0.3 mg to about 60 mg, from about 0.3 mg to about 50 mg, from about 0.3 mg to about 40 mg, from about 0.3 mg to about 30 mg, from about 0.3 mg to about 20 mg, from about 0.3 mg to about 10 mg, from about 0.3 mg to about 3 mg, from about 0.3 mg to about 1 mg, from about 1 mg to about 30 mg, from about 1 mg to about 20 mg, from about 3 mg to about 15 mg, or from about 3 mg to about 12 mg, on day 1), (b) a second dose of from 0.3 mg to 100 mg on day 8 (such as from about 0.3 mg to about 90 mg, from about 0.3 mg to about 80 mg, from about 0.3 mg to about 70 mg, from about 0.3 mg to about 60 mg, from about 0.3 mg to about 50 mg, from about 0.3 mg to about 40 mg, from about 0.3 mg to about 30 mg, from about 0.3 mg to about 20 mg, from about 0.3 mg to about 10 mg, from about 0.3 mg to about 3 mg, from about 0.3 mg to about 1 mg, from about 1 mg to about 30 mg, from about 1 mg to about 20 mg, from about 3 mg to about 15 mg, or from about 3 mg to about 12 mg, on day 8), and (c) one or more subsequence doses of from 0.3 mg to 100 mg (such as from about 0.3 mg to about 90 mg, from about 0.3 mg to about 80 mg, from about 0.3 mg to about 70 mg, from about 0.3 mg to about 60 mg, from about 0.3 mg to about 50 mg, from about 0.3 mg to about 40 mg, from about 0.3 mg to about 30 mg, from about 0.3 mg to about 20 mg, from about 0.3 mg to about 10 mg, from about 0.3 mg to about 3 mg, from about 0.3 mg to about 1 mg, or from about 1 mg to about 30 mg, from about 1 mg to about 20 mg, from about 3 mg to about 15 mg, or from about 3 mg to about 12 mg), starting on day 15 and once every two weeks thereafter, and wherein the second and the one or more subsequent doses are the same, and are higher than the first dose. In certain embodiments, the second and the one or more subsequent doses are at least 1.5-fold, at least 2-fold, at least 3-fold, at least 4-fold, at least 5-fold, at least 6-fold, at least 7-fold, at least 8-fold, at least 9-fold, at least 10-fold, at least 20-fold, at least 25-fold, at least 30-fold, at least 35-fold, at least 40-fold, at least 45-fold, at least 50-fold, at least 55-fold, at least 60-fold, at least 65-fold, at least 70-fold, at least 75-fold, at least 80-fold, at least 85-fold, at least 90-fold, at least 95-fold, or at least 100-fold higher than the first dose.

### 4. ARTICLES OF MANUFACTURE

Disclosed herein are articles of manufacture comprising: (a) a container comprising an anti-DLL3 agent; and (b) a package insert with instructions for treating DLL3-positive cancer (or treating SCLC) in a subject, wherein the instructions specifies that a dose of from about 0.3 mg to about 100 mg (or any of the dose ranges disclosed herein) of the anti-DLL3 agent be administered to the subject once every two weeks, such as on day 1 and day 15 of a 28-day cycle. The instructions may also specify that the anti-DLL3 agent be administered according to the following schedule: a) a first dose of from 0.3 mg to 100 mg (or any of the dose ranges disclosed herein) on day 1, b) a second dose of from 0.3 mg to 100 mg (or any of the dose ranges disclosed herein) on day 8, and c) one or more subsequence doses of from 0.3 mg to 100 mg (or any of the dose ranges disclosed herein), starting on day 15 and once every two weeks thereafter, and wherein the second and one or more subsequent doses are the same, and are higher than the first dose.

### EXAMPLES

### EXAMPLE 1 PREDICTION OF HUMAN AMG 757 PHARMACOKINETICS AND FIRST-INHUMAN DOSE SELECTION

The human PK parameters of AMG 757 were predicted using allometric scaling of PK parameters obtained from studies in cynomolgus monkeys at doses ranging from 12 to 4500 µg/kg. A two-compartment model with linear elimination was used to characterize the pharmacokinetics of AMG 757 from the pooled cynomolgus monkey data, excluding data from animals that were identified as positive for anti-drug antibodies after the administration of the first dose. The model was parameterized using linear clearance (CL), volume of distribution of central compartment (V_{C}), distribution clearance (CL_{D}), and peripheral volume of distribution (V_{T}). Allometry was used to predict human AMG 757 PK using exponents of 0.75 and 1 for clearance and volume parameters, respectively. The human and cynomolgus monkey body weights were assumed to be 60 kg and 3 kg, respectively. Derived monkey and predicted human AMG 757 PK parameters are provided in Table 1.

**Table 1 Cynomolgus Monkey and Predicted Human Pharmacokinetic Parameters Used for Human AMG 757 Exposure Predictions**

| | | | Reference | |
|---|---|---|---|---|
| Parameter (Units) | Cynomolgus Monkey^{a} | Human (Scaled)^{b} | Cynomolgus Monkey | Human (Scaled) |
| CL (mL/h/kg) | 0.490 | 0.249 | Fitted | Allometric scaling |
| CL_{D} (mUh/kg) | 1.95 | 0.991 | Fitted | Allometric scaling |
| V_{C}(mL/kg) | 70.6 | 70.6 | Fitted | Allometric scaling |
| V_{T} (mL/kg) | 112 | 112 | Fitted | Allometric scaling |
| t_{½} (h) | 258 | 560 | Calculated | Calculated |

| | | | | |
|---|---|---|---|---|
| CL = systemic clearance; CL_{D} = distribution clearance; t_{½} = terminal half-life; V_{C} = central volume of distribution; V_{T} = peripheral volume of distribution ^{a} assuming a monkey body weight of 3 kg ^{b} assuming a human body weight of 60 kg | | | | |

In combination with these PK predictions, the FIH starting dose was selected based on the identified in vitro Minimum Anticipated Biological Effect Level (MABEL). This concentration was determined by assessing the most sensitive marker of AMG 757 activity in the most sensitive DLL3-expressing cell line (Study 123564). In conjunction with animal exposures from the GLP toxicology studies, the predicted human exposures were used to calculate the exposure margins using standard ratio calculations based on AUCₜₐᵤ (168 hours for cynomolgus monkeys in the GLP toxicology study and 336 hours for humans) and Cₘₐₓ for the proposed doses in the FIH study.

The FIH study evaluates the safety, tolerability, and pharmacokinetics of AMG 757 in patients with small cell lung cancer. The predicted human PK parameters described above were used to simulate predicted exposures at the proposed FIH doses (Figure 1). The doses of AMG 757 for the FIH study are 0.003 mg, 0.01 mg, 0.03 mg, 0.1 mg, 0.3 mg, 1 mg, 3 mg, 10 mg, 30 mg, 100 mg, and higher administered as short-term IV infusions (approximately 1 hour) once every two weeks in patients with small cell lung cancer (SCLC) (Study 20160323). This regimen ensures that adequate exposures over the in vitro EC₉₀ for AMG 757-mediated cell killing (5.2 ng/mL) are achieved in lung while minimizing peak-to-trough ratios during multiple treatment cycles of AMG 757.

The selection of the starting dose for the FIH study was based on the in vitro MABEL. Briefly, the EC₅₀ values of AMG 757-mediated cell killing of SHP-77 tumor cells and induction of T cell activation (de novo expression of CD69 and CD25) from human peripheral blood mononuclear cells (PBMCs) were compared, and AMG 757-induced de novo expression of CD69 on T cells was identified as the most sensitive parameter of AMG 757 activity. Based on the assessment of individual dose-response curves from 12 different PBMC donors, the MABEL (mean EC₅₀) was calculated to be 0.61 ng/mL (5.8 pM; Study 123564).

The use of the in vitro EC₅₀ as the MABEL and basis for the FIH starting dose is supported by the previous and safe implementation of this strategy to identify the maximum recommended starting doses of previous BiTE® molecules in clinical development.

### EXAMPLE 2 PREDICTION OF MINUMUMLY EFFECIOUS EXPOSURES OF AMG 757 IN HUMANS

Efficacious exposures of AMG 757 were predicted based on in vitro data generated in Study 122717, which evaluated the in vitro pharmacology of AMG 757. Concentrations for half-maximal effect (EC₅₀) and 90% of maximal effect (EC₉₀) of AMG 757-mediated cell killing in SHP-77 cells (human DLL3-expressing cell line) were used to estimate a concentration range in which efficacy may be expected.

The lungs were used as the representative site of action for AMG 757 and were assumed to achieve exposures of approximately 1% of free serum exposures (Vugmeyser et al., J Pharm Sci. 99:1028-1045 (2010)). Based on this assumption, doses of AMG 757 to provide trough coverage of the EC₅₀ of AMG 757-mediated cell killing in SHP-77 cells were considered to be minimally efficacious. Early signs of efficacy were predicted at 10 mg IV once every two weeks based on trough coverage of the average EC₉₀ of cell killing in SHP-77 cells (assuming 1% lung exposure) for the entire treatment cycle (Figure 2).

### EXAMPLE 3 PHASE 1 STUDY EVALUATING THE SAFETY, TOLERABILITY AND PHARMACOKINETICS OF AMG 757 IN SUBJECTS WITH SCLC

### Background

Small Cell Lung Cancer (SCLC), accounting for 10-15% of lung cancer (Rudin et al, J Clin Oncol. 33:4106-4111(2015)), is an aggressive lung cancer subtype with neuroendocrine differentiation and strongly associated with smoking (Koinis et al, Transl Lung Cancer Res. 5:39-50 (2016)). It displays a distinct natural history characterized by a high growth fraction, rapid doubling time and early establishment of widespread metastatic lesions (Gustafsson et al, Cancer. 113:5-21(2008)). While 30% of patients present with disease confined to one hemithorax [limited disease (LD)], the majority of cases have disease not encompassed by one radiotherapy field [extended disease (ED)]. SCLC is exquisitely sensitive to first-line chemotherapy (approximately 60%-70% response rates) and to radiation which is stark contrast to subsequent resistance to second-line and subsequent therapies after disease recurrence (Byers et al, Cancer.121:664 672(2015)). Patients with ED develop drug resistance and die as a result of disease at a median time of 10 to 12 months from diagnosis (Rudin et al, 2015). For patients with ED SCLC, first line treatment is platinum-based chemotherapy. Most patients in the United States receive platinum-etoposide (EP) chemotherapy (with either carboplatin or cisplatin), and some patients receive platinum-irinotecan as an alternative, especially outside the United States. In March 2019, atezolizumab was approved by the United States Food and Drug Administration (US FDA) in combination with carboplatin and etoposide for the first-line treatment of adult patients with ED-SCLC (Tecentriq® United States Prescribing Information [USPI], 2019). After relapse, topotecan is the only second-line drug approved by the US FDA. However, despite its indication in this setting, topotecan has produced disappointing response rates (Byers et al, Cancer.121:664 672(2015)).

AMG 757 is a half-life extended (HLE) BiTE® molecule targeting DLL3 as a tumor-specific antigen and T-cell receptor-associated complex cluster of differentiation 3 (CD3) on T-cells. AMG 757 is developed for the treatment of SCLC and is a potent molecule acting by formation of an immunological synapse between CD3-positive T cells and cancer cells expressing the DLL3 protein. The resulting proximity triggers the redirected lysis of DLL3-positive target cells by the T cells. AMG 757 monotherapy significantly inhibited growth of subcutaneously implanted DLL3 expressing human melanoma WM266-4 cells and induced regression of orthotopic SHP-77-Luc lung tumors.

### Study Design

Study 20160323 is an open-label, ascending, multiple dose, phase 1 study evaluating AMG 757 administered as a short term intravenous (IV) infusion every 2 weeks (with or without Day 8 step dosing) in subjects with small cell lung cancer. There are two indications for this Study: **A:** Relapsed/refractory small cell lung cancer (RR SCLC) and **B:** Extensive disease SCLC (ED SCLC).

The study contains two parts:
(1) Part A: Evaluate AMG 757 in subjects with relapsed/refractory small cell lung cancer (RR SCLC). Part A contains two phases: (A1) Dose Exploration phase to determine maximum tolerated dose (MTD) or recommended phase 2 dose (RP2D) of AMG 757, and (A2) Dose Expansion phase to confirm the safety and tolerability of the selected dose.
(2) Part B: Evaluate AMG 757 in subjects with Extensive disease SCLC (ED SCLC). Part B commences once MTD or RP2D is identified in Part A.

The primary objectives for both Part A and Part B of the study are to evaluate the safety and tolerability of AMG 757 and to determine MTD or RP2D of AMG 757. The secondary objectives of both Part A and Part B of the study are to characterize the pharmacokinetics (PK) of AMG 757 and to evaluate preliminary anti-tumor activity of AMG 757.

### Part A1: Dose exploration phase

AMG 757 is administered as a short IV infusion (approximately 60 minutes). Pre-specified doses for use in the dose escalation are: 0.003 mg, 0.01 mg, 0.03 mg, 0.1 mg, 0.3 mg, 1 mg, 3 mg, 10 mg, 30 mg, and 100 mg, administered once every two weeks.

**Step Dosing:** subjects may experience first dose effects (e.g., cytokine release syndrome with associated manifestations and any other potentially evolving and unknown first dose effects) following the initial infusion of AMG 757. It is believed that an optimal MTD may require a step dosing approach (initial dose on day 1 and step dose on Day 8). Two MTDs may be estimated, one for the initial dosing (MTD1) and one for the subsequent dosing (MTD2).

At any point during the study, the first time a subject experiences a first dose effect (e.g., CRS event of any grade), the safety data need to be reviewed to determine the appropriate dose to be implemented as an initial dose (MTD1), which does not exceed the dose where a CRS of Grade 2 or higher is observed. These doses and dosing schedules are guided by modeling and simulation of emerging clinical data (e.g., pharmacokinetics, safety data, etc.) to ensure that systemic exposures of AMG 757 do not exceed those associated with doses at which first dose effects were seen. Increased dosing frequency of a step dosing regimen and potential for drug accumulation need to be taken into account.

For all subjects enrolled in subsequent cohorts throughout the study, dose escalation continues with a constant dose for the first dose of the first cycle of AMG 757 (MTD1) and only the step dose will be escalated according to the pre-specified doses in the dose escalation to determine MTD2. An example of step dosing is shown in Figure 3.
**Part A2: Dose expansion phase.** Part A2 commences once the MTD or RP2D is selected based on dose exploration phase (Part A1).
**Part B:** Part B will commence once a preliminary MTD or RP2D in Indication A (Part A1) is established.

Table 2 summaries the Eligibility Criteria for Study 20160323

**Table 2 Key Eligibility Criteria**

| Key inclusion criteria | Key exclusion criteria |
|---|---|
| Male or female ≥ 18 years of age with Histologically or cytologically confirmed Small Cell Lung Cancer (SCLC) | History of other malignancy within the past 2 years prior to first dose of AMG 757 with exceptions |
| **Part A:** RR SCLC who progressed or recurred following platinum-based chemotherapy | Major surgery within 28 days of first dose AMG 757 |
| **Part B:** ED SCLC with ongoing clinical benefit (stable disease [SD], partial response [PR], or complete response [CR]) following no more than 6 cycles of first-line platinum-based chemotherapy with the last dose of chemotherapy greater than or equal to 28 days prior to the study day 1 (first-line consolidation setting) | Untreated or symptomatic brain metastases and leptomeningeal disease |
| Eastern Cooperative Oncology Group (ECOG) performance status of 0-2 | Prior anti-cancer therapy: at least 28 days must have elapsed between any prior anti-cancer therapy and first dose of AMG 757 |
| Subjects with treated brain metastases are eligible provided they meet defined criteria | |
| Adequate organ function as defined in protocol | |

### EXAMPLE 4 STUDY ENDPOINTS OF TRIAL 20160323

The hypothesis of Study 20160323 is that AMG 757 is safe and tolerated in subjects with Indications A and B.

**Primary Endpoints:** Dose limiting toxicities (DLTs), treatment-emergent adverse events (AEs), treatment-related AEs, and clinically significant changes in vital signs, ECG, physical examinations, and clinical laboratory tests.

**Secondary Endpoints: For Indications A and B:** (1) PK parameters for AMG 757 following intravenous administration including but not limited to maximum observed concentration (Cₘₐₓ), minimum observed concentration (Cₘᵢₙ), area under the concentration-time curve (AUC) over the 2 week dosing interval, accumulation following multiple dosing, and, if feasible, half-life (t_{1/2}), (2) Objective Response (OR) per modified Response Evaluation Criteria in Solid Tumors (RECIST) 1.1, (3) Duration of Response (DOR), and (4) 1-year Progression-Free Survival (PFS), and (5)1-year Overall Survival (OS). **For Indication B only:** Relapse Free Survival (RFS).

**Exploratory Endpoints: For Indications A and B:** (1) Incidence of anti-AMG 757 antibody formation, (2) Changes in protein, nucleic acid and cellular biomarkers in blood (e.g., cytokines, lymphocyte status, CTCs, sDLL3), (3) Cell surface protein expression (e.g., DLL3) and tumor infiltrating lymphocyte status in tumor tissue at baseline. **For Indication B only:** Effect of prior chemotherapy on T cell cytokine production pre-AMG 757 treatment.

### EXAMPLE 5 EFFECTS IN HUMANS

Pharmacokinetics in humans: Preliminary AMG 757 PK analysis using noncompartmental approach was conducted for subjects in cohorts 1 to 5. Preliminary PK results show that AMG 757 exposures increased with dose from 0.003 mg to 0.3 mg. Estimated terminal half-life (t_{1/2}) is approximately a week across doses after multiple Q2W dosing. No significant accumulation was observed (< 2-fold).

There is one confirmed response at 0.3 mg IV Q2W (cohort 5).

The specification is most thoroughly understood in light of the teachings of the references cited within the specification. The embodiments within the specification provide an illustration of embodiments of the invention and should not be construed to limit the scope of the invention. The skilled artisan readily recognizes that many other embodiments are encompassed by the invention. All publications, patents, and sequences cited in this disclosure are incorporated by reference in their entirety. To the extent the material incorporated by reference contradicts or is inconsistent with this specification, the specification will supersede any such material. The citation of any references herein is not an admission that such references are prior art to the present invention.

Those skilled in the art will recognize, or be able to ascertain using no more than routine experimentation, many equivalents to the specific embodiments of the invention described herein. Such equivalents are intended to be encompassed by the following embodiments.

**Table 3 Sequences Table**

| **SEQ ID NO** | **DLL3 epitope** | **Designation** | **Format / Source** | **Amino acid sequence** |
|---|---|---|---|---|
| 1 | | **DLL3-1** | VH CDR1 | DYGIH |
| 2 | | **DLL3-1** | VH CDR2 | VISYHGSNKYYARSVKG |
| 3 | | **DLL3-1** | VH CDR3 | EIPFGMDV |
| 4 | | **DLL3-1** | VL CDR1 | RSSQSLLHSDGYNYLD |
| 5 | | **DLL3-1** | VL CDR2 | LGSNRAS |
| 6 | | **DLL3-1** | VL CDR3 | MQALQTPLT |
| 7 | | **DLL3-1** | VH | QVQLVESGGGVVQSGRSLRLSCAASGFTFSDYGIHWVRQAPGKGLE WVAVISYHGSNKYYARSVKGRFTVSRDNSKNTLYLQMNSLRAEDTA VYYCAREIPFGMDVWGQGTTVTVSS |
| 8 | | **DLL3-1** | VL | DIVMTQTPLSLPVTPGEPASISCRSSQSLLHSDGYNYLDWYLQKPG QSPQLLIYLGSNRASGVPDRFSGSGSGTDFTLTISRVEAEDVGVYY CMQALQTPLTFGGGTKVDIK |
| 9 | **N-term.** | **DLL3-1** | scFv | QVQLVESGGGVVQSGRSLRLSCAASGFTFSDYGIHWVRQAPGKGLE WVAVISYHGSNKYYARSVKGRFTVSRDNSKNTLYLQMNSLRAEDTA VYYCAREIPFGMDVWGQGTTVTVSSGGGGSGGGGSGGGGSDIVMTQ TPLSLPVTPGEPASISCRSSQSLLHSDGYNYLDWYLQKPGQSPQLL IYLGSNRASGVPDRFSGSGSGTDFTLTISRVEAEDVGVYYCMQALQ TPLTFGGGTKVDIK |
| 10 | | **DLL3-1xI2C** | bispecific molecule | QVQLVESGGGVVQSGRSLRLSCAASGFTFSDYGIHWVRQAPGKGLE WVAVISYHGSNKYYARSVKGRFTVSRDNSKNTLYLQMNSLRAEDTA VYYCAREIPFGMDVWGQGTTVTVSSGGGGSGGGGSGGGGSDIVMTQ TPLSLPVTPGEPASISCRSSQSLLHSDGYNYLDWYLQKPGQSPQLL IYLGSNRASGVPDRFSGSGSGTDFTLTISRVEAEDVGVYYCMQALQ TPLTFGGGTKVDIKSGGGGSEVQLVESGGGLVQPGGSLKLSCAASG FTFNKYAMNWVRQAPGKGLEWVARIRSKYNNYATYYADSVKDRFTI SRDDSKNTAYLQMNNLKTEDTAVYYCVRHGNFGNSYISYWAYWGQG TLVTVSSGGGGSGGGGSGGGGSQTVVTQEPSLTVSPGGTVTLTCGS STGAVTSGNYPNWVQQKPGQAPRGLIGGTKFLAPGTPARFSGSLLG GKAALTLSGVQPEDEAEYYCVLWYSNRWVFGGGTKLTVL |
| 11 | | **DLL3-2** | VH CDR1 | GYYMH |
| 12 | | **DLL3-2** | VH CDR2 | WINPNSGDTNYAQKFQG |
| 13 | | **DLL3-2** | VH CDR3 | DANIAALDAFEI |
| 14 | | **DLL3-2** | VL CDR1 | RASQSISSYLN |
| 15 | | **DLL3-2** | VL CDR2 | AASSLQS |
| 16 | | **DLL3-2** | VL CDR3 | QQSYSTPLT |
| 17 | | **DLL3-2** | VH | QVQLVQSGAEVKKPGASVKVSCKASGYTFTGYYMHWVRQAPGQGLE WMGWINPNSGDTNYAQKFQGRVTMTRDTSISTAYMELSRLTSDDTA VYYCARDANIAALDAFEIWGQGTMVTVSS |
| 18 | | **DLL3-2** | VL | |
| 19 | **N-term.** | **DLL3-2** | scFv | QVQLVQSGAEVKKPGASVKVSCKASGYTFTGYYMHWVRQAPGQGLE WMGWINPNSGDTNYAQKFQGRVTMTRDTSISTAYMELSRLTSDDTA VYYCARDANIAALDAFEIWGQGTMVTVSSGGGGSGGGGSGGGGSDI QMTQSPSSLSASVGDRVTITCRASQSISSYLNWYQQKPGKAPKLLI YAASSLQSGVPSRFSGSGSGTDFTLTISSLQPEDFATYYCQQSYST PLTFGGGTKVEIK |
| 20 | | **DLL3-2 xI2C** | bispecific molecule | QVQLVQSGAEVKKPGASVKVSCKASGYTFTGYYMHWVRQAPGQGLE WMGWINPNSGDTNYAQKFQGRVTMTRDTSISTAYMELSRLTSDDTA VYYCARDANIAALDAFEIWGQGTMVTVSSGGGGSGGGGSGGGGSDI QMTQSPSSLSASVGDRVTITCRASQSISSYLNWYQQKPGKAPKLLI YAASSLQSGVPSRFSGSGSGTDFTLTISSLQPEDFATYYCQQSYST PLTFGGGTKVEIKSGGGGSEVQLVESGGGLVQPGGSLKLSCAASGF TFNKYAMNWVRQAPGKGLEWVARIRSKYNNYATYYADSVKDRFTIS RDDSKNTAYLQMNNLKTEDTAVYYCVRHGNFGNSYISYWAYWGQGT LVTVSSGGGGSGGGGSGGGGSQTVVTQEPSLTVSPGGTVTLTCGSS TGAVTSGNYPNWVQQKPGQAPRGLIGGTKFLAPGTPARFSGSLLGG KAALTLSGVQPEDEAEYYCVLWYSNRWVFGGGTKLTVL |
| 21 | | **DLL3-3** | VH CDR1 | SYGMH |
| 22 | | **DLL3-3** | VH CDR2 | VISYHGRDTYYARSVKG |
| 23 | | **DLL3-3** | VH CDR3 | DGATVTSYYYSGMDV |
| 24 | | **DLL3-3** | VL CDR1 | RASQGISNYLA |
| 25 | | **DLL3-3** | VL CDR2 | LASSLQS |
| 26 | | **DLL3-3** | VL CDR3 | QQYNFYPFT |
| 27 | | **DLL3-3** | VH | QVQLVESGGGVVQPGRSLRLSCAASGFTFSSYGMHWVRQAPGKGLE WVAVISYHGRDTYYARSVKGRFTISRDNSKNTLYLHMNSLRAEDTA VYYCARDGATVTSYYYSGMDVWGQGTTVTVSSK |
| 28 | | **DLL3-3** | VL | DIQMTQSPSSLSASVGDRVTITCRASQGISNYLAWFQQKPGKAPKS LIYLASSLQSGVPSKFSGSGSGTDFTLTISSLQPEDFATYYCQQYN FYPFTFGPGTKVDIK |
| 29 | **EGF-1** | **DLL3-3** | scFv | QVQLVESGGGVVQPGRSLRLSCAASGFTFSSYGMHWVRQAPGKGLE WVAVISYHGRDTYYARSVKGRFTISRDNSKNTLYLHMNSLRAEDTA VYYCARDGATVTSYYYSGMDVWGQGTTVTVSSGGGGSGGGGSGGGG SDIQMTQSPSSLSASVGDRVTITCRASQGISNYLAWFQQKPGKAPK SLIYLASSLQSGVPSKFSGSGSGTDFTLTISSLQPEDFATYYCQQY NFYPFTFGPGTKVDIK |
| 30 | | **DLL3-3 xI2C** | bispecific molecule | QVQLVESGGGVVQPGRSLRLSCAASGFTFSSYGMHWVRQAPGKGLE WVAVISYHGRDTYYARSVKGRFTISRDNSKNTLYLHMNSLRAEDTA VYYCARDGATVTSYYYSGMDVWGQGTTVTVSSGGGGSGGGGSGGGG |
| | | | | SDIQMTQSPSSLSASVGDRVTITCRASQGISNYLAWFQQKPGKAPK SLIYLASSLQSGVPSKFSGSGSGTDFTLTISSLQPEDFATYYCQQY NFYPFTFGPGTKVDIKSGGGGSEVQLVESGGGLVQPGGSLKLSCAA SGFTFNKYAMNWVRQAPGKGLEWVARIRSKYNNYATYYADSVKDRF TISRDDSKNTAYLQMNNLKTEDTAVYYCVRHGNFGNSYISYWAYWG QGTLVTVSSGGGGSGGGGSGGGGSQTVVTQEPSLTVSPGGTVTLTC GSSTGAVTSGNYPNWVQQKPGQAPRGLIGGTKFLAPGTPARFSGSL LGGKAALTLSGVQPEDEAEYYCVLWYSNRWVFGGGTKLTVL |
| 31 | | **DLL3-4** | VH CDR1 | SYYWS |
| 32 | | **DLL3-4** | VH CDR2 | YVYYSGTTNYNPSLKS |
| 33 | | **DLL3-4** | VH CDR3 | IAVTGFYFDY |
| 34 | | **DLL3-4** | VL CDR1 | RASQRVNNNYLA |
| 35 | | **DLL3-4** | VL CDR2 | GASSRAT |
| 36 | | **DLL3-4** | VL CDR3 | QQYDRSPLT |
| 37 | | **DLL3-4** | VH | QVQLQESGPGLVKPSETLSLTCTVSGGSISSYYWSWIRQPPGKGLE WIGYVYYSGTTNYNPSLKSRVTISVDTSKNQFSLKLSSVTAADTAV YYCASIAVTGFYFDYWGQGTLVTVSS |
| 38 | | **DLL3-4** | VL | EIVLTQSPGTLSLSPGERVTLSCRASQRVNNNYLAWYQQRPGQAPR LLIYGASSRATGIPDRFSGSGSGTDFTLTISRLEPEDFAVYYCQQY DRSPLTFGGGTKLEIK |
| 39 | **EGF-3** | **DLL3-4** | scFv | QVQLQESGPGLVKPSETLSLTCTVSGGSISSYYWSWIRQPPGKGLE WIGYVYYSGTTNYNPSLKSRVTISVDTSKNQFSLKLSSVTAADTAV YYCASIAVTGFYFDYWGQGTLVTVSSGGGGSGGGGSGGGGSEIVLT QSPGTLSLSPGERVTLSCRASQRVNNNYLAWYQQRPGQAPRLLIYG ASSRATGIPDRFSGSGSGTDFTLTISRLEPEDFAVYYCQQYDRSPL TFGGGTKLEIK |
| 40 | | **DLL3-4 xI2C** | bispecific molecule | QVQLQESGPGLVKPSETLSLTCTVSGGSISSYYWSWIRQPPGKGLE WIGYVYYSGTTNYNPSLKSRVTISVDTSKNQFSLKLSSVTAADTAV YYCASIAVTGFYFDYWGQGTLVTVSSGGGGSGGGGSGGGGSEIVLT QSPGTLSLSPGERVTLSCRASQRVNNNYLAWYQQRPGQAPRLLIYG ASSRATGIPDRFSGSGSGTDFTLTISRLEPEDFAVYYCQQYDRSPL TFGGGTKLEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVL |
| 41 | | **DLL3-5** | VH CDR1 | SYYWS |
| 42 | | **DLL3-5** | VH CDR2 | YIYYSGRTNYYPSLKS |
| 43 | | **DLL3-5** | VH CDR3 | IAVAGFFFDY |
| 44 | | **DLL3-5** | VL CDR1 | RASQSVNKNYLA |
| 45 | | **DLL3-5** | VL CDR2 | GASSRAT |
| 46 | | **DLL3-5** | VL CDR3 | QQYDRSPLT |
| 47 | | **DLL3-5** | VH | QVQLQESGPGLVKPSETLSLTCTVSGGSISSYYWSWIRQPPGKGLE WIGYIYYSGRTNYYPSLKSRVTISIDTSKNQFSLKLSSVTAADTAV YYCARIAVAGFFFDYWGQGTLVTVSS |
| 48 | | **DLL3-5** | VL | EIVLTQSPGTLSLSPGERATLSCRASQSVNKNYLAWYQQKPGQAPR LLIYGASSRATGIPDRFSGSGSGTDFTLTISRLEPEDFAVYYCQQY DRSPLTFGGGTKLEIK |
| 49 | **EGF-3** | **DLL3-5** | scFv | QVQLQESGPGLVKPSETLSLTCTVSGGSISSYYWSWIRQPPGKGLE WIGYIYYSGRTNYYPSLKSRVTISIDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKLEIK |
| 50 | | **DLL3-5 xI2C** | bispecific molecule | QVQLQESGPGLVKPSETLSLTCTVSGGSISSYYWSWIRQPPGKGLE WIGYIYYSGRTNYYPSLKSRVTISIDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKLEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVL |
| 51 | | **DLL3-6** | VH CDR1 | SFYWS |
| 52 | | **DLL3-6** | VH CDR2 | YIYYSGTTNYNPSLKS |
| 53 | | **DLL3-6** | VH CDR3 | IAVAGFFFDY |
| 54 | | **DLL3-6** | VL CDR1 | RASQSVNKNYLA |
| 55 | | **DLL3-6** | VL CDR2 | GASSRAT |
| 56 | | **DLL3-6** | VL CDR3 | QQYDRSPLT |
| 57 | | **DLL3-6** | VH | QVQLQESGPGLVKPSETLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSS |
| 58 | | **DLL3-6** | VL | EIVLTQSPGTLSLSPGERATLSCRASQSVNKNYLAWYQQKPGQAPR LLIYGASSRATGIPDRFSGSGSGTDFTLTISRLEPEDFAVYYCQQY DRSPLTFGGGTKVEIK |
| 59 | **EGF-3** | **DLL3-6** | scFv | QVQLQESGPGLVKPSETLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKVEIK |
| 60 | | **DLL3-6 xI2C** | bispecific molecule | QVQLQESGPGLVKPSETLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKVEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVL |
| 61 | | **DLL3-7** | VH CDR1 | SFYWS |
| 62 | | **DLL3-7** | VH CDR2 | YIYYSGTTNYNPSLKS |
| 63 | | **DLL3-7** | VH CDR3 | IAVAGFFFDY |
| 64 | | **DLL3-7** | VL CDR1 | RASQSVNKNYLA |
| 65 | | **DLL3-7** | VL CDR2 | GASSRAT |
| 66 | | **DLL3-7** | VL CDR3 | QQYDRSPLT |
| 67 | | **DLL3-7** | VH | QVQLQESGPGLVKPSQTLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSS |
| 68 | | **DLL3-7** | VL | EIVLTQSPGTLSLSPGERATLSCRASQSVNKNYLAWYQQKPGQAPR LLIYGASSRATGIPDRFSGSGSGTDFTLTISRLEPEDFAVYYCQQY DRSPLTFGGGTKVEIK |
| 69 | **EGF-3** | **DLL3-7** | scFv | QVQLQESGPGLVKPSQTLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKVEIK |
| 70 | | **DLL3-7 xI2C** | bispecific molecule | QVQLQESGPGLVKPSQTLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKVEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVL |
| 71 | | **DLL3-8** | VH CDR1 | SFYWS |
| 72 | | **DLL3-8** | VH CDR2 | YIYYSGTTNYNPSLKS |
| 73 | | **DLL3-8** | VH CDR3 | IAVAGFFFDY |
| 74 | | **DLL3-8** | VL CDR1 | RASQSVNKNYLA |
| 75 | | **DLL3-8** | VL CDR2 | GASSRAT |
| 76 | | **DLL3-8** | VL CDR3 | QQYDRSPLT |
| 77 | | **DLL3-8** | VH | QVQLQEWGPGLVKPSETLSLTCTVSGASISSFYWSWIRQPPGKGLE WIGYIYYSGTTNYNPSLKSRVTISVDTSKNQLSLKLSSVTAADTAV YYCARIAVAGFFFDYWGQGTLVTVSSK |
| 78 | | **DLL3-8** | VL | EIVLTQSPGTLSLSPGERATLSCRASQSVNKNYLAWYQQKPGQAPR LLIYGASSRATGIPDRFSGSGSGTDFTLTISRLEPEDFAVYYCQQY DRSPLTFGGGTKVDIK |
| 79 | **EGF-3** | **DLL3-8** | scFv | QVQLQEWGPGLVKPSETLSLTCTVSGASISSFYWSWIRQPPGKGLE WIGYIYYSGTTNYNPSLKSRVTISVDTSKNQLSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKVDIK |
| 80 | | **DLL3-8 xI2C** | bispecific molecule | QVQLQEWGPGLVKPSETLSLTCTVSGASISSFYWSWIRQPPGKGLE WIGYIYYSGTTNYNPSLKSRVTISVDTSKNQLSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKVDIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVL |
| 81 | | **DLL3-9** | VH CDR1 | SFYWS |
| 82 | | **DLL3-9** | VH CDR2 | YIYYSGTTNYNPSLKS |
| 83 | | **DLL3-9** | VH CDR3 | IAVAGFFFDY |
| 84 | | **DLL3-9** | VL CDR1 | RASQSVNKNYLA |
| 85 | | **DLL3-9** | VL CDR2 | GASSRAT |
| 86 | | **DLL3-9** | VL CDR3 | QQYDRSPLT |
| 87 | | **DLL3-9** | VH | QVQLQESGPGLVKPSETLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSS |
| 88 | | **DLL3-9** | VL | EIVLTQSPGTLSLSPGESATLSCRASQSVNKNYLAWYQQKPGQAPR LLIYGASSRATGIPDRFSGSGSGTDFTLTISRLEPEDFAVYYCQQY DRSPLTFGGGTRLEIK |
| 89 | **EGF-3** | **DLL3-9** | scFv | QVQLQESGPGLVKPSETLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGESATLSCRASQSVNKNYLAWYQQKPGQAPRLLIYG ASSRATGIPDRFSGSGSGTDFTLTISRLEPEDFAVYYCQQYDRSPL TFGGGTRLEIK |
| 90 | | **DLL3-9 xI2C** | bispecific molecule | QVQLQESGPGLVKPSETLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGESATLSCRASQSVNKNYLAWYQQKPGQAPRLLIYG ASSRATGIPDRFSGSGSGTDFTLTISRLEPEDFAVYYCQQYDRSPL TFGGGTRLEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVL |
| 91 | | **DLL3-10** | VH CDR1 | SYYWS |
| 92 | | **DLL3-10** | VH CDR2 | YIFYNGITNYNPSLKS |
| 93 | | **DLL3-10** | VH CDR3 | IHSGSFSFDY |
| 94 | | **DLL3-10** | VL CDR1 | RASQSVSRGYLA |
| 95 | | **DLL3-10** | VL CDR2 | GASSRAT |
| 96 | | **DLL3-10** | VL CDR3 | QQYDTSPIT |
| 97 | | **DLL3-10** | VH | QVQLQESGPGLVKPSQTLSLTCTVSGGSISSYYWSWIRQPPGKGLE WIGYIFYNGITNYNPSLKSRVTISLDTSKNQFSLKLSSVTAADTAK YYCARIHSGSFSFDYWDQGTLVTVSS |
| 98 | | **DLL3-10** | VL | EIVMTQSPGTLSLSPGERATLSCRASQSVSRGYLAWYQQKPGQAPR LLIYGASSRATDIPDRFSGSGSGTDFTLTISRLEPEDFAVYYCQQY DTSPITFGQGTKVEIK |
| 99 | **EGF-3** | **DLL3-10** | scFv | QVQLQESGPGLVKPSQTLSLTCTVSGGSISSYYWSWIRQPPGKGLE WIGYIFYNGITNYNPSLKSRVTISLDTSKNQFSLKLSSVTAADTAK YYCARIHSGSFSFDYWDQGTLVTVSSGGGGSGGGGSGGGGSEIVMT |
| | | | | QSPGTLSLSPGERATLSCRASQSVSRGYLAWYQQKPGQAPRLLIYG ASSRATDIPDRFSGSGSGTDFTLTISRLEPEDFAVYYCQQYDTSPI TFGQGTKVEIK |
| 100 | | **DLL3-10 xI2C** | bispecific molecule | QVQLQESGPGLVKPSQTLSLTCTVSGGSISSYYWSWIRQPPGKGLE WIGYIFYNGITNYNPSLKSRVTISLDTSKNQFSLKLSSVTAADTAK YYCARIHSGSFSFDYWDQGTLVTVSSGGGGSGGGGSGGGGSEIVMT QSPGTLSLSPGERATLSCRASQSVSRGYLAWYQQKPGQAPRLLIYG ASSRATDIPDRFSGSGSGTDFTLTISRLEPEDFAVYYCQQYDTSPI TFGQGTKVEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVL |
| 101 | | **DLL3-11** | VH CDR1 | NAGMS |
| 102 | | **DLL3-11** | VH CDR2 | RIKNKIDGGTTDFAAPVKG |
| 103 | | **DLL3-11** | VH CDR3 | RGWYGDYFDY |
| 104 | | **DLL3-11** | VL CDR1 | RSSQSLLHSNGYNYLD |
| 105 | | **DLL3-11** | VL CDR2 | LGSNRAS |
| 106 | | **DLL3-11** | VL CDR3 | MQALQTPFT |
| 107 | | **DLL3-11** | VH | EVQLVESGGGLVKPGGSLRLSCAASGFIFNNAGMSWVRQAPGKGLE WVGRIKNKIDGGTTDFAAPVKGRFTISRDDSKNTLYLQMNSLKAED TAVYYCTARGWYGDYFDYWGQGTLVTVSS |
| 108 | | **DLL3-11** | VL | DIVMTQTPLSLPVTPGEPASISCRSSQSLLHSNGYNYLDWYLQKPG QSPQLLIYLGSNRASGVPDRFSGSGSGTDFTLKISRVEAEDVGIYY CMQALQTPFTFGPGTKVEIK |
| 109 | **EGF-3** | **DLL3-** | scFv | EVQLVESGGGLVKPGGSLRLSCAASGFIFNNAGMSWVRQAPGKGLE WVGRIKNKIDGGTTDFAAPVKGRFTISRDDSKNTLYLQMNSLKAED TAVYYCTARGWYGDYFDYWGQGTLVTVSSGGGGSGGGGSGGGGSDI VMTQTPLSLPVTPGEPASISCRSSQSLLHSNGYNYLDWYLQKPGQS PQLLIYLGSNRASGVPDRFSGSGSGTDFTLKISRVEAEDVGIYYCM QALQTPFTFGPGTKVEIK |
| 110 | | **DLL3-11 xI2C** | bispecific molecule | EVQLVESGGGLVKPGGSLRLSCAASGFIFNNAGMSWVRQAPGKGLE WVGRIKNKIDGGTTDFAAPVKGRFTISRDDSKNTLYLQMNSLKAED TAVYYCTARGWYGDYFDYWGQGTLVTVSSGGGGSGGGGSGGGGSDI VMTQTPLSLPVTPGEPASISCRSSQSLLHSNGYNYLDWYLQKPGQS PQLLIYLGSNRASGVPDRFSGSGSGTDFTLKISRVEAEDVGIYYCM QALQTPFTFGPGTKVEIKSGGGGSEVQLVESGGGLVQPGGSLKLSC AASGFTFNKYAMNWVRQAPGKGLEWVARIRSKYNNYATYYADSVKD RFTISRDDSKNTAYLQMNNLKTEDTAVYYCVRHGNFGNSYISYWAY WGQGTLVTVSSGGGGSGGGGSGGGGSQTVVTQEPSLTVSPGGTVTL TCGSSTGAVTSGNYPNWVQQKPGQAPRGLIGGTKFLAPGTPARFSG SLLGGKAALTLSGVQPEDEAEYYCVLWYSNRWVFGGGTKLTVL |
| 111 | | **DLL3-12** | VH CDR1 | SYDIH |
| 112 | | **DLL3-** | VH CDR2 | VISSHGSNKNYARSVKG |
| 113 | | **DLL3-12** | VH CDR3 | DGYSGNDPFYYYYHGMDV |
| 114 | | **DLL3-12** | VL CDR1 | RASQSISSYLN |
| 115 | | **DLL3-** | VL CDR2 | AASSLQS |
| 116 | | **DLL3-** | VL CDR3 | QQSFTTPLT |
| 117 | | **DLL3-12** | VH | QVQLVESGGGVVQPGRSLRLSCAASGFSFSSYDIHWVRQAPGKGLE WVAVISSHGSNKNYARSVKGRFTISRDNSKNTLYLQMNSLKAEDTA VYYCARDGYSGNDPFYYYYHGMDVWGQGTTVTVSS |
| 118 | | **DLL3-12** | VL | DIQMTQSPSSLSASVGDRVTITCRASQSISSYLNWYQQKPGKAPKL LIYAASSLQSGVPSRFSGSGSGTDFSLTISSLQPEDFATYYCQQSF TTPLTFGGGTKVEIK |
| 119 | **EGF-3/[4]** | **DLL3-12** | scFv | QVQLVESGGGVVQPGRSLRLSCAASGFSFSSYDIHWVRQAPGKGLE WVAVISSHGSNKNYARSVKGRFTISRDNSKNTLYLQMNSLKAEDTA VYYCARDGYSGNDPFYYYYHGMDVWGQGTTVTVSSGGGGSGGGGSG GGGSDIQMTQSPSSLSASVGDRVTITCRASQSISSYLNWYQQKPGK APKLLIYAASSLQSGVPSRFSGSGSGTDFSLTISSLQPEDFATYYC QQSFTTPLTFGGGTKVEIK |
| 120 | | **DLL3-12 xI2C** | bispecific molecule | QVQLVESGGGVVQPGRSLRLSCAASGFSFSSYDIHWVRQAPGKGLE WVAVISSHGSNKNYARSVKGRFTISRDNSKNTLYLQMNSLKAEDTA VYYCARDGYSGNDPFYYYYHGMDVWGQGTTVTVSSGGGGSGGGGSG GGGSDIQMTQSPSSLSASVGDRVTITCRASQSISSYLNWYQQKPGK APKLLIYAASSLQSGVPSRFSGSGSGTDFSLTISSLQPEDFATYYC QQSFTTPLTFGGGTKVEIKSGGGGSEVQLVESGGGLVQPGGSLKLS CAASGFTFNKYAMNWVRQAPGKGLEWVARIRSKYNNYATYYADSVK DRFTISRDDSKNTAYLQMNNLKTEDTAVYYCVRHGNFGNSYISYWA YWGQGTLVTVSSGGGGSGGGGSGGGGSQTVVTQEPSLTVSPGGTVT LTCGSSTGAVTSGNYPNWVQQKPGQAPRGLIGGTKFLAPGTPARFS GSLLGGKAALTLSGVQPEDEAEYYCVLWYSNRWVFGGGTKLTVL |
| 121 | | **DLL3-13** | VH CDR1 | SYYMH |
| 122 | | **DLL3-13** | VH CDR2 | IINPSDGSTNYAQNFQG |
| 123 | | **DLL3-13** | VH CDR3 | GGNSAFYSYYDMDV |
| 124 | | **DLL3-13** | VL CDR1 | RSSQSLVYRDGNTYLS |
| 125 | | **DLL3-13** | VL CDR2 | KVSNWQS |
| 126 | | **DLL3-13** | VL CDR3 | MQGTHWPPT |
| 127 | | **DLL3-13** | VH | QVQLVQSGAEVKKPGASVKVSCKASGYTFTSYYMHWVRQAPGQGLE WMGIINPSDGSTNYAQNFQGRVTMTRDTSTNTVYMELSSLRSEDTA VYYCARGGNSAFYSYYDMDVWGQGTTVTVSS |
| 128 | | **DLL3-13** | VL | DVVMTQSPLSLPVTLGQPASISCRSSQSLVYRDGNTYLSWFQQRPG QSPRRLIYKVSNWQSGVPDRFSGSGSGTDFTLKISRVEAEDVGVYF CMQGTHWPPTFGQGTKVEIK |
| 129 | **EGF-4** | **DLL3-13** | scFv | QVQLVQSGAEVKKPGASVKVSCKASGYTFTSYYMHWVRQAPGQGLE WMGIINPSDGSTNYAQNFQGRVTMTRDTSTNTVYMELSSLRSEDTA VYYCARGGNSAFYSYYDMDVWGQGTTVTVSSGGGGSGGGGSGGGGS |
| | | | | DVVMTQSPLSLPVTLGQPASISCRSSQSLVYRDGNTYLSWFQQRPG QSPRRLIYKVSNWQSGVPDRFSGSGSGTDFTLKISRVEAEDVGVYF CMQGTHWPPTFGQGTKVEIK |
| 130 | | **DLL3-13 xI2C** | bispecific molecule | QVQLVQSGAEVKKPGASVKVSCKASGYTFTSYYMHWVRQAPGQGLE WMGIINPSDGSTNYAQNFQGRVTMTRDTSTNTVYMELSSLRSEDTA VYYCARGGNSAFYSYYDMDVWGQGTTVTVSSGGGGSGGGGSGGGGS DVVMTQSPLSLPVTLGQPASISCRSSQSLVYRDGNTYLSWFQQRPG QSPRRLIYKVSNWQSGVPDRFSGSGSGTDFTLKISRVEAEDVGVYF CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 131 | | **DLL3-14** | VH CDR1 | NYYMH |
| 132 | | **DLL3-14** | VH CDR2 | IINPSDGSTSYAQKFQG |
| 133 | | **DLL3-14** | VH CDR3 | GGNSAFYSYYDMDV |
| 134 | | **DLL3-14** | VL CDR1 | RSSQSLVYRDGNTYLS |
| 135 | | **DLL3-14** | VL CDR2 | KVSNWQS |
| 136 | | **DLL3-14** | VL CDR3 | MQGTHWPPT |
| 137 | | **DLL3-14** | VH | QVQLVQSGAEVKKPGASVKVSCKASGYTFTNYYMHWVRQAPGLGLE WMGIINPSDGSTSYAQKFQGRVTMTRDTSTNTVYMDLSSLRSEDTA VYYCARGGNSAFYSYYDMDVWGQGTTVTVSS |
| 138 | | **DLL3-14** | VL | DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGQGTKVEIK |
| 139 | **EGF-4** | **DLL3-14** | scFv | QVQLVQSGAEVKKPGASVKVSCKASGYTFTNYYMHWVRQAPGLGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGQGTKVEIK |
| 140 | | **DLL3-14 xI2C** | bispecific molecule | QVQLVQSGAEVKKPGASVKVSCKASGYTFTNYYMHWVRQAPGLGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 141 | | **DLL3-** | VH CDR1 | GYYIH |
| 142 | | **DLL3-** | VH CDR2 | IINPSDGSTSYGQNFQG |
| 143 | | **DLL3-15** | VH CDR3 | GGNSAFYSYYDMDV |
| 144 | | **DLL3-15** | VL CDR1 | RSSQSLAYRDGNTYLS |
| 145 | | **DLL3-** | VL CDR2 | KVSNWQS |
| 146 | | **DLL3-** | VL CDR3 | MQGTHWPPT |
| 147 | | **DLL3-15** | VH | QVQLVQSGAEVKKPGASVKVSCKASGYTFTGYYIHWVRQAPGQGLE WMGIINPSDGSTSYGQNFQGRVTMTRDTSTNTVYMELSSLRSEDTA VYYCARGGNSAFYSYYDMDVWGQGTTVTVSS |
| 148 | | **DLL3-15** | VL | DVVMTQSPLSLPVTLGQPASISCRSSQSLAYRDGNTYLSWFQQRPG QSPRRLIYKVSNWQSGVPDRFSGSGSGTDFTLKISRVEAEDVGVYF CMQGTHWPPTFGQGTKVEIK |
| 149 | **EGF-4** | **DLL3-15** | scFv | QVQLVQSGAEVKKPGASVKVSCKASGYTFTGYYIHWVRQAPGQGLE WMGIINPSDGSTSYGQNFQGRVTMTRDTSTNTVYMELSSLRSEDTA VYYCARGGNSAFYSYYDMDVWGQGTTVTVSSGGGGSGGGGSGGGGS DVVMTQSPLSLPVTLGQPASISCRSSQSLAYRDGNTYLSWFQQRPG QSPRRLIYKVSNWQSGVPDRFSGSGSGTDFTLKISRVEAEDVGVYF CMQGTHWPPTFGQGTKVEIK |
| 150 | | **DLL3-15 xI2C** | bispecific molecule | QVQLVQSGAEVKKPGASVKVSCKASGYTFTGYYIHWVRQAPGQGLE WMGIINPSDGSTSYGQNFQGRVTMTRDTSTNTVYMELSSLRSEDTA VYYCARGGNSAFYSYYDMDVWGQGTTVTVSSGGGGSGGGGSGGGGS DVVMTQSPLSLPVTLGQPASISCRSSQSLAYRDGNTYLSWFQQRPG QSPRRLIYKVSNWQSGVPDRFSGSGSGTDFTLKISRVEAEDVGVYF CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 151 | | **DLL3-16** | VH CDR1 | GHYMH |
| 152 | | **DLL3-16** | VH CDR2 | IINPSDGSTNYAQKFQG |
| 153 | | **DLL3-16** | VH CDR3 | GTTWHYSYYDMDV |
| 154 | | **DLL3-16** | VL CDR1 | RSSQSLVYRDGNTYLT |
| 155 | | **DLL3-16** | VL CDR2 | KVSNWQS |
| 156 | | **DLL3-16** | VL CDR3 | MQGTHWPPT |
| 157 | | **DLL3-16** | VH | QVQLVQSGAEVKKPGASVKVSCKASGYTFTGHYMHWVRQAPGQGLE WMGIINPSDGSTNYAQKFQGRVTMTRDTSTSTVYMELRSLRSEDTA VYYCTRGTTVVHYSYYDMDVWGQGTTVTVSS |
| 158 | | **DLL3-16** | VL | DVVMTQTPLSLPVTLGQPASISCRSSQSLVYRDGNTYLTWFQQRPG QSPRRLIYKVSNWQSGVPDRFSGSGSGTDFTLKISRVEAEDVGVYY CMQGTHWPPTFGGGTKVEIK |
| 159 | **EGF-4** | **DLL3-16** | scFv | QVQLVQSGAEVKKPGASVKVSCKASGYTFTGHYMHWVRQAPGQGLE WMGIINPSDGSTNYAQKFQGRVTMTRDTSTSTVYMELRSLRSEDTA VYYCTRGTTVVHYSYYDMDVWGQGTTVTVSSGGGGSGGGGSGGGGS |
| | | | | DVVMTQTPLSLPVTLGQPASISCRSSQSLVYRDGNTYLTWFQQRPG QSPRRLIYKVSNWQSGVPDRFSGSGSGTDFTLKISRVEAEDVGVYY CMQGTHWPPTFGGGTKVEIK |
| 160 | | **DLL3-16 xI2C** | bispecific molecule | QVQLVQSGAEVKKPGASVKVSCKASGYTFTGHYMHWVRQAPGQGLE WMGIINPSDGSTNYAQKFQGRVTMTRDTSTSTVYMELRSLRSEDTA VYYCTRGTTVVHYSYYDMDVWGQGTTVTVSSGGGGSGGGGSGGGGS DVVMTQTPLSLPVTLGQPASISCRSSQSLVYRDGNTYLTWFQQRPG QSPRRLIYKVSNWQSGVPDRFSGSGSGTDFTLKISRVEAEDVGVYY CMQGTHWPPTFGGGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 161 | | **DLL3-17** | VH CDR1 | NYFMH |
| 162 | | **DLL3-17** | VH CDR2 | IINPSDGSTSYAQNFQG |
| 163 | | **DLL3-17** | VH CDR3 | GGNSAFYSYYDMDV |
| 164 | | **DLL3-17** | VL CDR1 | RSSQSLVYRDGNTYLS |
| 165 | | **DLL3-17** | VL CDR2 | RVSNWQS |
| 166 | | **DLL3-17** | VL CDR3 | MQGTYWPPT |
| 167 | | **DLL3-17** | VH | QVQLVQSGAEVKKPGASVKVSCKASGYTFTNYFMHWVRQAPGLGLE WMGIINPSDGSTSYAQNFQGRVTMTRDTSTNTVYMELSSLRSEDTA VYYCARGGNSAFYSYYDMDVWGQGTTVTVSS |
| 168 | | **DLL3-17** | VL | DVVMTQSPLSLPVTLGQPASISCRSSQSLVYRDGNTYLSWFQQRPG QSPRRLIYRVSNWQSGVPDRFSGSGSGTDFTLKISRVEAEDVGVYF CMQGTYWPPTFGQGTKVDIK |
| 169 | **EGF-4** | **DLL3-17** | scFv | QVQLVQSGAEVKKPGASVKVSCKASGYTFTNYFMHWVRQAPGLGLE WMGIINPSDGSTSYAQNFQGRVTMTRDTSTNTVYMELSSLRSEDTA VYYCARGGNSAFYSYYDMDVWGQGTTVTVSSGGGGSGGGGSGGGGS DVVMTQSPLSLPVTLGQPASISCRSSQSLVYRDGNTYLSWFQQRPG QSPRRLIYRVSNWQSGVPDRFSGSGSGTDFTLKISRVEAEDVGVYF CMQGTYWPPTFGQGTKVDIK |
| 170 | | **DLL3-17 xI2C** | bispecific molecule | QVQLVQSGAEVKKPGASVKVSCKASGYTFTNYFMHWVRQAPGLGLE WMGIINPSDGSTSYAQNFQGRVTMTRDTSTNTVYMELSSLRSEDTA VYYCARGGNSAFYSYYDMDVWGQGTTVTVSSGGGGSGGGGSGGGGS DVVMTQSPLSLPVTLGQPASISCRSSQSLVYRDGNTYLSWFQQRPG QSPRRLIYRVSNWQSGVPDRFSGSGSGTDFTLKISRVEAEDVGVYF CMQGTYWPPTFGQGTKVDIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 171 | | **DLL3-18** | VH CDR1 | NYGMH |
| 172 | | **DLL3-18** | VH CDR2 | VISHHGSSKYYARSVKG |
| 173 | | **DLL3-18** | VH CDR3 | DWWELVFDY |
| 174 | | **DLL3-18** | VL CDR1 | KSSQSLLHSDGKTFLY |
| 175 | | **DLL3-18** | VL CDR2 | EVSNRFS |
| 176 | | **DLL3-18** | VL CDR3 | LQGIHLPFT |
| 177 | | **DLL3-18** | VH | QVQLVESGGGAVQPGRSLRLSCAASGFTFSNYGMHWVRQAPGKGLE WVAVISHHGSSKYYARSVKGRFTISRDNSKNTLYLEMNSLRAEDTA VYYCARDWWELVFDYWGQGTLVTVSS |
| 178 | | **DLL3-18** | VL | DIVMTQTPLSLSVTPGQPASISCKSSQSLLHSDGKTFLYWYLQKPG QPPQLLIYEVSNRFSGVPDRFSGSGSGTDFTLKISRVEAEDVGVYY CLQGIHLPFTFGPGTKVEIK |
| 179 | **EGF-5/[6]** | **DLL3-18** | scFv | QVQLVESGGGAVQPGRSLRLSCAASGFTFSNYGMHWVRQAPGKGLE WVAVISHHGSSKYYARSVKGRFTISRDNSKNTLYLEMNSLRAEDTA VYYCARDWWELVFDYWGQGTLVTVSSGGGGSGGGGSGGGGSDIVMT QTPLSLSVTPGQPASISCKSSQSLLHSDGKTFLYWYLQKPGQPPQL LIYEVSNRFSGVPDRFSGSGSGTDFTLKISRVEAEDVGVYYCLQGI HLPFTFGPGTKVEIK |
| 180 | | **DLL3-18 xI2C** | bispecific molecule | QVQLVESGGGAVQPGRSLRLSCAASGFTFSNYGMHWVRQAPGKGLE WVAVISHHGSSKYYARSVKGRFTISRDNSKNTLYLEMNSLRAEDTA VYYCARDWWELVFDYWGQGTLVTVSSGGGGSGGGGSGGGGSDIVMT QTPLSLSVTPGQPASISCKSSQSLLHSDGKTFLYWYLQKPGQPPQL LIYEVSNRFSGVPDRFSGSGSGTDFTLKISRVEAEDVGVYYCLQGI HLPFTFGPGTKVEIKSGGGGSEVQLVESGGGLVQPGGSLKLSCAAS GFTFNKYAMNWVRQAPGKGLEWVARIRSKYNNYATYYADSVKDRFT ISRDDSKNTAYLQMNNLKTEDTAVYYCVRHGNFGNSYISYWAYWGQ GTLVTVSSGGGGSGGGGSGGGGSQTVVTQEPSLTVSPGGTVTLTCG SSTGAVTSGNYPNWVQQKPGQAPRGLIGGTKFLAPGTPARFSGSLL GGKAALTLSGVQPEDEAEYYCVLWYSNRWVFGGGTKLTVL |
| 181 | | **DLL3-19** | VH CDR1 | NSRMGVS |
| 182 | | **DLL3-19** | VH CDR2 | HIFSNDGKSYSTSLKS |
| 183 | | **DLL3-19** | VH CDR3 | YNYDSSGYYYSFFDY |
| 184 | | **DLL3-19** | VL CDR1 | RASQSISSYLN |
| 185 | | **DLL3-19** | VL CDR2 | AASSLQS |
| 186 | | **DLL3-19** | VL CDR3 | QQGYSSPFT |
| 187 | | **DLL3-19** | VH | QVTLKESGPMLVKPTETLTLTCTVSGFSLSNSRMGVSWIRQPPGRA LEWLAHIFSNDGKSYSTSLKSRLTISKDTSKSQVVLTMTNMDPVDT ATYYCARYNYDSSGYYYSFFDYWGQGTLVTVSS |
| 188 | | **DLL3-19** | VL | DIQMTQSPSSLSASVGDRVTITCRASQSISSYLNWYQQKPGKAPKL LIYAASSLQSGVPSRFSGSGSGTDFTLTISSLQPEDFATYYCQQGY SSPFTFGGGTKVEIK |
| 189 | **EGF-5/[6]** | **DLL3-19** | scFv | QVTLKESGPMLVKPTETLTLTCTVSGFSLSNSRMGVSWIRQPPGRA LEWLAHIFSNDGKSYSTSLKSRLTISKDTSKSQVVLTMTNMDPVDT ATYYCARYNYDSSGYYYSFFDYWGQGTLVTVSSGGGGSGGGGSGGG |
| | | | | GSDIQMTQSPSSLSASVGDRVTITCRASQSISSYLNWYQQKPGKAP KLLIYAASSLQSGVPSRFSGSGSGTDFTLTISSLQPEDFATYYCQQ GYSSPFTFGGGTKVEIK |
| 190 | | **DLL3-19 xI2C** | bispecific molecule | QVTLKESGPMLVKPTETLTLTCTVSGFSLSNSRMGVSWIRQPPGRA LEWLAHIFSNDGKSYSTSLKSRLTISKDTSKSQVVLTMTNMDPVDT ATYYCARYNYDSSGYYYSFFDYWGQGTLVTVSSGGGGSGGGGSGGG GSDIQMTQSPSSLSASVGDRVTITCRASQSISSYLNWYQQKPGKAP KLLIYAASSLQSGVPSRFSGSGSGTDFTLTISSLQPEDFATYYCQQ GYSSPFTFGGGTKVEIKSGGGGSEVQLVESGGGLVQPGGSLKLSCA ASGFTFNKYAMNWVRQAPGKGLEWVARIRSKYNNYATYYADSVKDR FTISRDDSKNTAYLQMNNLKTEDTAVYYCVRHGNFGNSYISYWAYW GQGTLVTVSSGGGGSGGGGSGGGGSQTVVTQEPSLTVSPGGTVTLT CGSSTGAVTSGNYPNWVQQKPGQAPRGLIGGTKFLAPGTPARFSGS LLGGKAALTLSGVQPEDEAEYYCVLWYSNRWVFGGGTKLTVL |
| 191 | | **DLL3-20** | VH CDR1 | NARMGVS |
| 192 | | **DLL3-20** | VH CDR2 | HIFSTDEKSYSTSLKS |
| 193 | | **DLL3-20** | VH CDR3 | YYYDSSGYYYSFFDY |
| 194 | | **DLL3-20** | VL CDR1 | RASQSIRSYLN |
| 195 | | **DLL3-20** | VL CDR2 | GASNLQS |
| 196 | | **DLL3-** | VL CDR3 | QQSYSSPFT |
| 197 | | **DLL3-20** | VH | QVTLKESGPVLVKPTETLTLTCTVSGFSLSNARMGVSWLRQPPGKA LEWLAHIFSTDEKSYSTSLKSRLTISKDTSKSQVVLTMTNMDPVDT ATYYCARYYYDSSGYYYSFFDYWGQGTLVTVSS |
| 198 | | **DLL3-20** | VL | DIQMTQSPSSLSASVGDRVTITCRASQSIRSYLNWYQQKPGKAPKL LIYGASNLQSGVPSRFSGSGSGTDFTLTISSLQPEDFATYYCQQSY SSPFTFGGGTKVEIK |
| 199 | **EGF-5/[6]** | **DLL3-20** | scFv | QVTLKESGPVLVKPTETLTLTCTVSGFSLSNARMGVSWLRQPPGKA LEWLAHIFSTDEKSYSTSLKSRLTISKDTSKSQVVLTMTNMDPVDT ATYYCARYYYDSSGYYYSFFDYWGQGTLVTVSSGGGGSGGGGSGGG GSDIQMTQSPSSLSASVGDRVTITCRASQSIRSYLNWYQQKPGKAP KLLIYGASNLQSGVPSRFSGSGSGTDFTLTISSLQPEDFATYYCQQ SYSSPFTFGGGTKVEIK |
| 200 | | **DLL3-20 xI2C** | bispecific molecule | QVTLKESGPVLVKPTETLTLTCTVSGFSLSNARMGVSWLRQPPGKA LEWLAHIFSTDEKSYSTSLKSRLTISKDTSKSQVVLTMTNMDPVDT ATYYCARYYYDSSGYYYSFFDYWGQGTLVTVSSGGGGSGGGGSGGG GSDIQMTQSPSSLSASVGDRVTITCRASQSIRSYLNWYQQKPGKAP KLLIYGASNLQSGVPSRFSGSGSGTDFTLTISSLQPEDFATYYCQQ SYSSPFTFGGGTKVEIKSGGGGSEVQLVESGGGLVQPGGSLKLSCA ASGFTFNKYAMNWVRQAPGKGLEWVARIRSKYNNYATYYADSVKDR FTISRDDSKNTAYLQMNNLKTEDTAVYYCVRHGNFGNSYISYWAYW GQGTLVTVSSGGGGSGGGGSGGGGSQTVVTQEPSLTVSPGGTVTLT CGSSTGAVTSGNYPNWVQQKPGQAPRGLIGGTKFLAPGTPARFSGS LLGGKAALTLSGVQPEDEAEYYCVLWYSNRWVFGGGTKLTVL |
| 201 | | **DLL3-** | VH CDR1 | SYYIH |
| 202 | | **DLL3-21** | VH CDR2 | IINPSGGSKSYAQKFRG |
| 203 | | **DLL3-21** | VH CDR3 | SMSTVTSDAFDI |
| 204 | | **DLL3-21** | VL CDR1 | RASQSISNYLN |
| 205 | | **DLL3-21** | VL CDR2 | AASSLQS |
| 206 | | **DLL3-21** | VL CDR3 | QQSYSAPLT |
| 207 | | **DLL3-21** | VH | QVQLVQSGAEVKKPGASVKVSCKASGYAFTSYYIHWVRQAPGQGLE WMGIINPSGGSKSYAQKFRGRVTMTRDTSTSTVYMELSSLTSEDTA VYYCARSMSTVTSDAFDIWGQGTMVTVSS |
| 208 | | **DLL3-** | VL | DIQMTQSPSSLSASVGDRVTITCRASQSISNYLNWYQQKPGKAPKL LIYAASSLQSGVPSRFSGSGSGTEFTLTISSLQPEDFATYYCQQSY SAPLTFGGGTKVDIK |
| 209 | **EGF-5/[6]** | **DLL3-21** | scFv | QVQLVQSGAEVKKPGASVKVSCKASGYAFTSYYIHWVRQAPGQGLE WMGIINPSGGSKSYAQKFRGRVTMTRDTSTSTVYMELSSLTSEDTA VYYCARSMSTVTSDAFDIWGQGTMVTVSSGGGGSGGGGSGGGGSDI QMTQSPSSLSASVGDRVTITCRASQSISNYLNWYQQKPGKAPKLLI YAASSLQSGVPSRFSGSGSGTEFTLTISSLQPEDFATYYCQQSYSA PLTFGGGTKVDIK |
| 210 | | **DLL3-21 xI2C** | bispecific molecule | QVQLVQSGAEVKKPGASVKVSCKASGYAFTSYYIHWVRQAPGQGLE WMGIINPSGGSKSYAQKFRGRVTMTRDTSTSTVYMELSSLTSEDTA VYYCARSMSTVTSDAFDIWGQGTMVTVSSGGGGSGGGGSGGGGSDI QMTQSPSSLSASVGDRVTITCRASQSISNYLNWYQQKPGKAPKLLI YAASSLQSGVPSRFSGSGSGTEFTLTISSLQPEDFATYYCQQSYSA PLTFGGGTKVDIKSGGGGSEVQLVESGGGLVQPGGSLKLSCAASGF TFNKYAMNWVRQAPGKGLEWVARIRSKYNNYATYYADSVKDRFTIS RDDSKNTAYLQMNNLKTEDTAVYYCVRHGNFGNSYISYWAYWGQGT LVTVSSGGGGSGGGGSGGGGSQTVVTQEPSLTVSPGGTVTLTCGSS TGAVTSGNYPNWVQQKPGQAPRGLIGGTKFLAPGTPARFSGSLLGG KAALTLSGVQPEDEAEYYCVLWYSNRWVFGGGTKLTVL |
| 211 | **EGF-3** | **DLL3-4 xF12Q** | bispecific molecule | QVQLQESGPGLVKPSETLSLTCTVSGGSISSYYWSWIRQPPGKGLE WIGYVYYSGTTNYNPSLKSRVTISVDTSKNQFSLKLSSVTAADTAV YYCASIAVTGFYFDYWGQGTLVTVSSGGGGSGGGGSGGGGSEIVLT QSPGTLSLSPGERVTLSCRASQRVNNNYLAWYQQRPGQAPRLLIYG ASSRATGIPDRFSGSGSGTDFTLTISRLEPEDFAVYYCQQYDRSPL TFGGGTKLEIKSGGGGSEVQLVESGGGLVQPGGSLRLSCAASGFTF NSYAMNWVRQAPGKGLEWVARIRSKYNNYATYYADSVKGRFTISRD DSKNTAYLQMNSLKTEDTAVYYCVRHGNFGNSYVSWWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVL |
| 212 | **EGF-3** | **DLL3-5 xF12Q** | bispecific molecule | QVQLQESGPGLVKPSETLSLTCTVSGGSISSYYWSWIRQPPGKGLE WIGYIYYSGRTNYYPSLKSRVTISIDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKLEIKSGGGGSEVQLVESGGGLVQPGGSLRLSCAASGFTF NSYAMNWVRQAPGKGLEWVARIRSKYNNYATYYADSVKGRFTISRD DSKNTAYLQMNSLKTEDTAVYYCVRHGNFGNSYVSWWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVL |
| 213 | **EGF-3** | **DLL3-6 xF12Q** | bispecific molecule | QVQLQESGPGLVKPSETLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT |
| | | | | QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKVEIKSGGGGSEVQLVESGGGLVQPGGSLRLSCAASGFTF NSYAMNWVRQAPGKGLEWVARIRSKYNNYATYYADSVKGRFTISRD DSKNTAYLQMNSLKTEDTAVYYCVRHGNFGNSYVSWWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVL |
| 214 | **EGF-3** | **DLL3-7 xF12Q** | bispecific molecule | QVQLQESGPGLVKPSQTLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKVEIKSGGGGSEVQLVESGGGLVQPGGSLRLSCAASGFTF NSYAMNWVRQAPGKGLEWVARIRSKYNNYATYYADSVKGRFTISRD DSKNTAYLQMNSLKTEDTAVYYCVRHGNFGNSYVSWWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVL |
| 215 | **EGF-3** | **DLL3-8 xF12Q** | bispecific molecule | QVQLQEWGPGLVKPSETLSLTCTVSGASISSFYWSWIRQPPGKGLE WIGYIYYSGTTNYNPSLKSRVTISVDTSKNQLSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKVDIKSGGGGSEVQLVESGGGLVQPGGSLRLSCAASGFTF NSYAMNWVRQAPGKGLEWVARIRSKYNNYATYYADSVKGRFTISRD DSKNTAYLQMNSLKTEDTAVYYCVRHGNFGNSYVSWWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVL |
| 216 | **EGF-3** | **DLL3-9 xF12Q** | bispecific molecule | QVQLQESGPGLVKPSETLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGESATLSCRASQSVNKNYLAWYQQKPGQAPRLLIYG ASSRATGIPDRFSGSGSGTDFTLTISRLEPEDFAVYYCQQYDRSPL TFGGGTRLEIKSGGGGSEVQLVESGGGLVQPGGSLRLSCAASGFTF NSYAMNWVRQAPGKGLEWVARIRSKYNNYATYYADSVKGRFTISRD DSKNTAYLQMNSLKTEDTAVYYCVRHGNFGNSYVSWWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVL |
| 217 | **EGF-3** | **DLL3-10 xF12Q** | bispecific molecule | QVQLQESGPGLVKPSQTLSLTCTVSGGSISSYYWSWIRQPPGKGLE WIGYIFYNGITNYNPSLKSRVTISLDTSKNQFSLKLSSVTAADTAK YYCARIHSGSFSFDYWDQGTLVTVSSGGGGSGGGGSGGGGSEIVMT QSPGTLSLSPGERATLSCRASQSVSRGYLAWYQQKPGQAPRLLIYG ASSRATDIPDRFSGSGSGTDFTLTISRLEPEDFAVYYCQQYDTSPI TFGQGTKVEIKSGGGGSEVQLVESGGGLVQPGGSLRLSCAASGFTF NSYAMNWVRQAPGKGLEWVARIRSKYNNYATYYADSVKGRFTISRD DSKNTAYLQMNSLKTEDTAVYYCVRHGNFGNSYVSWWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVL |
| 218 | **EGF-4** | **DLL3-13 xF12Q** | bispecific molecule | QVQLVQSGAEVKKPGASVKVSCKASGYTFTSYYMHWVRQAPGQGLE WMGIINPSDGSTNYAQNFQGRVTMTRDTSTNTVYMELSSLRSEDTA VYYCARGGNSAFYSYYDMDVWGQGTTVTVSSGGGGSGGGGSGGGGS DVVMTQSPLSLPVTLGQPASISCRSSQSLVYRDGNTYLSWFQQRPG QSPRRLIYKVSNWQSGVPDRFSGSGSGTDFTLKISRVEAEDVGVYF CMQGTHWPPTFGQGTKVEIKSGGGGSEVQLVESGGGLVQPGGSLRL SCAASGFTFNSYAMNWVRQAPGKGLEWVARIRSKYNNYATYYADSV |
| | | | | KGRFTISRDDSKNTAYLQMNSLKTEDTAVYYCVRHGNFGNSYVSWW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 219 | **EGF-4** | **DLL3-14 xF12Q** | bispecific molecule | QVQLVQSGAEVKKPGASVKVSCKASGYTFTNYYMHWVRQAPGLGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGQGTKVEIKSGGGGSEVQLVESGGGLVQPGGSLRL SCAASGFTFNSYAMNWVRQAPGKGLEWVARIRSKYNNYATYYADSV KGRFTISRDDSKNTAYLQMNSLKTEDTAVYYCVRHGNFGNSYVSWW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 220 | **EGF-4** | **DLL3-15 xF12Q** | bispecific molecule | QVQLVQSGAEVKKPGASVKVSCKASGYTFTGYYIHWVRQAPGQGLE WMGIINPSDGSTSYGQNFQGRVTMTRDTSTNTVYMELSSLRSEDTA VYYCARGGNSAFYSYYDMDVWGQGTTVTVSSGGGGSGGGGSGGGGS DVVMTQSPLSLPVTLGQPASISCRSSQSLAYRDGNTYLSWFQQRPG QSPRRLIYKVSNWQSGVPDRFSGSGSGTDFTLKISRVEAEDVGVYF CMQGTHWPPTFGQGTKVEIKSGGGGSEVQLVESGGGLVQPGGSLRL SCAASGFTFNSYAMNWVRQAPGKGLEWVARIRSKYNNYATYYADSV KGRFTISRDDSKNTAYLQMNSLKTEDTAVYYCVRHGNFGNSYVSWW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 221 | **N-term.** | **DLL3-1 xI2C - HALB variant 1** | bispecific molecule -HALB variant 1 | QVQLVESGGGVVQSGRSLRLSCAASGFTFSDYGIHWVRQAPGKGLE WVAVISYHGSNKYYARSVKGRFTVSRDNSKNTLYLQMNSLRAEDTA VYYCAREIPFGMDVWGQGTTVTVSSGGGGSGGGGSGGGGSDIVMTQ TPLSLPVTPGEPASISCRSSQSLLHSDGYNYLDWYLQKPGQSPQLL IYLGSNRASGVPDRFSGSGSGTDFTLTISRVEAEDVGVYYCMQALQ TPLTFGGGTKVDIKSGGGGSEVQLVESGGGLVQPGGSLKLSCAASG FTFNKYAMNWVRQAPGKGLEWVARIRSKYNNYATYYADSVKDRFTI SRDDSKNTAYLQMNNLKTEDTAVYYCVRHGNFGNSYISYWAYWGQG TLVTVSSGGGGSGGGGSGGGGSQTVVTQEPSLTVSPGGTVTLTCGS STGAVTSGNYPNWVQQKPGQAPRGLIGGTKFLAPGTPARFSGSLLG GKAALTLSGVQPEDEAEYYCVLWYSNRWVFGGGTKLTVLPGGGGSD AHKSEVAHRFKDLGEENFKALVLIAFAQYLQQCPFEDHVKLVNEVT EFAKTCVADESAENCDKSLHTLFGDKLCTVATLRETYGEMADCCAK QEPERNECFLQHKDDNPNLPRLVRPEVDVMCTAFHDNEETFLKKYL YEIARRHPYFYAPELLFFAKRYKAAFTECCQAADKAACLLPKLDEL RDEGKASSAKQRLKCASLQKFGERAFKAWAVARLSQRFPKAEFAEV SKLVTDLTKVHTECCHGDLLECADDRADLAKYICENQDSISSKLKE CCEKPLLEKSHCIAEVENDEMPADLPSLAADFVESKDVCKNYAEAK DVFLGMFLYEYARRHPDYSVVLLLRLAKTYETTLEKCCAAADPHEC YAKVFDEFKPLVEEPQNLIKQNCELFEQLGEYKFQNALLVRYTKKV PQVSTPTLVEVSRNLGKVGSKCCKHPEAKRMPCAEDYLSVVLNQLC VLHEKTPVSDRVTKCCTESLVNRRPCFSALEVDETYVPKEFNAGTF TFHADICTLSEKERQIKKQTALVELVKHKPKATKEQLKAAMDDFAA FVEKCCKADDKETCFAEEGKKLVAASQAALGLHHHHHH |
| 222 | **N-term.** | **DLL3-2 xI2C - HALB variant 1** | bispecific molecule -HALB variant 1 | QVQLVQSGAEVKKPGASVKVSCKASGYTFTGYYMHWVRQAPGQGLE WMGWINPNSGDTNYAQKFQGRVTMTRDTSISTAYMELSRLTSDDTA VYYCARDANIAALDAFEIWGQGTMVTVSSGGGGSGGGGSGGGGSDI QMTQSPSSLSASVGDRVTITCRASQSISSYLNWYQQKPGKAPKLLI YAASSLQSGVPSRFSGSGSGTDFTLTISSLQPEDFATYYCQQSYST PLTFGGGTKVEIKSGGGGSEVQLVESGGGLVQPGGSLKLSCAASGF TFNKYAMNWVRQAPGKGLEWVARIRSKYNNYATYYADSVKDRFTIS RDDSKNTAYLQMNNLKTEDTAVYYCVRHGNFGNSYISYWAYWGQGT LVTVSSGGGGSGGGGSGGGGSQTVVTQEPSLTVSPGGTVTLTCGSS |
| | | | | TGAVTSGNYPNWVQQKPGQAPRGLIGGTKFLAPGTPARFSGSLLGG KAALTLSGVQPEDEAEYYCVLWYSNRWVFGGGTKLTVLPGGGGSDA HKSEVAHRFKDLGEENFKALVLIAFAQYLQQCPFEDHVKLVNEVTE FAKTCVADESAENCDKSLHTLFGDKLCTVATLRETYGEMADCCAKQ EPERNECFLQHKDDNPNLPRLVRPEVDVMCTAFHDNEETFLKKYLY EIARRHPYFYAPELLFFAKRYKAAFTECCQAADKAACLLPKLDELR DEGKASSAKQRLKCASLQKFGERAFKAWAVARLSQRFPKAEFAEVS KLVTDLTKVHTECCHGDLLECADDRADLAKYICENQDSISSKLKEC CEKPLLEKSHCIAEVENDEMPADLPSLAADFVESKDVCKNYAEAKD VFLGMFLYEYARRHPDYSVVLLLRLAKTYETTLEKCCAAADPHECY AKVFDEFKPLVEEPQNLIKQNCELFEQLGEYKFQNALLVRYTKKVP QVSTPTLVEVSRNLGKVGSKCCKHPEAKRMPCAEDYLSVVLNQLCV LHEKTPVSDRVTKCCTESLVNRRPCFSALEVDETYVPKEFNAGTFT FHADICTLSEKERQIKKQTALVELVKHKPKATKEQLKAAMDDFAAF VEKCCKADDKETCFAEEGKKLVAASQAALGLHHHHHH |
| 223 | **EGF-1** | **DLL3-3 xI2C - HALB** | bispecific molecule -HALB | QVQLVESGGGVVQPGRSLRLSCAASGFTFSSYGMHWVRQAPGKGLE WVAVISYHGRDTYYARSVKGRFTISRDNSKNTLYLHMNSLRAEDTA VYYCARDGATVTSYYYSGMDVWGQGTTVTVSSGGGGSGGGGSGGGG SDIQMTQSPSSLSASVGDRVTITCRASQGISNYLAWFQQKPGKAPK SLIYLASSLQSGVPSKFSGSGSGTDFTLTISSLQPEDFATYYCQQY NFYPFTFGPGTKVDIKSGGGGSEVQLVESGGGLVQPGGSLKLSCAA SGFTFNKYAMNWVRQAPGKGLEWVARIRSKYNNYATYYADSVKDRF TISRDDSKNTAYLQMNNLKTEDTAVYYCVRHGNFGNSYISYWAYWG QGTLVTVSSGGGGSGGGGSGGGGSQTVVTQEPSLTVSPGGTVTLTC GSSTGAVTSGNYPNWVQQKPGQAPRGLIGGTKFLAPGTPARFSGSL LGGKAALTLSGVQPEDEAEYYCVLWYSNRWVFGGGTKLTVLPGGGG SDAHKSEVAHRFKDLGEENFKALVLIAFAQYLQQCPFEDHVKLVNE VTEFAKTCVADESAENCDKSLHTLFGDKLCTVATLRETYGEMADCC AKQEPERNECFLQHKDDNPNLPRLVRPEVDVMCTAFHDNEETFLKK YLYEIARRHPYFYAPELLFFAKRYKAAFTECCQAADKAACLLPKLD ELRDEGKASSAKQRLKCASLQKFGERAFKAWAVARLSQRFPKAEFA EVSKLVTDLTKVHTECCHGDLLECADDRADLAKYICENQDSISSKL KECCEKPLLEKSHCIAEVENDEMPADLPSLAADFVESKDVCKNYAE AKDVFLGMFLYEYARRHPDYSVVLLLRLAKTYETTLEKCCAAADPH ECYAKVFDEFKPLVEEPQNLIKQNCELFEQLGEYKFQNALLVRYTK KVPQVSTPTLVEVSRNLGKVGSKCCKHPEAKRMPCAEDYLSVVLNQ LCVLHEKTPVSDRVTKCCTESLVNRRPCFSALEVDETYVPKEFNAE TFTFHADICTLSEKERQIKKQTALVELVKHKPKATKEQLKAVMDDF AAFVEKCCKADDKETCFAEEGKKLVAASQAALGLHHHHHH |
| 224 | **EGF-3** | **DLL3-4 xI2C** - **HALB** | bispecific molecule -HALB | QVQLQESGPGLVKPSETLSLTCTVSGGSISSYYWSWIRQPPGKGLE WIGYVYYSGTTNYNPSLKSRVTISVDTSKNQFSLKLSSVTAADTAV YYCASIAVTGFYFDYWGQGTLVTVSSGGGGSGGGGSGGGGSEIVLT QSPGTLSLSPGERVTLSCRASQRVNNNYLAWYQQRPGQAPRLLIYG ASSRATGIPDRFSGSGSGTDFTLTISRLEPEDFAVYYCQQYDRSPL TFGGGTKLEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLPGGGGSDAHK SEVAHRFKDLGEENFKALVLIAFAQYLQQCPFEDHVKLVNEVTEFA KTCVADESAENCDKSLHTLFGDKLCTVATLRETYGEMADCCAKQEP ERNECFLQHKDDNPNLPRLVRPEVDVMCTAFHDNEETFLKKYLYEI ARRHPYFYAPELLFFAKRYKAAFTECCQAADKAACLLPKLDELRDE GKASSAKQRLKCASLQKFGERAFKAWAVARLSQRFPKAEFAEVSKL VTDLTKVHTECCHGDLLECADDRADLAKYICENQDSISSKLKECCE KPLLEKSHCIAEVENDEMPADLPSLAADFVESKDVCKNYAEAKDVF LGMFLYEYARRHPDYSVVLLLRLAKTYETTLEKCCAAADPHECYAK VFDEFKPLVEEPQNLIKQNCELFEQLGEYKFQNALLVRYTKKVPQV |
| | | | | STPTLVEVSRNLGKVGSKCCKHPEAKRMPCAEDYLSVVLNQLCVLH EKTPVSDRVTKCCTESLVNRRPCFSALEVDETYVPKEFNAETFTFH ADICTLSEKERQIKKQTALVELVKHKPKATKEQLKAVMDDFAAFVE KCCKADDKETCFAEEGKKLVAASQAALGLHHHHHH |
| 225 | **EGF-3** | **DLL3-5 xI2C - HALB** | bispecific molecule -HALB | QVQLQESGPGLVKPSETLSLTCTVSGGSISSYYWSWIRQPPGKGLE WIGYIYYSGRTNYYPSLKSRVTISIDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKLEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLPGGGGSDAHK SEVAHRFKDLGEENFKALVLIAFAQYLQQCPFEDHVKLVNEVTEFA KTCVADESAENCDKSLHTLFGDKLCTVATLRETYGEMADCCAKQEP ERNECFLQHKDDNPNLPRLVRPEVDVMCTAFHDNEETFLKKYLYEI ARRHPYFYAPELLFFAKRYKAAFTECCQAADKAACLLPKLDELRDE GKASSAKQRLKCASLQKFGERAFKAWAVARLSQRFPKAEFAEVSKL VTDLTKVHTECCHGDLLECADDRADLAKYICENQDSISSKLKECCE KPLLEKSHCIAEVENDEMPADLPSLAADFVESKDVCKNYAEAKDVF LGMFLYEYARRHPDYSVVLLLRLAKTYETTLEKCCAAADPHECYAK VFDEFKPLVEEPQNLIKQNCELFEQLGEYKFQNALLVRYTKKVPQV STPTLVEVSRNLGKVGSKCCKHPEAKRMPCAEDYLSVVLNQLCVLH EKTPVSDRVTKCCTESLVNRRPCFSALEVDETYVPKEFNAETFTFH ADICTLSEKERQIKKQTALVELVKHKPKATKEQLKAVMDDFAAFVE KCCKADDKETCFAEEGKKLVAASQAALGLHHHHHH |
| 226 | **EGF-3** | **DLL3-6 xI2C - HALB** | bispecific molecule -HALB | QVQLQESGPGLVKPSETLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKVEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLPGGGGSDAHK SEVAHRFKDLGEENFKALVLIAFAQYLQQCPFEDHVKLVNEVTEFA KTCVADESAENCDKSLHTLFGDKLCTVATLRETYGEMADCCAKQEP ERNECFLQHKDDNPNLPRLVRPEVDVMCTAFHDNEETFLKKYLYEI ARRHPYFYAPELLFFAKRYKAAFTECCQAADKAACLLPKLDELRDE GKASSAKQRLKCASLQKFGERAFKAWAVARLSQRFPKAEFAEVSKL VTDLTKVHTECCHGDLLECADDRADLAKYICENQDSISSKLKECCE KPLLEKSHCIAEVENDEMPADLPSLAADFVESKDVCKNYAEAKDVF LGMFLYEYARRHPDYSVVLLLRLAKTYETTLEKCCAAADPHECYAK VFDEFKPLVEEPQNLIKQNCELFEQLGEYKFQNALLVRYTKKVPQV STPTLVEVSRNLGKVGSKCCKHPEAKRMPCAEDYLSVVLNQLCVLH EKTPVSDRVTKCCTESLVNRRPCFSALEVDETYVPKEFNAETFTFH ADICTLSEKERQIKKQTALVELVKHKPKATKEQLKAVMDDFAAFVE KCCKADDKETCFAEEGKKLVAASQAALGLHHHHHH |
| 227 | **EGF-3** | **DLL3-7 xI2C - HALB** | bispecific molecule -HALB | QVQLQESGPGLVKPSQTLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKVEIKSGGGGSEVQLVESGGGLVQPGGSLKLSCAASGFTF NKYAMNWVRQAPGKGLEWVARIRSKYNNYATYYADSVKDRFTISRD |
| | | | | DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLPGGGGSDAHK SEVAHRFKDLGEENFKALVLIAFAQYLQQCPFEDHVKLVNEVTEFA KTCVADESAENCDKSLHTLFGDKLCTVATLRETYGEMADCCAKQEP ERNECFLQHKDDNPNLPRLVRPEVDVMCTAFHDNEETFLKKYLYEI ARRHPYFYAPELLFFAKRYKAAFTECCQAADKAACLLPKLDELRDE GKASSAKQRLKCASLQKFGERAFKAWAVARLSQRFPKAEFAEVSKL VTDLTKVHTECCHGDLLECADDRADLAKYICENQDSISSKLKECCE KPLLEKSHCIAEVENDEMPADLPSLAADFVESKDVCKNYAEAKDVF LGMFLYEYARRHPDYSVVLLLRLAKTYETTLEKCCAAADPHECYAK VFDEFKPLVEEPQNLIKQNCELFEQLGEYKFQNALLVRYTKKVPQV STPTLVEVSRNLGKVGSKCCKHPEAKRMPCAEDYLSVVLNQLCVLH EKTPVSDRVTKCCTESLVNRRPCFSALEVDETYVPKEFNAETFTFH ADICTLSEKERQIKKQTALVELVKHKPKATKEQLKAVMDDFAAFVE KCCKADDKETCFAEEGKKLVAASQAALGLHHHHHH |
| 228 | **EGF-3** | **DLL3-8 xI2C - HALB** | bispecific molecule -HALB | QVQLQEWGPGLVKPSETLSLTCTVSGASISSFYWSWIRQPPGKGLE WIGYIYYSGTTNYNPSLKSRVTISVDTSKNQLSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKVDIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLPGGGGSDAHK SEVAHRFKDLGEENFKALVLIAFAQYLQQCPFEDHVKLVNEVTEFA KTCVADESAENCDKSLHTLFGDKLCTVATLRETYGEMADCCAKQEP ERNECFLQHKDDNPNLPRLVRPEVDVMCTAFHDNEETFLKKYLYEI ARRHPYFYAPELLFFAKRYKAAFTECCQAADKAACLLPKLDELRDE GKASSAKQRLKCASLQKFGERAFKAWAVARLSQRFPKAEFAEVSKL VTDLTKVHTECCHGDLLECADDRADLAKYICENQDSISSKLKECCE KPLLEKSHCIAEVENDEMPADLPSLAADFVESKDVCKNYAEAKDVF LGMFLYEYARRHPDYSVVLLLRLAKTYETTLEKCCAAADPHECYAK VFDEFKPLVEEPQNLIKQNCELFEQLGEYKFQNALLVRYTKKVPQV STPTLVEVSRNLGKVGSKCCKHPEAKRMPCAEDYLSVVLNQLCVLH EKTPVSDRVTKCCTESLVNRRPCFSALEVDETYVPKEFNAETFTFH ADICTLSEKERQIKKQTALVELVKHKPKATKEQLKAVMDDFAAFVE KCCKADDKETCFAEEGKKLVAASQAALGLHHHHHH |
| 229 | **EGF-3** | **DLL3-9 xI2C - HALB** | bispecific molecule -HALB | QVQLQESGPGLVKPSETLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGESATLSCRASQSVNKNYLAWYQQKPGQAPRLLIYG ASSRATGIPDRFSGSGSGTDFTLTISRLEPEDFAVYYCQQYDRSPL TFGGGTRLEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLPGGGGSDAHK SEVAHRFKDLGEENFKALVLIAFAQYLQQCPFEDHVKLVNEVTEFA KTCVADESAENCDKSLHTLFGDKLCTVATLRETYGEMADCCAKQEP ERNECFLQHKDDNPNLPRLVRPEVDVMCTAFHDNEETFLKKYLYEI ARRHPYFYAPELLFFAKRYKAAFTECCQAADKAACLLPKLDELRDE GKASSAKQRLKCASLQKFGERAFKAWAVARLSQRFPKAEFAEVSKL VTDLTKVHTECCHGDLLECADDRADLAKYICENQDSISSKLKECCE KPLLEKSHCIAEVENDEMPADLPSLAADFVESKDVCKNYAEAKDVF |
| | | | | LGMFLYEYARRHPDYSVVLLLRLAKTYETTLEKCCAAADPHECYAK VFDEFKPLVEEPQNLIKQNCELFEQLGEYKFQNALLVRYTKKVPQV STPTLVEVSRNLGKVGSKCCKHPEAKRMPCAEDYLSVVLNQLCVLH EKTPVSDRVTKCCTESLVNRRPCFSALEVDETYVPKEFNAETFTFH ADICTLSEKERQIKKQTALVELVKHKPKATKEQLKAVMDDFAAFVE KCCKADDKETCFAEEGKKLVAASQAALGLHHHHHH |
| 230 | **EGF-3** | **DLL3-10 xI2C - HALB** | bispecific molecule -HALB | QVQLQESGPGLVKPSQTLSLTCTVSGGSISSYYWSWIRQPPGKGLE WIGYIFYNGITNYNPSLKSRVTISLDTSKNQFSLKLSSVTAADTAK YYCARIHSGSFSFDYWDQGTLVTVSSGGGGSGGGGSGGGGSEIVMT QSPGTLSLSPGERATLSCRASQSVSRGYLAWYQQKPGQAPRLLIYG ASSRATDIPDRFSGSGSGTDFTLTISRLEPEDFAVYYCQQYDTSPI TFGQGTKVEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLPGGGGSDAHK SEVAHRFKDLGEENFKALVLIAFAQYLQQCPFEDHVKLVNEVTEFA KTCVADESAENCDKSLHTLFGDKLCTVATLRETYGEMADCCAKQEP ERNECFLQHKDDNPNLPRLVRPEVDVMCTAFHDNEETFLKKYLYEI ARRHPYFYAPELLFFAKRYKAAFTECCQAADKAACLLPKLDELRDE GKASSAKQRLKCASLQKFGERAFKAWAVARLSQRFPKAEFAEVSKL VTDLTKVHTECCHGDLLECADDRADLAKYICENQDSISSKLKECCE KPLLEKSHCIAEVENDEMPADLPSLAADFVESKDVCKNYAEAKDVF LGMFLYEYARRHPDYSVVLLLRLAKTYETTLEKCCAAADPHECYAK VFDEFKPLVEEPQNLIKQNCELFEQLGEYKFQNALLVRYTKKVPQV STPTLVEVSRNLGKVGSKCCKHPEAKRMPCAEDYLSVVLNQLCVLH EKTPVSDRVTKCCTESLVNRRPCFSALEVDETYVPKEFNAETFTFH ADICTLSEKERQIKKQTALVELVKHKPKATKEQLKAVMDDFAAFVE KCCKADDKETCFAEEGKKLVAASQAALGLHHHHHH |
| 231 | **EGF-3** | **DLL3-11 xI2C - HALB** | bispecific molecule -HALB | EVQLVESGGGLVKPGGSLRLSCAASGFIFNNAGMSWVRQAPGKGLE WVGRIKNKIDGGTTDFAAPVKGRFTISRDDSKNTLYLQMNSLKAED TAVYYCTARGWYGDYFDYWGQGTLVTVSSGGGGSGGGGSGGGGSDI VMTQTPLSLPVTPGEPASISCRSSQSLLHSNGYNYLDWYLQKPGQS PQLLIYLGSNRASGVPDRFSGSGSGTDFTLKISRVEAEDVGIYYCM QALQTPFTFGPGTKVEIKSGGGGSEVQLVESGGGLVQPGGSLKLSC AASGFTFNKYAMNWVRQAPGKGLEWVARIRSKYNNYATYYADSVKD RFTISRDDSKNTAYLQMNNLKTEDTAVYYCVRHGNFGNSYISYWAY WGQGTLVTVSSGGGGSGGGGSGGGGSQTVVTQEPSLTVSPGGTVTL TCGSSTGAVTSGNYPNWVQQKPGQAPRGLIGGTKFLAPGTPARFSG SLLGGKAALTLSGVQPEDEAEYYCVLWYSNRWVFGGGTKLTVLPGG GGSDAHKSEVAHRFKDLGEENFKALVLIAFAQYLQQCPFEDHVKLV NEVTEFAKTCVADESAENCDKSLHTLFGDKLCTVATLRETYGEMAD CCAKQEPERNECFLQHKDDNPNLPRLVRPEVDVMCTAFHDNEETFL KKYLYEIARRHPYFYAPELLFFAKRYKAAFTECCQAADKAACLLPK LDELRDEGKASSAKQRLKCASLQKFGERAFKAWAVARLSQRFPKAE FAEVSKLVTDLTKVHTECCHGDLLECADDRADLAKYICENQDSISS KLKECCEKPLLEKSHCIAEVENDEMPADLPSLAADFVESKDVCKNY AEAKDVFLGMFLYEYARRHPDYSVVLLLRLAKTYETTLEKCCAAAD PHECYAKVFDEFKPLVEEPQNLIKQNCELFEQLGEYKFQNALLVRY TKKVPQVSTPTLVEVSRNLGKVGSKCCKHPEAKRMPCAEDYLSVVL NQLCVLHEKTPVSDRVTKCCTESLVNRRPCFSALEVDETYVPKEFN AETFTFHADICTLSEKERQIKKQTALVELVKHKPKATKEQLKAVMD DFAAFVEKCCKADDKETCFAEEGKKLVAASQAALGLHHHHHH |
| 232 | **EGF-3/[4]** | **DLL3-12 xI2C - HALB** | bispecific molecule -HALB | QVQLVESGGGVVQPGRSLRLSCAASGFSFSSYDIHWVRQAPGKGLE WVAVISSHGSNKNYARSVKGRFTISRDNSKNTLYLQMNSLKAEDTA VYYCARDGYSGNDPFYYYYHGMDVWGQGTTVTVSSGGGGSGGGGSG GGGSDIQMTQSPSSLSASVGDRVTITCRASQSISSYLNWYQQKPGK APKLLIYAASSLQSGVPSRFSGSGSGTDFSLTISSLQPEDFATYYC |
| | | | | QQSFTTPLTFGGGTKVEIKSGGGGSEVQLVESGGGLVQPGGSLKLS CAASGFTFNKYAMNWVRQAPGKGLEWVARIRSKYNNYATYYADSVK DRFTISRDDSKNTAYLQMNNLKTEDTAVYYCVRHGNFGNSYISYWA YWGQGTLVTVSSGGGGSGGGGSGGGGSQTVVTQEPSLTVSPGGTVT LTCGSSTGAVTSGNYPNWVQQKPGQAPRGLIGGTKFLAPGTPARFS GSLLGGKAALTLSGVQPEDEAEYYCVLWYSNRWVFGGGTKLTVLPG GGGSDAHKSEVAHRFKDLGEENFKALVLIAFAQYLQQCPFEDHVKL VNEVTEFAKTCVADESAENCDKSLHTLFGDKLCTVATLRETYGEMA DCCAKQEPERNECFLQHKDDNPNLPRLVRPEVDVMCTAFHDNEETF LKKYLYEIARRHPYFYAPELLFFAKRYKAAFTECCQAADKAACLLP KLDELRDEGKASSAKQRLKCASLQKFGERAFKAWAVARLSQRFPKA EFAEVSKLVTDLTKVHTECCHGDLLECADDRADLAKYICENQDSIS SKLKECCEKPLLEKSHCIAEVENDEMPADLPSLAADFVESKDVCKN YAEAKDVFLGMFLYEYARRHPDYSVVLLLRLAKTYETTLEKCCAAA DPHECYAKVFDEFKPLVEEPQNLIKQNCELFEQLGEYKFQNALLVR YTKKVPQVSTPTLVEVSRNLGKVGSKCCKHPEAKRMPCAEDYLSVV LNQLCVLHEKTPVSDRVTKCCTESLVNRRPCFSALEVDETYVPKEF NAETFTFHADICTLSEKERQIKKQTALVELVKHKPKATKEQLKAVM DDFAAFVEKCCKADDKETCFAEEGKKLVAASQAALGLHHHHHH |
| 233 | **EGF-4** | **DLL3-13 xI2C - HALB** | bispecific molecule -HALB | QVQLVQSGAEVKKPGASVKVSCKASGYTFTSYYMHWVRQAPGQGLE WMGIINPSDGSTNYAQNFQGRVTMTRDTSTNTVYMELSSLRSEDTA VYYCARGGNSAFYSYYDMDVWGQGTTVTVSSGGGGSGGGGSGGGGS DVVMTQSPLSLPVTLGQPASISCRSSQSLVYRDGNTYLSWFQQRPG QSPRRLIYKVSNWQSGVPDRFSGSGSGTDFTLKISRVEAEDVGVYF CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVLP GGGGSDAHKSEVAHRFKDLGEENFKALVLIAFAQYLQQCPFEDHVK LVNEVTEFAKTCVADESAENCDKSLHTLFGDKLCTVATLRETYGEM ADCCAKQEPERNECFLQHKDDNPNLPRLVRPEVDVMCTAFHDNEET FLKKYLYEIARRHPYFYAPELLFFAKRYKAAFTECCQAADKAACLL PKLDELRDEGKASSAKQRLKCASLQKFGERAFKAWAVARLSQRFPK AEFAEVSKLVTDLTKVHTECCHGDLLECADDRADLAKYICENQDSI SSKLKECCEKPLLEKSHCIAEVENDEMPADLPSLAADFVESKDVCK NYAEAKDVFLGMFLYEYARRHPDYSVVLLLRLAKTYETTLEKCCAA ADPHECYAKVFDEFKPLVEEPQNLIKQNCELFEQLGEYKFQNALLV RYTKKVPQVSTPTLVEVSRNLGKVGSKCCKHPEAKRMPCAEDYLSV VLNQLCVLHEKTPVSDRVTKCCTESLVNRRPCFSALEVDETYVPKE FNAETFTFHADICTLSEKERQIKKQTALVELVKHKPKATKEQLKAV MDDFAAFVEKCCKADDKETCFAEEGKKLVAASQAALGLHHHHHH |
| 234 | **EGF-4** | **DLL3-14 xI2C - HALB** | bispecific molecule -HALB | QVQLVQSGAEVKKPGASVKVSCKASGYTFTNYYMHWVRQAPGLGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVLP GGGGSDAHKSEVAHRFKDLGEENFKALVLIAFAQYLQQCPFEDHVK LVNEVTEFAKTCVADESAENCDKSLHTLFGDKLCTVATLRETYGEM ADCCAKQEPERNECFLQHKDDNPNLPRLVRPEVDVMCTAFHDNEET FLKKYLYEIARRHPYFYAPELLFFAKRYKAAFTECCQAADKAACLL PKLDELRDEGKASSAKQRLKCASLQKFGERAFKAWAVARLSQRFPK |
| | | | | AEFAEVSKLVTDLTKVHTECCHGDLLECADDRADLAKYICENQDSI SSKLKECCEKPLLEKSHCIAEVENDEMPADLPSLAADFVESKDVCK NYAEAKDVFLGMFLYEYARRHPDYSVVLLLRLAKTYETTLEKCCAA ADPHECYAKVFDEFKPLVEEPQNLIKQNCELFEQLGEYKFQNALLV RYTKKVPQVSTPTLVEVSRNLGKVGSKCCKHPEAKRMPCAEDYLSV VLNQLCVLHEKTPVSDRVTKCCTESLVNRRPCFSALEVDETYVPKE FNAETFTFHADICTLSEKERQIKKQTALVELVKHKPKATKEQLKAV MDDFAAFVEKCCKADDKETCFAEEGKKLVAASQAALGLHHHHHH |
| 235 | **EGF-4** | **DLL3-15 xI2C - HALB** | bispecific molecule -HALB | QVQLVQSGAEVKKPGASVKVSCKASGYTFTGYYIHWVRQAPGQGLE WMGIINPSDGSTSYGQNFQGRVTMTRDTSTNTVYMELSSLRSEDTA VYYCARGGNSAFYSYYDMDVWGQGTTVTVSSGGGGSGGGGSGGGGS DVVMTQSPLSLPVTLGQPASISCRSSQSLAYRDGNTYLSWFQQRPG QSPRRLIYKVSNWQSGVPDRFSGSGSGTDFTLKISRVEAEDVGVYF CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVLP GGGGSDAHKSEVAHRFKDLGEENFKALVLIAFAQYLQQCPFEDHVK LVNEVTEFAKTCVADESAENCDKSLHTLFGDKLCTVATLRETYGEM ADCCAKQEPERNECFLQHKDDNPNLPRLVRPEVDVMCTAFHDNEET FLKKYLYEIARRHPYFYAPELLFFAKRYKAAFTECCQAADKAACLL PKLDELRDEGKASSAKQRLKCASLQKFGERAFKAWAVARLSQRFPK AEFAEVSKLVTDLTKVHTECCHGDLLECADDRADLAKYICENQDSI SSKLKECCEKPLLEKSHCIAEVENDEMPADLPSLAADFVESKDVCK NYAEAKDVFLGMFLYEYARRHPDYSVVLLLRLAKTYETTLEKCCAA ADPHECYAKVFDEFKPLVEEPQNLIKQNCELFEQLGEYKFQNALLV RYTKKVPQVSTPTLVEVSRNLGKVGSKCCKHPEAKRMPCAEDYLSV VLNQLCVLHEKTPVSDRVTKCCTESLVNRRPCFSALEVDETYVPKE FNAETFTFHADICTLSEKERQIKKQTALVELVKHKPKATKEQLKAV MDDFAAFVEKCCKADDKETCFAEEGKKLVAASQAALGLHHHHHH |
| 236 | **EGF-4** | **DLL3-16 xI2C - HALB** | bispecific molecule -HALB | QVQLVQSGAEVKKPGASVKVSCKASGYTFTGHYMHWVRQAPGQGLE WMGIINPSDGSTNYAQKFQGRVTMTRDTSTSTVYMELRSLRSEDTA VYYCTRGTTVVHYSYYDMDVWGQGTTVTVSSGGGGSGGGGSGGGGS DVVMTQTPLSLPVTLGQPASISCRSSQSLVYRDGNTYLTWFQQRPG QSPRRLIYKVSNWQSGVPDRFSGSGSGTDFTLKISRVEAEDVGVYY CMQGTHWPPTFGGGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVLP GGGGSDAHKSEVAHRFKDLGEENFKALVLIAFAQYLQQCPFEDHVK LVNEVTEFAKTCVADESAENCDKSLHTLFGDKLCTVATLRETYGEM ADCCAKQEPERNECFLQHKDDNPNLPRLVRPEVDVMCTAFHDNEET FLKKYLYEIARRHPYFYAPELLFFAKRYKAAFTECCQAADKAACLL PKLDELRDEGKASSAKQRLKCASLQKFGERAFKAWAVARLSQRFPK AEFAEVSKLVTDLTKVHTECCHGDLLECADDRADLAKYICENQDSI SSKLKECCEKPLLEKSHCIAEVENDEMPADLPSLAADFVESKDVCK NYAEAKDVFLGMFLYEYARRHPDYSVVLLLRLAKTYETTLEKCCAA ADPHECYAKVFDEFKPLVEEPQNLIKQNCELFEQLGEYKFQNALLV RYTKKVPQVSTPTLVEVSRNLGKVGSKCCKHPEAKRMPCAEDYLSV VLNQLCVLHEKTPVSDRVTKCCTESLVNRRPCFSALEVDETYVPKE FNAETFTFHADICTLSEKERQIKKQTALVELVKHKPKATKEQLKAV MDDFAAFVEKCCKADDKETCFAEEGKKLVAASQAALGLHHHHHH |
| 237 | **EGF-4** | **DLL3-17 xI2C - HALB** | bispecific molecule -HALB | QVQLVQSGAEVKKPGASVKVSCKASGYTFTNYFMHWVRQAPGLGLE WMGIINPSDGSTSYAQNFQGRVTMTRDTSTNTVYMELSSLRSEDTA VYYCARGGNSAFYSYYDMDVWGQGTTVTVSSGGGGSGGGGSGGGGS |
| | | | | DVVMTQSPLSLPVTLGQPASISCRSSQSLVYRDGNTYLSWFQQRPG QSPRRLIYRVSNWQSGVPDRFSGSGSGTDFTLKISRVEAEDVGVYF CMQGTYWPPTFGQGTKVDIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVLP GGGGSDAHKSEVAHRFKDLGEENFKALVLIAFAQYLQQCPFEDHVK LVNEVTEFAKTCVADESAENCDKSLHTLFGDKLCTVATLRETYGEM ADCCAKQEPERNECFLQHKDDNPNLPRLVRPEVDVMCTAFHDNEET FLKKYLYEIARRHPYFYAPELLFFAKRYKAAFTECCQAADKAACLL PKLDELRDEGKASSAKQRLKCASLQKFGERAFKAWAVARLSQRFPK AEFAEVSKLVTDLTKVHTECCHGDLLECADDRADLAKYICENQDSI SSKLKECCEKPLLEKSHCIAEVENDEMPADLPSLAADFVESKDVCK NYAEAKDVFLGMFLYEYARRHPDYSVVLLLRLAKTYETTLEKCCAA ADPHECYAKVFDEFKPLVEEPQNLIKQNCELFEQLGEYKFQNALLV RYTKKVPQVSTPTLVEVSRNLGKVGSKCCKHPEAKRMPCAEDYLSV VLNQLCVLHEKTPVSDRVTKCCTESLVNRRPCFSALEVDETYVPKE FNAETFTFHADICTLSEKERQIKKQTALVELVKHKPKATKEQLKAV MDDFAAFVEKCCKADDKETCFAEEGKKLVAASQAALGLHHHHHH |
| 238 | **EGF-5/[6]** | **DLL3-18 xI2C** - **HALB** | bispecific molecule -HALB | QVQLVESGGGAVQPGRSLRLSCAASGFTFSNYGMHWVRQAPGKGLE WVAVISHHGSSKYYARSVKGRFTISRDNSKNTLYLEMNSLRAEDTA VYYCARDWWELVFDYWGQGTLVTVSSGGGGSGGGGSGGGGSDIVMT QTPLSLSVTPGQPASISCKSSQSLLHSDGKTFLYWYLQKPGQPPQL LIYEVSNRFSGVPDRFSGSGSGTDFTLKISRVEAEDVGVYYCLQGI HLPFTFGPGTKVEIKSGGGGSEVQLVESGGGLVQPGGSLKLSCAAS GFTFNKYAMNWVRQAPGKGLEWVARIRSKYNNYATYYADSVKDRFT ISRDDSKNTAYLQMNNLKTEDTAVYYCVRHGNFGNSYISYWAYWGQ GTLVTVSSGGGGSGGGGSGGGGSQTVVTQEPSLTVSPGGTVTLTCG SSTGAVTSGNYPNWVQQKPGQAPRGLIGGTKFLAPGTPARFSGSLL GGKAALTLSGVQPEDEAEYYCVLWYSNRWVFGGGTKLTVLPGGGGS DAHKSEVAHRFKDLGEENFKALVLIAFAQYLQQCPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALEVDETYVPKEFNAET FTFHADICTLSEKERQIKKQTALVELVKHKPKATKEQLKAVMDDFA AFVEKCCKADDKETCFAEEGKKLVAASQAALGLHHHHHH |
| 239 | **EGF-5/[6]** | **DLL3-19 xI2C** - **HALB** | bispecific molecule -HALB | QVTLKESGPMLVKPTETLTLTCTVSGFSLSNSRMGVSWIRQPPGRA LEWLAHIFSNDGKSYSTSLKSRLTISKDTSKSQVVLTMTNMDPVDT ATYYCARYNYDSSGYYYSFFDYWGQGTLVTVSSGGGGSGGGGSGGG GSDIQMTQSPSSLSASVGDRVTITCRASQSISSYLNWYQQKPGKAP KLLIYAASSLQSGVPSRFSGSGSGTDFTLTISSLQPEDFATYYCQQ GYSSPFTFGGGTKVEIKSGGGGSEVQLVESGGGLVQPGGSLKLSCA ASGFTFNKYAMNWVRQAPGKGLEWVARIRSKYNNYATYYADSVKDR FTISRDDSKNTAYLQMNNLKTEDTAVYYCVRHGNFGNSYISYWAYW GQGTLVTVSSGGGGSGGGGSGGGGSQTVVTQEPSLTVSPGGTVTLT CGSSTGAVTSGNYPNWVQQKPGQAPRGLIGGTKFLAPGTPARFSGS LLGGKAALTLSGVQPEDEAEYYCVLWYSNRWVFGGGTKLTVLPGGG GSDAHKSEVAHRFKDLGEENFKALVLIAFAQYLQQCPFEDHVKLVN EVTEFAKTCVADESAENCDKSLHTLFGDKLCTVATLRETYGEMADC CAKQEPERNECFLQHKDDNPNLPRLVRPEVDVMCTAFHDNEETFLK |
| | | | | KYLYEIARRHPYFYAPELLFFAKRYKAAFTECCQAADKAACLLPKL DELRDEGKASSAKQRLKCASLQKFGERAFKAWAVARLSQRFPKAEF AEVSKLVTDLTKVHTECCHGDLLECADDRADLAKYICENQDSISSK LKECCEKPLLEKSHCIAEVENDEMPADLPSLAADFVESKDVCKNYA EAKDVFLGMFLYEYARRHPDYSVVLLLRLAKTYETTLEKCCAAADP HECYAKVFDEFKPLVEEPQNLIKQNCELFEQLGEYKFQNALLVRYT KKVPQVSTPTLVEVSRNLGKVGSKCCKHPEAKRMPCAEDYLSVVLN QLCVLHEKTPVSDRVTKCCTESLVNRRPCFSALEVDETYVPKEFNA ETFTFHADICTLSEKERQIKKQTALVELVKHKPKATKEQLKAVMDD FAAFVEKCCKADDKETCFAEEGKKLVAASQAALGLHHHHHH |
| 240 | **EGF-5/[6]** | **DLL3-20 xI2C - HALB** | bispecific molecule -HALB | QVTLKESGPVLVKPTETLTLTCTVSGFSLSNARMGVSWLRQPPGKA LEWLAHIFSTDEKSYSTSLKSRLTISKDTSKSQVVLTMTNMDPVDT ATYYCARYYYDSSGYYYSFFDYWGQGTLVTVSSGGGGSGGGGSGGG GSDIQMTQSPSSLSASVGDRVTITCRASQSIRSYLNWYQQKPGKAP KLLIYGASNLQSGVPSRFSGSGSGTDFTLTISSLQPEDFATYYCQQ SYSSPFTFGGGTKVEIKSGGGGSEVQLVESGGGLVQPGGSLKLSCA ASGFTFNKYAMNWVRQAPGKGLEWVARIRSKYNNYATYYADSVKDR FTISRDDSKNTAYLQMNNLKTEDTAVYYCVRHGNFGNSYISYWAYW GQGTLVTVSSGGGGSGGGGSGGGGSQTVVTQEPSLTVSPGGTVTLT CGSSTGAVTSGNYPNWVQQKPGQAPRGLIGGTKFLAPGTPARFSGS LLGGKAALTLSGVQPEDEAEYYCVLWYSNRWVFGGGTKLTVLPGGG GSDAHKSEVAHRFKDLGEENFKALVLIAFAQYLQQCPFEDHVKLVN EVTEFAKTCVADESAENCDKSLHTLFGDKLCTVATLRETYGEMADC CAKQEPERNECFLQHKDDNPNLPRLVRPEVDVMCTAFHDNEETFLK KYLYEIARRHPYFYAPELLFFAKRYKAAFTECCQAADKAACLLPKL DELRDEGKASSAKQRLKCASLQKFGERAFKAWAVARLSQRFPKAEF AEVSKLVTDLTKVHTECCHGDLLECADDRADLAKYICENQDSISSK LKECCEKPLLEKSHCIAEVENDEMPADLPSLAADFVESKDVCKNYA EAKDVFLGMFLYEYARRHPDYSVVLLLRLAKTYETTLEKCCAAADP HECYAKVFDEFKPLVEEPQNLIKQNCELFEQLGEYKFQNALLVRYT KKVPQVSTPTLVEVSRNLGKVGSKCCKHPEAKRMPCAEDYLSVVLN QLCVLHEKTPVSDRVTKCCTESLVNRRPCFSALEVDETYVPKEFNA ETFTFHADICTLSEKERQIKKQTALVELVKHKPKATKEQLKAVMDD FAAFVEKCCKADDKETCFAEEGKKLVAASQAALGLHHHHHH |
| 241 | **EGF-5/[6]** | **DLL3-21 xI2C - HALB** | bispecific molecule -HALB | QVQLVQSGAEVKKPGASVKVSCKASGYAFTSYYIHWVRQAPGQGLE WMGIINPSGGSKSYAQKFRGRVTMTRDTSTSTVYMELSSLTSEDTA VYYCARSMSTVTSDAFDIWGQGTMVTVSSGGGGSGGGGSGGGGSDI QMTQSPSSLSASVGDRVTITCRASQSISNYLNWYQQKPGKAPKLLI YAASSLQSGVPSRFSGSGSGTEFTLTISSLQPEDFATYYCQQSYSA PLTFGGGTKVDIKSGGGGSEVQLVESGGGLVQPGGSLKLSCAASGF TFNKYAMNWVRQAPGKGLEWVARIRSKYNNYATYYADSVKDRFTIS RDDSKNTAYLQMNNLKTEDTAVYYCVRHGNFGNSYISYWAYWGQGT LVTVSSGGGGSGGGGSGGGGSQTVVTQEPSLTVSPGGTVTLTCGSS TGAVTSGNYPNWVQQKPGQAPRGLIGGTKFLAPGTPARFSGSLLGG KAALTLSGVQPEDEAEYYCVLWYSNRWVFGGGTKLTVLPGGGGSDA HKSEVAHRFKDLGEENFKALVLIAFAQYLQQCPFEDHVKLVNEVTE FAKTCVADESAENCDKSLHTLFGDKLCTVATLRETYGEMADCCAKQ EPERNECFLQHKDDNPNLPRLVRPEVDVMCTAFHDNEETFLKKYLY EIARRHPYFYAPELLFFAKRYKAAFTECCQAADKAACLLPKLDELR DEGKASSAKQRLKCASLQKFGERAFKAWAVARLSQRFPKAEFAEVS KLVTDLTKVHTECCHGDLLECADDRADLAKYICENQDSISSKLKEC CEKPLLEKSHCIAEVENDEMPADLPSLAADFVESKDVCKNYAEAKD VFLGMFLYEYARRHPDYSVVLLLRLAKTYETTLEKCCAAADPHECY AKVFDEFKPLVEEPQNLIKQNCELFEQLGEYKFQNALLVRYTKKVP QVSTPTLVEVSRNLGKVGSKCCKHPEAKRMPCAEDYLSVVLNQLCV LHEKTPVSDRVTKCCTESLVNRRPCFSALEVDETYVPKEFNAETFT FHADICTLSEKERQIKKQTALVELVKHKPKATKEQLKAVMDDFAAF VEKCCKADDKETCFAEEGKKLVAASQAALGLHHHHHH |
| 242 | **EGF-3** | **DLL3-4 xF12Q -HALB** | bispecific molecule -HALB | QVQLQESGPGLVKPSETLSLTCTVSGGSISSYYWSWIRQPPGKGLE |
| | | | | WIGYVYYSGTTNYNPSLKSRVTISVDTSKNQFSLKLSSVTAADTAV YYCASIAVTGFYFDYWGQGTLVTVSSGGGGSGGGGSGGGGSEIVLT QSPGTLSLSPGERVTLSCRASQRVNNNYLAWYQQRPGQAPRLLIYG ASSRATGIPDRFSGSGSGTDFTLTISRLEPEDFAVYYCQQYDRSPL TFGGGTKLEIKSGGGGSEVQLVESGGGLVQPGGSLRLSCAASGFTF NSYAMNWVRQAPGKGLEWVARIRSKYNNYATYYADSVKGRFTISRD DSKNTAYLQMNSLKTEDTAVYYCVRHGNFGNSYVSWWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLPGGGGSDAHK SEVAHRFKDLGEENFKALVLIAFAQYLQQCPFEDHVKLVNEVTEFA KTCVADESAENCDKSLHTLFGDKLCTVATLRETYGEMADCCAKQEP ERNECFLQHKDDNPNLPRLVRPEVDVMCTAFHDNEETFLKKYLYEI ARRHPYFYAPELLFFAKRYKAAFTECCQAADKAACLLPKLDELRDE GKASSAKQRLKCASLQKFGERAFKAWAVARLSQRFPKAEFAEVSKL VTDLTKVHTECCHGDLLECADDRADLAKYICENQDSISSKLKECCE KPLLEKSHCIAEVENDEMPADLPSLAADFVESKDVCKNYAEAKDVF LGMFLYEYARRHPDYSVVLLLRLAKTYETTLEKCCAAADPHECYAK VFDEFKPLVEEPQNLIKQNCELFEQLGEYKFQNALLVRYTKKVPQV STPTLVEVSRNLGKVGSKCCKHPEAKRMPCAEDYLSVVLNQLCVLH EKTPVSDRVTKCCTESLVNRRPCFSALEVDETYVPKEFNAETFTFH ADICTLSEKERQIKKQTALVELVKHKPKATKEQLKAVMDDFAAFVE KCCKADDKETCFAEEGKKLVAASQAALGLHHHHHH |
| 243 | **EGF-3** | **DLL3-5 xF12Q -HALB** | bispecific molecule -HALB | QVQLQESGPGLVKPSETLSLTCTVSGGSISSYYWSWIRQPPGKGLE WIGYIYYSGRTNYYPSLKSRVTISIDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKLEIKSGGGGSEVQLVESGGGLVQPGGSLRLSCAASGFTF NSYAMNWVRQAPGKGLEWVARIRSKYNNYATYYADSVKGRFTISRD DSKNTAYLQMNSLKTEDTAVYYCVRHGNFGNSYVSWWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLPGGGGSDAHK SEVAHRFKDLGEENFKALVLIAFAQYLQQCPFEDHVKLVNEVTEFA KTCVADESAENCDKSLHTLFGDKLCTVATLRETYGEMADCCAKQEP ERNECFLQHKDDNPNLPRLVRPEVDVMCTAFHDNEETFLKKYLYEI ARRHPYFYAPELLFFAKRYKAAFTECCQAADKAACLLPKLDELRDE GKASSAKQRLKCASLQKFGERAFKAWAVARLSQRFPKAEFAEVSKL VTDLTKVHTECCHGDLLECADDRADLAKYICENQDSISSKLKECCE KPLLEKSHCIAEVENDEMPADLPSLAADFVESKDVCKNYAEAKDVF LGMFLYEYARRHPDYSVVLLLRLAKTYETTLEKCCAAADPHECYAK VFDEFKPLVEEPQNLIKQNCELFEQLGEYKFQNALLVRYTKKVPQV STPTLVEVSRNLGKVGSKCCKHPEAKRMPCAEDYLSVVLNQLCVLH EKTPVSDRVTKCCTESLVNRRPCFSALEVDETYVPKEFNAETFTFH ADICTLSEKERQIKKQTALVELVKHKPKATKEQLKAVMDDFAAFVE KCCKADDKETCFAEEGKKLVAASQAALGLHHHHHH |
| 244 | **EGF-3** | **DLL3-6 xF12Q -HALB** | bispecific molecule -HALB | QVQLQESGPGLVKPSETLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKVEIKSGGGGSEVQLVESGGGLVQPGGSLRLSCAASGFTF NSYAMNWVRQAPGKGLEWVARIRSKYNNYATYYADSVKGRFTISRD DSKNTAYLQMNSLKTEDTAVYYCVRHGNFGNSYVSWWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLPGGGGSDAHK SEVAHRFKDLGEENFKALVLIAFAQYLQQCPFEDHVKLVNEVTEFA |
| | | | | KTCVADESAENCDKSLHTLFGDKLCTVATLRETYGEMADCCAKQEP ERNECFLQHKDDNPNLPRLVRPEVDVMCTAFHDNEETFLKKYLYEI ARRHPYFYAPELLFFAKRYKAAFTECCQAADKAACLLPKLDELRDE GKASSAKQRLKCASLQKFGERAFKAWAVARLSQRFPKAEFAEVSKL VTDLTKVHTECCHGDLLECADDRADLAKYICENQDSISSKLKECCE KPLLEKSHCIAEVENDEMPADLPSLAADFVESKDVCKNYAEAKDVF LGMFLYEYARRHPDYSVVLLLRLAKTYETTLEKCCAAADPHECYAK VFDEFKPLVEEPQNLIKQNCELFEQLGEYKFQNALLVRYTKKVPQV STPTLVEVSRNLGKVGSKCCKHPEAKRMPCAEDYLSVVLNQLCVLH EKTPVSDRVTKCCTESLVNRRPCFSALEVDETYVPKEFNAETFTFH ADICTLSEKERQIKKQTALVELVKHKPKATKEQLKAVMDDFAAFVE KCCKADDKETCFAEEGKKLVAASQAALGLHHHHHH |
| 245 | **EGF-3** | **DLL3-7 xF12Q -HALB** | bispecific molecule -HALB | QVQLQESGPGLVKPSQTLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKVEIKSGGGGSEVQLVESGGGLVQPGGSLRLSCAASGFTF NSYAMNWVRQAPGKGLEWVARIRSKYNNYATYYADSVKGRFTISRD DSKNTAYLQMNSLKTEDTAVYYCVRHGNFGNSYVSWWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLPGGGGSDAHK SEVAHRFKDLGEENFKALVLIAFAQYLQQCPFEDHVKLVNEVTEFA KTCVADESAENCDKSLHTLFGDKLCTVATLRETYGEMADCCAKQEP ERNECFLQHKDDNPNLPRLVRPEVDVMCTAFHDNEETFLKKYLYEI ARRHPYFYAPELLFFAKRYKAAFTECCQAADKAACLLPKLDELRDE GKASSAKQRLKCASLQKFGERAFKAWAVARLSQRFPKAEFAEVSKL VTDLTKVHTECCHGDLLECADDRADLAKYICENQDSISSKLKECCE KPLLEKSHCIAEVENDEMPADLPSLAADFVESKDVCKNYAEAKDVF LGMFLYEYARRHPDYSVVLLLRLAKTYETTLEKCCAAADPHECYAK VFDEFKPLVEEPQNLIKQNCELFEQLGEYKFQNALLVRYTKKVPQV STPTLVEVSRNLGKVGSKCCKHPEAKRMPCAEDYLSVVLNQLCVLH EKTPVSDRVTKCCTESLVNRRPCFSALEVDETYVPKEFNAETFTFH ADICTLSEKERQIKKQTALVELVKHKPKATKEQLKAVMDDFAAFVE KCCKADDKETCFAEEGKKLVAASQAALGLHHHHHH |
| 246 | **EGF-3** | **DLL3-8 xF12Q -HALB** | bispecific molecule -HALB | QVQLQEWGPGLVKPSETLSLTCTVSGASISSFYWSWIRQPPGKGLE WIGYIYYSGTTNYNPSLKSRVTISVDTSKNQLSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKVDIKSGGGGSEVQLVESGGGLVQPGGSLRLSCAASGFTF NSYAMNWVRQAPGKGLEWVARIRSKYNNYATYYADSVKGRFTISRD DSKNTAYLQMNSLKTEDTAVYYCVRHGNFGNSYVSWWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLPGGGGSDAHK SEVAHRFKDLGEENFKALVLIAFAQYLQQCPFEDHVKLVNEVTEFA KTCVADESAENCDKSLHTLFGDKLCTVATLRETYGEMADCCAKQEP ERNECFLQHKDDNPNLPRLVRPEVDVMCTAFHDNEETFLKKYLYEI ARRHPYFYAPELLFFAKRYKAAFTECCQAADKAACLLPKLDELRDE GKASSAKQRLKCASLQKFGERAFKAWAVARLSQRFPKAEFAEVSKL VTDLTKVHTECCHGDLLECADDRADLAKYICENQDSISSKLKECCE KPLLEKSHCIAEVENDEMPADLPSLAADFVESKDVCKNYAEAKDVF LGMFLYEYARRHPDYSVVLLLRLAKTYETTLEKCCAAADPHECYAK VFDEFKPLVEEPQNLIKQNCELFEQLGEYKFQNALLVRYTKKVPQV STPTLVEVSRNLGKVGSKCCKHPEAKRMPCAEDYLSVVLNQLCVLH EKTPVSDRVTKCCTESLVNRRPCFSALEVDETYVPKEFNAETFTFH ADICTLSEKERQIKKQTALVELVKHKPKATKEQLKAVMDDFAAFVE |
| | | | | KCCKADDKETCFAEEGKKLVAASQAALGLHHHHHH |
| 247 | **EGF-3** | **DLL3-9 xF12Q -HALB** | bispecific molecule -HALB | QVQLQESGPGLVKPSETLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGESATLSCRASQSVNKNYLAWYQQKPGQAPRLLIYG ASSRATGIPDRFSGSGSGTDFTLTISRLEPEDFAVYYCQQYDRSPL TFGGGTRLEIKSGGGGSEVQLVESGGGLVQPGGSLRLSCAASGFTF NSYAMNWVRQAPGKGLEWVARIRSKYNNYATYYADSVKGRFTISRD DSKNTAYLQMNSLKTEDTAVYYCVRHGNFGNSYVSWWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLPGGGGSDAHK SEVAHRFKDLGEENFKALVLIAFAQYLQQCPFEDHVKLVNEVTEFA KTCVADESAENCDKSLHTLFGDKLCTVATLRETYGEMADCCAKQEP ERNECFLQHKDDNPNLPRLVRPEVDVMCTAFHDNEETFLKKYLYEI ARRHPYFYAPELLFFAKRYKAAFTECCQAADKAACLLPKLDELRDE GKASSAKQRLKCASLQKFGERAFKAWAVARLSQRFPKAEFAEVSKL VTDLTKVHTECCHGDLLECADDRADLAKYICENQDSISSKLKECCE KPLLEKSHCIAEVENDEMPADLPSLAADFVESKDVCKNYAEAKDVF LGMFLYEYARRHPDYSVVLLLRLAKTYETTLEKCCAAADPHECYAK VFDEFKPLVEEPQNLIKQNCELFEQLGEYKFQNALLVRYTKKVPQV STPTLVEVSRNLGKVGSKCCKHPEAKRMPCAEDYLSVVLNQLCVLH EKTPVSDRVTKCCTESLVNRRPCFSALEVDETYVPKEFNAETFTFH ADICTLSEKERQIKKQTALVELVKHKPKATKEQLKAVMDDFAAFVE KCCKADDKETCFAEEGKKLVAASQAALGLHHHHHH |
| 248 | **EGF-3** | **DLL3-10 xF12Q -HALB** | bispecific molecule -HALB | QVQLQESGPGLVKPSQTLSLTCTVSGGSISSYYWSWIRQPPGKGLE WIGYIFYNGITNYNPSLKSRVTISLDTSKNQFSLKLSSVTAADTAK YYCARIHSGSFSFDYWDQGTLVTVSSGGGGSGGGGSGGGGSEIVMT QSPGTLSLSPGERATLSCRASQSVSRGYLAWYQQKPGQAPRLLIYG ASSRATDIPDRFSGSGSGTDFTLTISRLEPEDFAVYYCQQYDTSPI TFGQGTKVEIKSGGGGSEVQLVESGGGLVQPGGSLRLSCAASGFTF NSYAMNWVRQAPGKGLEWVARIRSKYNNYATYYADSVKGRFTISRD DSKNTAYLQMNSLKTEDTAVYYCVRHGNFGNSYVSWWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLPGGGGSDAHK SEVAHRFKDLGEENFKALVLIAFAQYLQQCPFEDHVKLVNEVTEFA KTCVADESAENCDKSLHTLFGDKLCTVATLRETYGEMADCCAKQEP ERNECFLQHKDDNPNLPRLVRPEVDVMCTAFHDNEETFLKKYLYEI ARRHPYFYAPELLFFAKRYKAAFTECCQAADKAACLLPKLDELRDE GKASSAKQRLKCASLQKFGERAFKAWAVARLSQRFPKAEFAEVSKL VTDLTKVHTECCHGDLLECADDRADLAKYICENQDSISSKLKECCE KPLLEKSHCIAEVENDEMPADLPSLAADFVESKDVCKNYAEAKDVF LGMFLYEYARRHPDYSVVLLLRLAKTYETTLEKCCAAADPHECYAK VFDEFKPLVEEPQNLIKQNCELFEQLGEYKFQNALLVRYTKKVPQV STPTLVEVSRNLGKVGSKCCKHPEAKRMPCAEDYLSVVLNQLCVLH EKTPVSDRVTKCCTESLVNRRPCFSALEVDETYVPKEFNAETFTFH ADICTLSEKERQIKKQTALVELVKHKPKATKEQLKAVMDDFAAFVE KCCKADDKETCFAEEGKKLVAASQAALGLHHHHHH |
| 249 | **EGF-4** | **DLL3-13 xF12Q -HALB** | bispecific molecule -HALB | QVQLVQSGAEVKKPGASVKVSCKASGYTFTSYYMHWVRQAPGQGLE WMGIINPSDGSTNYAQNFQGRVTMTRDTSTNTVYMELSSLRSEDTA VYYCARGGNSAFYSYYDMDVWGQGTTVTVSSGGGGSGGGGSGGGGS DVVMTQSPLSLPVTLGQPASISCRSSQSLVYRDGNTYLSWFQQRPG QSPRRLIYKVSNWQSGVPDRFSGSGSGTDFTLKISRVEAEDVGVYF CMQGTHWPPTFGQGTKVEIKSGGGGSEVQLVESGGGLVQPGGSLRL SCAASGFTFNSYAMNWVRQAPGKGLEWVARIRSKYNNYATYYADSV KGRFTISRDDSKNTAYLQMNSLKTEDTAVYYCVRHGNFGNSYVSWW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF |
| | | | | SGSLLGGKAALTLSGVQPEDEAEYYCVLWYSNRWVFGGGTKLTVLP GGGGSDAHKSEVAHRFKDLGEENFKALVLIAFAQYLQQCPFEDHVK LVNEVTEFAKTCVADESAENCDKSLHTLFGDKLCTVATLRETYGEM ADCCAKQEPERNECFLQHKDDNPNLPRLVRPEVDVMCTAFHDNEET FLKKYLYEIARRHPYFYAPELLFFAKRYKAAFTECCQAADKAACLL PKLDELRDEGKASSAKQRLKCASLQKFGERAFKAWAVARLSQRFPK AEFAEVSKLVTDLTKVHTECCHGDLLECADDRADLAKYICENQDSI SSKLKECCEKPLLEKSHCIAEVENDEMPADLPSLAADFVESKDVCK NYAEAKDVFLGMFLYEYARRHPDYSVVLLLRLAKTYETTLEKCCAA ADPHECYAKVFDEFKPLVEEPQNLIKQNCELFEQLGEYKFQNALLV RYTKKVPQVSTPTLVEVSRNLGKVGSKCCKHPEAKRMPCAEDYLSV VLNQLCVLHEKTPVSDRVTKCCTESLVNRRPCFSALEVDETYVPKE FNAETFTFHADICTLSEKERQIKKQTALVELVKHKPKATKEQLKAV MDDFAAFVEKCCKADDKETCFAEEGKKLVAASQAALGLHHHHHH |
| 250 | **EGF-4** | **DLL3-14 xF12Q -HALB** | bispecific molecule -HALB | QVQLVQSGAEVKKPGASVKVSCKASGYTFTNYYMHWVRQAPGLGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGQGTKVEIKSGGGGSEVQLVESGGGLVQPGGSLRL SCAASGFTFNSYAMNWVRQAPGKGLEWVARIRSKYNNYATYYADSV KGRFTISRDDSKNTAYLQMNSLKTEDTAVYYCVRHGNFGNSYVSWW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVLP GGGGSDAHKSEVAHRFKDLGEENFKALVLIAFAQYLQQCPFEDHVK LVNEVTEFAKTCVADESAENCDKSLHTLFGDKLCTVATLRETYGEM ADCCAKQEPERNECFLQHKDDNPNLPRLVRPEVDVMCTAFHDNEET FLKKYLYEIARRHPYFYAPELLFFAKRYKAAFTECCQAADKAACLL PKLDELRDEGKASSAKQRLKCASLQKFGERAFKAWAVARLSQRFPK AEFAEVSKLVTDLTKVHTECCHGDLLECADDRADLAKYICENQDSI SSKLKECCEKPLLEKSHCIAEVENDEMPADLPSLAADFVESKDVCK NYAEAKDVFLGMFLYEYARRHPDYSVVLLLRLAKTYETTLEKCCAA ADPHECYAKVFDEFKPLVEEPQNLIKQNCELFEQLGEYKFQNALLV RYTKKVPQVSTPTLVEVSRNLGKVGSKCCKHPEAKRMPCAEDYLSV VLNQLCVLHEKTPVSDRVTKCCTESLVNRRPCFSALEVDETYVPKE FNAETFTFHADICTLSEKERQIKKQTALVELVKHKPKATKEQLKAV MDDFAAFVEKCCKADDKETCFAEEGKKLVAASQAALGLHHHHHH |
| 251 | **EGF-4** | **DLL3-15 xF12Q -HALB** | bispecific molecule -HALB | QVQLVQSGAEVKKPGASVKVSCKASGYTFTGYYIHWVRQAPGQGLE WMGIINPSDGSTSYGQNFQGRVTMTRDTSTNTVYMELSSLRSEDTA VYYCARGGNSAFYSYYDMDVWGQGTTVTVSSGGGGSGGGGSGGGGS DVVMTQSPLSLPVTLGQPASISCRSSQSLAYRDGNTYLSWFQQRPG QSPRRLIYKVSNWQSGVPDRFSGSGSGTDFTLKISRVEAEDVGVYF CMQGTHWPPTFGQGTKVEIKSGGGGSEVQLVESGGGLVQPGGSLRL SCAASGFTFNSYAMNWVRQAPGKGLEWVARIRSKYNNYATYYADSV KGRFTISRDDSKNTAYLQMNSLKTEDTAVYYCVRHGNFGNSYVSWW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVLP GGGGSDAHKSEVAHRFKDLGEENFKALVLIAFAQYLQQCPFEDHVK LVNEVTEFAKTCVADESAENCDKSLHTLFGDKLCTVATLRETYGEM ADCCAKQEPERNECFLQHKDDNPNLPRLVRPEVDVMCTAFHDNEET FLKKYLYEIARRHPYFYAPELLFFAKRYKAAFTECCQAADKAACLL PKLDELRDEGKASSAKQRLKCASLQKFGERAFKAWAVARLSQRFPK AEFAEVSKLVTDLTKVHTECCHGDLLECADDRADLAKYICENQDSI SSKLKECCEKPLLEKSHCIAEVENDEMPADLPSLAADFVESKDVCK NYAEAKDVFLGMFLYEYARRHPDYSVVLLLRLAKTYETTLEKCCAA ADPHECYAKVFDEFKPLVEEPQNLIKQNCELFEQLGEYKFQNALLV RYTKKVPQVSTPTLVEVSRNLGKVGSKCCKHPEAKRMPCAEDYLSV |
| | | | | VLNQLCVLHEKTPVSDRVTKCCTESLVNRRPCFSALEVDETYVPKE FNAETFTFHADICTLSEKERQIKKQTALVELVKHKPKATKEQLKAV MDDFAAFVEKCCKADDKETCFAEEGKKLVAASQAALGLHHHHHH |
| 252 | - | **Human DLL3** | human | |
| 253 | - | **Human DLL3 ECD** | human | MVSPRMSGLLSQTVILALIFLPQTRPAGVFELQIHSFGPGPGPGAP RSPCSARLPCRLFFRVCLKPGLSEEAAESPCALGAALSARGPVYTE QPGAPAPDLPLPDGLLQVPFRDAWPGTFSFIIETWREELGDQIGGP AWSLLARVAGRRRLAAGGPWARDIQRAGAWELRFSYRARCEPPAVG TACTRLCRPRSAPSRCGPGLRPCAPLEDECEAPLVCRAGCSPEHGF CEQPGECRCLEGWTGPLCTVPVSTSSCLSPRGPSSATTGCLVPGPG PCDGNPCANGGSCSETPRSFECTCPRGFYGLRCEVSGVTCADGPCF NGGLCVGGADPDSAYICHCPPGFQGSNCEKRVDRCSLQPCRNGGLC LDLGHALRCRCRAGFAGPRCEHDLDDCAGRACANGGTCVEGGGAHR CSCALGFGGRDCRERADPCAARPCAHGGRCYAHFSGLVCACAPGYM GARCEFPVHPDGASALPAAPPGLRPGDPQRYL |
| 254 | - | **Hu DLL3 N-term.** | human | MVSPRMSGLLSQTVILALIFLPQTRPAGVFELQIHSFGPGPGPGAP RSPCSARLPCRLFFRVCLKPGLSEEAAESPCALGAALSARGPVYTE QPGAPAPDLPLPDGLLQVPFRDAWPGTFSFIIETWREELGDQIGGP AWSLLARVAGRRRLAAGGPWARDIQRAGAWELRFSYR |
| 255 | - | **Hu DLL3 DSL dom** | human | ARCEPPAVGTACTRLCRPRSAPSRCGPGLRPCAPLEDECE |
| 256 | - | **Hu DLL3 EGF-1** | human | APLVCRAGCSPEHGFCEQPGECRCLEGWTGPLCT |
| 257 | - | **Hu DLL3 EGF-2** | human | GPGPCDGNPCANGGSCSETPRSFECTCPRGFYGLRCE |
| 258 | - | **Hu DLL3 EGF-3** | human | SGVTCADGPCFNGGLCVGGADPDSAYICHCPPGFQGSNCE |
| 259 | - | **Hu DLL3 EGF-4** | human | RVDRCSLQPCRNGGLCLDLGHALRCRCRAGFAGPRCE |
| 260 | - | **Hu DLL3 EGF-3+4** | human | SGVTCADGPCFNGGLCVGGADPDSAYICHCPPGFQGSNCEKRVDRC SLQPCRNGGLCLDLGHALRCRCRAGFAGPRCE |
| 261 | - | **Hu DLL3 EGF-5** | human | DLDDCAGRACANGGTCVEGGGAHRCSCALGFGGRDCR |
| 262 | - | **Hu DLL3 EGF-6** | human | RADPCAARPCAHGGRCYAHFSGLVCACAPGYMGARCE |
| 263 | - | **Human DLL3 ECD x EpCAM** | artificial | MVSPRMSGLLSQTVILALIFLPQTRPAGVFELQIHSFGPGPGPGAP RSPCSARLPCRLFFRVCLKPGLSEEAAESPCALGAALSARGPVYTE QPGAPAPDLPLPDGLLQVPFRDAWPGTFSFIIETWREELGDQIGGP AWSLLARVAGRRRLAAGGPWARDIQRAGAWELRFSYRARCEPPAVG TACTRLCRPRSAPSRCGPGLRPCAPLEDECEAPLVCRAGCSPEHGF CEQPGECRCLEGWTGPLCTVPVSTSSCLSPRGPSSATTGCLVPGPG PCDGNPCANGGSCSETPRSFECTCPRGFYGLRCEVSGVTCADGPCF NGGLCVGGADPDSAYICHCPPGFQGSNCEKRVDRCSLQPCRNGGLC LDLGHALRCRCRAGFAGPRCEHDLDDCAGRACANGGTCVEGGGAHR CSCALGFGGRDCRERADPCAARPCAHGGRCYAHFSGLVCACAPGYM GARCEFPVHPDGASALPAAPPGLRPGDPQRYLSGGGGSGAGVIAVI VVVVIAIVAGIVVLVISRKKRMAKYEKAEIKEMGEMHRELNA |
| 264 | - | **V5 x hu DLL3-DSL x EpCAM** | artificial | MGWSCIILFLVATATGVHSGKPIPNPLLGLDSTSGARCEPPAVGTA CTRLCRPRSAPSRCGPGLRPCAPLEDECEAPLVCRAGCSPEHGFCE QPGECRCLEGWTGPLCTVPVSTSSCLSPRGPSSATTGCLVPGPGPC DGNPCANGGSCSETPRSFECTCPRGFYGLRCEVSGVTCADGPCFNG GLCVGGADPDSAYICHCPPGFQGSNCEKRVDRCSLQPCRNGGLCLD LGHALRCRCRAGFAGPRCEHDLDDCAGRACANGGTCVEGGGAHRCS CALGFGGRDCRERADPCAARPCAHGGRCYAHFSGLVCACAPGYMGA RCEFPVHPDGASALPAAPPGLRPGDPQRYLSGGGGSGAGVIAVIVV VVIAIVAGIVVLVISRKKRMAKYEKAEIKEMGEMHRELNA |
| 265 | - | **V5 x hu DLL3-EGF1 x EpCAM** | artificial | MGWSCIILFLVATATGVHSGKPIPNPLLGLDSTSGAPLVCRAGCSP EHGFCEQPGECRCLEGWTGPLCTVPVSTSSCLSPRGPSSATTGCLV PGPGPCDGNPCANGGSCSETPRSFECTCPRGFYGLRCEVSGVTCAD GPCFNGGLCVGGADPDSAYICHCPPGFQGSNCEKRVDRCSLQPCRN GGLCLDLGHALRCRCRAGFAGPRCEHDLDDCAGRACANGGTCVEGG GAHRCSCALGFGGRDCRERADPCAARPCAHGGRCYAHFSGLVCACA PGYMGARCEFPVHPDGASALPAAPPGLRPGDPQRYLSGGGGSGAGV IAVIVVVVIAIVAGIVVLVISRKKRMAKYEKAEIKEMGEMHRELNA |
| 266 | - | **V5 x hu DLL3-EGF2 x EpCAM** | artificial | MGWSCIILFLVATATGVHSGKPIPNPLLGLDSTSGGPGPCDGNPCA NGGSCSETPRSFECTCPRGFYGLRCEVSGVTCADGPCFNGGLCVGG ADPDSAYICHCPPGFQGSNCEKRVDRCSLQPCRNGGLCLDLGHALR CRCRAGFAGPRCEHDLDDCAGRACANGGTCVEGGGAHRCSCALGFG GRDCRERADPCAARPCAHGGRCYAHFSGLVCACAPGYMGARCEFPV HPDGASALPAAPPGLRPGDPQRYLSGGGGSGAGVIAVIVVVVIAIV AGIVVLVISRKKRMAKYEKAEIKEMGEMHRELNA |
| 267 | - | **V5 x hu DLL3-EGF3 x EpCAM** | artificial | MGWSCIILFLVATATGVHSGKPIPNPLLGLDSTSGSGVTCADGPCF NGGLCVGGADPDSAYICHCPPGFQGSNCEKRVDRCSLQPCRNGGLC LDLGHALRCRCRAGFAGPRCEHDLDDCAGRACANGGTCVEGGGAHR CSCALGFGGRDCRERADPCAARPCAHGGRCYAHFSGLVCACAPGYM GARCEFPVHPDGASALPAAPPGLRPGDPQRYLSGGGGSAGVIAVI VVVVIAIVAGIVVLVISRKKRMAKYEKAEIKEMGEMHRELNA |
| 268 | - | **V5 x hu DLL3-EGF4 x EpCAM** | artificial | MGWSCIILFLVATATGVHSGKPIPNPLLGLDSTSGRVDRCSLQPCR NGGLCLDLGHALRCRCRAGFAGPRCEHDLDDCAGRACANGGTCVEG GGAHRCSCALGFGGRDCRERADPCAARPCAHGGRCYAHFSGLVCAC APGYMGARCEFPVHPDGASALPAAPPGLRPGDPQRYLSGGGGSGAG VIAVIVVVVIAIVAGIVVLVISRKKRMAKYEKAEIKEMGEMHRELN A |
| 269 | - | **V5 x hu DLL3-EGF5 x EpCAM** | artificial | MGWSCIILFLVATATGVHSGKPIPNPLLGLDSTSGDLDDCAGRACA NGGTCVEGGGAHRCSCALGFGGRDCRERADPCAARPCAHGGRCYAH FSGLVCACAPGYMGARCEFPVHPDGASALPAAPPGLRPGDPQRYLS GGGGSGAGVIAVIVVVVIAIVAGIVVLVISRKKRMAKYEKAEIKEM GEMHRELNA |
| 270 | - | **V5 x hu DLL3-EGF6 x EpCAM** | artificial | MGWSCIILFLVATATGVHSGKPIPNPLLGLDSTSGRADPCAARPCA HGGRCYAHFSGLVCACAPGYMGARCEFPVHPDGASALPAAPPGLRP GDPQRYLSGGGGSGAGVIAVIVVVVIAIVAGIVVLVISRKKRMAKY EKAEIKEMGEMHRELNA |
| 271 | - | **Macaque DLL3** | cyno | MVSPRMSRLLSQTVILALIFIPQARPAGVFELQIHSFGPGPGPGAP RSPCSARGPCRLFFRVCLKPGLSEEAAESPCALGAALSARGPVYTE QPEAPAPDLPLPNGLLQVPFRDAWPGTFSLIIETWREELGDQIGGP AWSLLARVTRRRRLAAGGPWARDIQRAGAWELRFSYRARCELPAVG TACTRLCRPRSAPSRCGPGLRPCAPLEDECEAPPVCRAGCSLEHGF CEQPGECRCLEGWTGPLCMVPASTSSCLGLRGPSSATTGCLVPGPG PCDGNPCANGGSCSETPGSFECTCPRGFYGLRCEVSGVTCADGPCF NGGLCVGGADPDSAYICHCPPGFQGSNCEKRVDRCSLQPCRNGGLC LDLGHALRCRCRAGFAGPRCEHNLDDCAGRACANGGTCVEGGGAHR CSCALGFGGRDCRERADPCAARPCAHGGRCYAHFSGLVCACAPGYM GSRCEFPVHPDGVSALPAAPPGLRPGDPQRYLLPPALGLLVAAGVA GAALLLVHVRRRGHAQDAGSRLLAGTPEPSVHALPDALNNQRTQEG PGDVPSSSVDWNRPEDVDSRGIYVISAPSIYAREA |
| 272 | - | **Macaque DLL3 ECD** | cyno | MVSPRMSRLLSQTVILALIFIPQARPAGVFELQIHSFGPGPGPGAP RSPCSARGPCRLFFRVCLKPGLSEEAAESPCALGAALSARGPVYTE QPEAPAPDLPLPNGLLQVPFRDAWPGTFSLIIETWREELGDQIGGP AWSLLARVTRRRRLAAGGPWARDIQRAGAWELRFSYRARCELPAVG TACTRLCRPRSAPSRCGPGLRPCAPLEDECEAPPVCRAGCSLEHGF CEQPGECRCLEGWTGPLCMVPASTSSCLGLRGPSSATTGCLVPGPG PCDGNPCANGGSCSETPGSFECTCPRGFYGLRCEVSGVTCADGPCF NGGLCVGGADPDSAYICHCPPGFQGSNCEKRVDRCSLQPCRNGGLC LDLGHALRCRCRAGFAGPRCEHNLDDCAGRACANGGTCVEGGGAHR CSCALGFGGRDCRERADPCAARPCAHGGRCYAHFSGLVCACAPGYM GSRCEFPVHPDGVSALPAAPPGLRPGDPQRYL |
| 273 | - | **Ma DLL3 N-term.** | cyno | MVSPRMSRLLSQTVILALIFIPQARPAGVFELQIHSFGPGPGPGAP RSPCSARGPCRLFFRVCLKPGLSEEAAESPCALGAALSARGPVYTE QPEAPAPDLPLPNGLLQVPFRDAWPGTFSLIIETWREELGDQIGGP AWSLLARVTRRRRLAAGGPWARDIQRAGAWELRFSYR |
| 274 | - | **Ma DLL3 DSL dom.** | cyno | ARCELPAVGTACTRLCRPRSAPSRCGPGLRPCAPLEDECE |
| 275 | - | **Ma DLL3 EGF-1** | cyno | APPVCRAGCSLEHGFCEQPGECRCLEGWTGPLCM |
| 276 | - | **Ma DLL3 EGF-2** | cyno | GPGPCDGNPCANGGSCSETPGSFECTCPRGFYGLRCE |
| 277 | - | **Ma DLL3 EGF-3** | cyno | SGVTCADGPCFNGGLCVGGADPDSAYICHCPPGFQGSNCE |
| 278 | - | **Ma DLL3 EGF-4** | cyno | RVDRCSLQPCRNGGLCLDLGHALRCRCRAGFAGPRCE |
| 279 | - | **Ma DLL3 EGF-3+4** | cyno | SGVTCADGPCFNGGLCVGGADPDSAYICHCPPGFQGSNCEKRVDRC SLQPCRNGGLCLDLGHALRCRCRAGFAGPRCE |
| 280 | - | **Ma DLL3 EGF-5** | cyno | NLDDCAGRACANGGTCVEGGGAHRCSCALGFGGRDCR |
| 281 | - | **Ma DLL3 EGF-6** | cyno | RADPCAARPCAHGGRCYAHFSGLVCACAPGYMGSRCE |
| 282 | - | **Ma DLL3 ECD x EpCAM** | artificial | MVSPRMSRLLSQTVILALIFIPQARPAGVFELQIHSFGPGPGPGAP RSPCSARGPCRLFFRVCLKPGLSEEAAESPCALGAALSARGPVYTE QPEAPAPDLPLPNGLLQVPFRDAWPGTFSLIIETWREELGDQIGGP AWSLLARVTRRRRLAAGGPWARDIQRAGAWELRFSYRARCELPAVG TACTRLCRPRSAPSRCGPGLRPCAPLEDECEAPPVCRAGCSLEHGF |
| | | | | CEQPGECRCLEGWTGPLCMVPASTSSCLGLRGPSSATTGCLVPGPG PCDGNPCANGGSCSETPGSFECTCPRGFYGLRCEVSGVTCADGPCF NGGLCVGGADPDSAYICHCPPGFQGSNCEKRVDRCSLQPCRNGGLC LDLGHALRCRCRAGFAGPRCEHNLDDCAGRACANGGTCVEGGGAHR CSCALGFGGRDCRERADPCAARPCAHGGRCYAHFSGLVCACAPGYM GSRCEFPVHPDGVSALPAAPPGLRPGDPQRYLSGGGGSGAGVIAVI VVVVIAIVAGIVVLVISRKKRMAKYEKAEIKEMGEMHRELNA |
| 283 | - | **Human DLL1** | human | |
| 284 | - | **Human DLL4** | human | |
| 285 | - | **linker 1** | artificial | GGGG |
| 286 | - | **linker 2** | artificial | GGGGS |
| 287 | - | **linker 3** | artificial | GGGGQ |
| 288 | - | **linker 4** | artificial | SGGGGS |
| 289 | - | **linker 5** | artificial | PGGGGS |
| 290 | - | **linker 6** | artificial | PGGDGS |
| 291 | - | **linker 7** | artific | GGGGSGGGS |
| 292 | - | **linker 8** | artific | GGGGSGGGGS |
| 293 | | **linker 9** | artific | GGGGSGGGGSGGGGS |
| 294 | - | **Hexahis** | artificial | HHHHHH |
| 295 | - | **Ab156** | artific | RDWDFDVFGGGTPVGG |
| 296 | - | linear FcRn BP | artificial | QRFVTGHFGGLXPANG |
| 297 | - | linear FcRn BP-Y | artificial | QRFVTGHFGGLYPANG |
| 298 | - | linear FcRn BP-H | artificial | QRFVTGHFGGLHPANG |
| 299 | | core FcRn BP-H | artificial | TGHFGGLHP |
| 300 | | cyclic FcRn BP-H | artificial | QRFCTGHFGGLHPCNG |
| 301 | - | **HALB** | human | |
| 302 | - | **HALB variant 1** | artificial | DAHKSEVAHRFKDLGEENFKALVLIAFAQYLQQCPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALEVDETYVPKEFNAGT FTFHADICTLSEKERQIKKQTALVELVKHKPKATKEQLKAAMDDFA AFVEKCCKADDKETCFAEEGKKLVAASQAALGL |
| 303 | - | **HALB variant 2** | artificial | DAHKSEVAHRFKDLGEENFKALVLIAFAQYLQQCPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALEVDETYVPKEFNAET FTFHADICTLSEKERQIKKQTALVELVKHKPKATKEQLKAVMDDFA AFVEKCCKADDKETCFAEEGPKLVAASQAALGL |
| 304 | - | **HALB variant 3** | artificial | DAHKSEVAHRFKDLGEENFKALVLIAFAQYLQQCPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY |
| | | | | LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALDVDETYVPKEFNAET FTFHADICTLSEKERQIKKQTALVELVKHKPKATKEQLKAVMDDFA AFVEKCCKADDKETCFAEEGPHLVAASKAALGL |
| 305 | - | **HALB variant 4** | artificial | DAHKSEVAHRFKDLGEENFKALVLIAFAQYLQQCPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALGVDETYVPKEFNAET FTFHADICTLSEKERQIKKQTALVELVKHKPKATKEQLKAVMDKFA AFVEKCCKADDKETCFAEEGPKLVAASQAALGL |
| 306 | - | **HALB variant 5** | artificial | DAHKSEVAHRFKDLGEENFKALVLIAFAQYLQQCPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALDVDETYVPKEFNAET FTFHADICTLSEKERQIKKQTALVELVKHKPKATKEQLKAVMDKFA AFVEKCCKADDKETCFAEEGPKLVAASQAALGL |
| 307 | - | **HALB variant 6** | artificial | DAHKSEVAHRFKDLGEENFKALVLIAFAQYLQQCPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALEVDETYVPKEFNAET FTFHADICTLSEKERQIKKQTALVELVKHKPKATKEQLKAVMDKFA AFVEKCCKADDKETCFAEEGPHLVAASQAALGL |
| 308 | - | **HALB variant 7** | artificial | DAHKSEVAHRFKDLGEENFKALVLIAFAQYLQQCPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL |
| | | | | CVLHEKTPVSDRVTKCCTESLVNRRPCFSALEVDETYVPKEFNAET FTFHADICTLSEKERQIKKQTALVELVKHKPKATKEQLKAVMDDFA AFVEKCCKADDKETCFAEEGPHLVAASKAALGL |
| 309 | - | **HALB variant 8** | artificial | DAHKSEVAHRFKDLGEENFKALVLIAFAQYLQQCPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALEVDETYVPKEFNAET FTFHADICTLSEKERQIKKQTALVELVKHKPKATKEQLKAVMDKFA AFVEKCCKADDKETCFAEEGPKLVAASKAALGL |
| 310 | - | **HALB variant 9** | artificial | DAHKSEVAHRFKDLGEENFKALVLIAFAQYLQQCPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALDVDETYVPKEFNAET FTFHADICTLSEKERQIKKQTALVELVKHKPKATKEQLKAVMDDFA AFVEKCCKADDKETCFAEEGPKLVAASKAALGL |
| 311 | - | **HALB variant 10** | artificial | DAHKSEVAHRFKDLGEENFKALVLIAFAQYLQQSPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALEVDETYVPKEFNAET FTFHADICTLSEKERQIKKQTALVELVKHKPKATKEQLKAVMDDFA AFVEKCCKADDKETCFAEEGKKLVAASQAALGL |
| 312 | - | **HALB variant 11** | artificial | DAHKSEVAHRFKDLGEENFKALVLIAFAQYLQQSPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALEVDETYVPKEFNAGT FTFHADICTLSEKERQIKKQTALVELVKHKPKATKEQLKAAMDDFA AFVEKCCKADDKETCFAEEGKKLVAASQAALGL |
| 313 | - | **HALB variant 12** | artificial | DAHKSEVAHRFKDLGEENFKALVLIAFAQYLQQSPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE |
| | | | | LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALEVDETYVPKEFNAET FTFHADICTLSEKERQIKKQTALVELVKHKPKATKEQLKAVMDDFA AFVEKCCKADDKETCFAEEGPKLVAASQAALGL |
| 314 | - | **HALB variant 13** | artificial | DAHKSEVAHRFKDLGEENFKALVLIAFAQYLQQSPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALDVDETYVPKEFNAET FTFHADICTLSEKERQIKKQTALVELVKHKPKATKEQLKAVMDDFA AFVEKCCKADDKETCFAEEGPHLVAASKAALGL |
| 315 | - | **HALB variant 14** | artificial | DAHKSEVAHRFKDLGEENFKALVLIAFAQYLQQSPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALGVDETYVPKEFNAET FTFHADICTLSEKERQIKKQTALVELVKHKPKATKEQLKAVMDKFA AFVEKCCKADDKETCFAEEGPKLVAASQAALGL |
| 316 | - | **HALB variant 15** | artificial | DAHKSEVAHRFKDLGEENFKALVLIAFAQYLQQSPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALDVDETYVPKEFNAET FTFHADICTLSEKERQIKKQTALVELVKHKPKATKEQLKAVMDKFA AFVEKCCKADDKETCFAEEGPKLVAASQAALGL |
| 317 | - | **HALB variant 16** | artificial | DAHKSEVAHRFKDLGEENFKALVLIAFAQYLQQSPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALEVDETYVPKEFNAET |
| | | | | FTFHADICTLSEKERQIKKQTALVELVKHKPKATKEQLKAVMDKFA AFVEKCCKADDKETCFAEEGPHLVAASQAALGL |
| 318 | - | **HALB variant 17** | artificial | DAHKSEVAHRFKDLGEENFKALVLIAFAQYLQQSPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALEVDETYVPKEFNAET FTFHADICTLSEKERQIKKQTALVELVKHKPKATKEQLKAVMDDFA AFVEKCCKADDKETCFAEEGPHLVAASKAALGL |
| 319 | - | **HALB variant 18** | artificial | DAHKSEVAHRFKDLGEENFKALVLIAFAQYLQQSPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALEVDETYVPKEFNAET FTFHADICTLSEKERQIKKQTALVELVKHKPKATKEQLKAVMDKFA AFVEKCCKADDKETCFAEEGPKLVAASKAALGL |
| 320 | - | **HALB variant 19** | artificial | DAHKSEVAHRFKDLGEENFKALVLIAFAQYLQQSPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALDVDETYVPKEFNAET FTFHADICTLSEKERQIKKQTALVELVKHKPKATKEQLKAVMDDFA AFVEKCCKADDKETCFAEEGPKLVAASKAALGL |
| 321 | - | **HALB variant 20** | artificial | DAHKSEVAHRFKDLGEENFKALVLIAFAQYLQQAPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALEVDETYVPKEFNAET FTFHADICTLSEKERQIKKQTALVELVKHKPKATKEQLKAVMDDFA AFVEKCCKADDKETCFAEEGKKLVAASQAALGL |
| 322 | - | **HALB variant 21** | artificial | DAHKSEVAHRFKDLGEENFKALVLIAFAQYLQQAPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE |
| | | | | VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALEVDETYVPKEFNAGT FTFHADICTLSEKERQIKKQTALVELVKHKPKATKEQLKAAMDDFA AFVEKCCKADDKETCFAEEGKKLVAASQAALGL |
| 323 | - | **HALB variant 22** | artificial | DAHKSEVAHRFKDLGEENFKALVLIAFAQYLQQAPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALEVDETYVPKEFNAET FTFHADICTLSEKERQIKKQTALVELVKHKPKATKEQLKAVMDDFA AFVEKCCKADDKETCFAEEGPKLVAASQAALGL |
| 324 | - | **HALB variant 23** | artificial | DAHKSEVAHRFKDLGEENFKALVLIAFAQYLQQAPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALDVDETYVPKEFNAET FTFHADICTLSEKERQIKKQTALVELVKHKPKATKEQLKAVMDDFA AFVEKCCKADDKETCFAEEGPHLVAASKAALGL |
| 325 | - | **HALB variant 24** | artificial | DAHKSEVAHRFKDLGEENFKALVLIAFAQYLQQAPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALGVDETYVPKEFNAET FTFHADICTLSEKERQIKKQTALVELVKHKPKATKEQLKAVMDKFA AFVEKCCKADDKETCFAEEGPKLVAASQAALGL |
| 326 | - | **HALB variant 25** | artificial | DAHKSEVAHRFKDLGEENFKALVLIAFAQYLQQAPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALDVDETYVPKEFNAET FTFHADICTLSEKERQIKKQTALVELVKHKPKATKEQLKAVMDKFA |
| | | | | AFVEKCCKADDKETCFAEEGPKLVAASQAALGL |
| 327 | - | **HALB variant 26** | artificial | DAHKSEVAHRFKDLGEENFKALVLIAFAQYLQQAPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALEVDETYVPKEFNAET FTFHADICTLSEKERQIKKQTALVELVKHKPKATKEQLKAVMDKFA AFVEKCCKADDKETCFAEEGPHLVAASQAALGL |
| 328 | - | **HALB variant 27** | artificial | DAHKSEVAHRFKDLGEENFKALVLIAFAQYLQQAPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALEVDETYVPKEFNAET FTFHADICTLSEKERQIKKQTALVELVKHKPKATKEQLKAVMDDFA AFVEKCCKADDKETCFAEEGPHLVAASKAALGL |
| 329 | - | **HALB variant 28** | artificial | DAHKSEVAHRFKDLGEENFKALVLIAFAQYLQQAPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALEVDETYVPKEFNAET FTFHADICTLSEKERQIKKQTALVELVKHKPKATKEQLKAVMDKFA AFVEKCCKADDKETCFAEEGPKLVAASKAALGL |
| 330 | - | **HALB variant 29** | artificial | DAHKSEVAHRFKDLGEENFKALVLIAFAQYLQQAPFEDHVKLVNEV TEFAKTCVADESAENCDKSLHTLFGDKLCTVATLRETYGEMADCCA KQEPERNECFLQHKDDNPNLPRLVRPEVDVMCTAFHDNEETFLKKY LYEIARRHPYFYAPELLFFAKRYKAAFTECCQAADKAACLLPKLDE LRDEGKASSAKQRLKCASLQKFGERAFKAWAVARLSQRFPKAEFAE VSKLVTDLTKVHTECCHGDLLECADDRADLAKYICENQDSISSKLK ECCEKPLLEKSHCIAEVENDEMPADLPSLAADFVESKDVCKNYAEA KDVFLGMFLYEYARRHPDYSVVLLLRLAKTYETTLEKCCAAADPHE CYAKVFDEFKPLVEEPQNLIKQNCELFEQLGEYKFQNALLVRYTKK VPQVSTPTLVEVSRNLGKVGSKCCKHPEAKRMPCAEDYLSVVLNQL CVLHEKTPVSDRVTKCCTESLVNRRPCFSALDVDETYVPKEFNAET FTFHADICTLSEKERQIKKQTALVELVKHKPKATKEQLKAVMDDFA AFVEKCCKADDKETCFAEEGPKLVAASKAALGL |
| 331 | - | **Cross body 1 HC** | artificial | ASTKGPSVFPLAPSSKSTSGGTAALGCLVKDYFPEPVTVSWNSGAL TSGVHTFPAVLQSSGLYSLSSVVTVPSSSLGTQTYICNVNHKPSNT KVDKKVEPKSCDKTHTCPPCPAPELLGGPSVFLFPPKPKDTLMISR TPEVTCVVVDVSHEDPEVKFNWYVDGVEVHNAKTKPCEEQYGSTYR CVSVLTVLHQDWLNGKEYKCKVSNKALPAPIEKTISKAKGQPREPQ VYTLPPSREEMTKNQVSLTCLVKGFYPSDIAVEWESNGQPENNYDT |
| | | | | TPPVLDSDGSFFLYSDLTVDKSRWQQGNVFSCSVMHEALHNHYTQK SLSLSPGK |
| 332 | - | **Cross body 1 LC** | artificial | GQPKAAPSVTLFPPSSEELQANKATLVCLISDFYPGAVTVAWKADS SPVKAGVETTTPSKQSNNKYAASSYLSLTPEQWKSHRSYSCQVTHE GSTVEKTVAPTECSDKTHTCPPCPAPELLGGPSVFLFPPKPKDTLM ISRTPEVTCVVVDVSHEDPEVKFNWYVDGVEVHNAKTKPCEEQYGS TYRCVSVLTVLHQDWLNGKEYKCKVSNKALPAPIEKTISKAKGQPR EPQVYTLPPSRKEMTKNQVSLTCLVKGFYPSDIAVEWESNGQPENN YKTTPPVLKSDGSFFLYSKLTVDKSRWQQGNVFSCSVMHEALHNHY TQKSLSLSPGK |
| 333 | - | **Cross body 2 HC** | artificial | ASTKGPSVFPLAPCSRSTSESTAALGCLVKDYFPEPVTVSWNSGAL TSGVHTFPAVLQSSGLYSLSSVVTVPSSNFGTQTYTCNVDHKPSNT KVDKTVEPKSSDKTHTCPPCPAPEAAGGPSVFLFPPKPKDTLMISR TPEVTCVVVDVSHEDPEVKFNWYVDGVEVHNAKTKPREEQYNSTYR VVSVLTVLHQDWLNGKEYKCKVSNKALPAPIEKTISKAKGQPREPQ VYTLPPSREEMTKNQVSLTCLVKGFYPSDIAVEWESNGQPENNYDT TPPVLDSDGSFFLYSDLTVDKSRWQQGNVFSCSVMHEALHNHYTQK SLSLSPGK |
| 334 | - | **Cross body 2 LC** | artificial | GQPKAAPSVTLFPPSSEELQANKATLVCLISDFYPGAVTVAWKADS SPVKAGVETTTPSKQSNNKYAASSYLSLTPEQWKSHRSYSCQVTHE GSTVEKTVAPTECSEPKSSDKTHTCPPCPAPEAAGGPSVFLFPPKP KDTLMISRTPEVTCVVVDVSHEDPEVKFNWYVDGVEVHNAKTKPRE EQYNSTYRVVSVLTVLHQDWLNGKEYKCKVSNKALPAPIEKTISKA KGQPREPQVYTLPPSRKEMTKNQVSLTCLVKGFYPSDIAVEWESNG QPENNYKTTPPVLKSDGSFFLYSKLTVDKSRWQQGNVFSCSVMHEA LHNHYTQKSLSLSPGK |
| 335 | - | **Hetero -Fc binder Fc** | artificial | DKTHTCPPCPAPELLGGPSVFLFPPKPKDTLMISRTPEVTCVVVDV SHEDPEVKFNWYVDGVEVHNAKTKPCEEQYGSTYRCVSVLTVLHQD WLNGKEYKCKVSNKALPAPIEKTISKAKGQPREPQVYTLPPSRKEM TKNQVSLTCLVKGFYPSDIAVEWESNGQPENNYKTTPPVLKSDGSF FLYSKLTVDKSRWQQGNVFSCSVMHEALHNHYTQKSLSLSPGK |
| 336 | - | **Hetero -Fc partner Fc** | artificial | DKTHTCPPCPAPELLGGPSVFLFPPKPKDTLMISRTPEVTCVVVDV SHEDPEVKFNWYVDGVEVHNAKTKPCEEQYGSTYRCVSVLTVLHQD WLNGKEYKCKVSNKALPAPIEKTISKAKGQPREPQVYTLPPSREEM TKNQVSLTCLVKGFYPSDIAVEWESNGQPENNYDTTPPVLDSDGSF FLYSDLTVDKSRWQQGNVFSCSVMHEALHNHYTQKSLSLSPGK |
| 337 | - | **Maxi-body 1 target Fc** | artificial | EPKSSDKTHTCPPCPAPELLGGPSVFLFPPKPKDTLMISRTPEVTC VVVDVSHEDPEVKFNWYVDGVEVHNAKTKPCEEQYGSTYRCVSVLT VLHQDWLNGKEYKCKVSNKALPAPIEKTISKAKGQPREPQVYTLPP SRKEMTKNQVSLTCLVKGFYPSDIAVEWESNGQPENNYKTTPPVLK SDGSFFLYSKLTVDKSRWQQGNVFSCSVMHEALHNHYTQKSLSLSP GK |
| 338 | - | **Maxi-body 1 CD3 Fc** | artificial | EPKSSDKTHTCPPCPAPELLGGPSVFLFPPKPKDTLMISRTPEVTC VVVDVSHEDPEVKFNWYVDGVEVHNAKTKPCEEQYGSTYRCVSVLT VLHQDWLNGKEYKCKVSNKALPAPIEKTISKAKGQPREPQVYTLPP SREEMTKNQVSLTCLVKGFYPSDIAVEWESNGQPENNYDTTPPVLD SDGSFFLYSDLTVDKSRWQQGNVFSCSVMHEALHNHYTQKSLSLSP GK |
| 339 | - | **Maxi-body 2 target Fc** | artificial | EPKSSDKTHTCPPCPAPEAAGGPSVFLFPPKPKDTLMISRTPEVTC VVVDVSHEDPEVKFNWYVDGVEVHNAKTKPREEQYNSTYRVVSVLT VLHQDWLNGKEYKCKVSNKALPAPIEKTISKAKGQPREPQVYTLPP SRKEMTKNQVSLTCLVKGFYPSDIAVEWESNGQPENNYKTTPPVLK SDGSFFLYSKLTVDKSRWQQGNVFSCSVMHEALHNHYTQKSLSLSP GK |
| 340 | - | **Maxi-body 2 CD3 Fc** | artificial | EPKSSDKTHTCPPCPAPEAAGGPSVFLFPPKPKDTLMISRTPEVTC VVVDVSHEDPEVKFNWYVDGVEVHNAKTKPREEQYNSTYRVVSVLT VLHQDWLNGKEYKCKVSNKALPAPIEKTISKAKGQPREPQVYTLPP SREEMTKNQVSLTCLVKGFYPSDIAVEWESNGQPENNYDTTPPVLD SDGSFFLYSDLTVDKSRWQQGNVFSCSVMHEALHNHYTQKSLSLSP |
| | | | | GK |
| 341 | - | **Mono Fc** | artificial | APELLGGPSVFLFPPKPKDTLMISRTPEVTCVVVDVSHEDPEVKFN WYVDGVEVHNAKTKPREEQYNSTYRVVSVLTVLHQDWLNGKEYKCK VSNKALPAPIEKTISKAKGQPREPQVTTLPPSREEMTKNQVSLTCL VKGFYPSDIAVEWESNGQPENNYDTTPPVLDSDGSFFLYSDLTVDK SRWQQGNVFSCSVMHEALHNHYTQKSLSLSPGK |
| 342 | - | CDR-L1 of F6A | artificial | GSSTGAVTSGYYPN |
| 343 | | CDR-L2 of F6A | artificial | GTKFLAP |
| 344 | | CDR-L3 of F6A | artificial | ALWYSNRWV |
| 345 | | CDR-H1 of F6A | artificial | IYAMN |
| 346 | | CDR-H2 of F6A | artificial | RIRSKYNNYATYYADSVKS |
| 347 | | CDR-H3 of F6A | artificial | HGNFGNSYVSFFAY |
| 348 | | VH of F6A | artificial | EVQLVESGGGLVQPGGSLKLSCAASGFTFNIYAMNWVRQAPGKGLE WVARIRSKYNNYATYYADSVKSRFTISRDDSKNTAYLQMNNLKTED TAVYYCVRHGNFGNSYVSFFAYWGQGTLVTVSS |
| 349 | | VL of F6A | artificial | QTVVTQEPSLTVSPGGTVTLTCGSSTGAVTSGYYPNWVQQKPGQAP RGLIGGTKFLAPGTPARFSGSLLGGKAALTLSGVQPEDEAEYYCAL WYSNRWVFGGGTKLTVL |
| 350 | | VH-VL of F6A | artificial | EVQLVESGGGLVQPGGSLKLSCAASGFTFNIYAMNWVRQAPGKGLE WVARIRSKYNNYATYYADSVKSRFTISRDDSKNTAYLQMNNLKTED TAVYYCVRHGNFGNSYVSFFAYWGQGTLVTVSSGGGGSGGGGSGGG GSQTVVTQEPSLTVSPGGTVTLTCGSSTGAVTSGYYPNWVQQKPGQ APRGLIGGTKFLAPGTPARFSGSLLGGKAALTLSGVQPEDEAEYYC ALWYSNRWVFGGGTKLTVL |
| 351 | | CDR-L1 of H2C | artificial | GSSTGAVTSGYYPN |
| 352 | | CDR-L2 of H2C | artificial | GTKFLAP |
| 353 | | CDR-L3 of H2C | artificial | ALWYSNRWV |
| 354 | | CDR-H1 of H2C | artificial | KYAMN |
| 355 | | CDR-H2 of H2C | artificial | RIRSKYNNYATYYADSVKD |
| 356 | | CDR-H3 of H2C | artificial | HGNFGNSYISYWAY |
| 357 | | VH of H2C | artificial | EVQLVESGGGLVQPGGSLKLSCAASGFTFNKYAMNWVRQAPGKGLE WVARIRSKYNNYATYYADSVKDRFTISRDDSKNTAYLQMNNLKTED TAVYYCVRHGNFGNSYISYWAYWGQGTLVTVSS |
| 358 | | VL of H2C | artificial | QTVVTQEPSLTVSPGGTVTLTCGSSTGAVTSGYYPNWVQQKPGQAP RGLIGGTKFLAPGTPARFSGSLLGGKAALTLSGVQPEDEAEYYCAL WYSNRWVFGGGTKLTVL |
| 359 | | VH-VL of H2C | artificial | EVQLVESGGGLVQPGGSLKLSCAASGFTFNKYAMNWVRQAPGKGLE WVARIRSKYNNYATYYADSVKDRFTISRDDSKNTAYLQMNNLKTED TAVYYCVRHGNFGNSYISYWAYWGQGTLVTVSSGGGGSGGGGSGGG GSQTVVTQEPSLTVSPGGTVTLTCGSSTGAVTSGYYPNWVQQKPGQ APRGLIGGTKFLAPGTPARFSGSLLGGKAALTLSGVQPEDEAEYYC ALWYSNRWVFGGGTKLTVL |
| 360 | | CDR-L1 of H1E | artificial | GSSTGAVTSGYYPN |
| 361 | | CDR-L2 of H1E | artificial | GTKFLAP |
| 362 | | CDR-L3 of H1E | artificial | ALWYSNRWV |
| 363 | | CDR-H1 of H1E | artificial | SYAMN |
| 364 | | CDR-H2 of H1E | artificial | RIRSKYNNYATYYADSVKG |
| 365 | | CDR-H3 of H1E | artificial | HGNFGNSYLSFWAY |
| 366 | | VH of H1E | artificial | EVQLVESGGGLEQPGGSLKLSCAASGFTFNSYAMNWVRQAPGKGLE WVARIRSKYNNYATYYADSVKGRFTISRDDSKNTAYLQMNNLKTED TAVYYCVRHGNFGNSYLSFWAYWGQGTLVTVSS |
| 367 | | VL of H1E | artificial | QTVVTQEPSLTVSPGGTVTLTCGSSTGAVTSGYYPNWVQQKPGQAP RGLIGGTKFLAPGTPARFSGSLLGGKAALTLSGVQPEDEAEYYCAL WYSNRWVFGGGTKLTVL |
| 368 | | VH-VL of H1E | artificial | EVQLVESGGGLEQPGGSLKLSCAASGFTFNSYAMNWVRQAPGKGLE WVARIRSKYNNYATYYADSVKGRFTISRDDSKNTAYLQMNNLKTED TAVYYCVRHGNFGNSYLSFWAYWGQGTLVTVSSGGGGSGGGGSGGG GSQTVVTQEPSLTVSPGGTVTLTCGSSTGAVTSGYYPNWVQQKPGQ APRGLIGGTKFLAPGTPARFSGSLLGGKAALTLSGVQPEDEAEYYC ALWYSNRWVFGGGTKLTVL |
| 369 | | CDR-L1 of G4H | artificial | GSSTGAVTSGYYPN |
| 370 | | CDR-L2 of G4H | artificial | GTKFLAP |
| 371 | | CDR-L3 of G4H | artificial | ALWYSNRWV |
| 372 | | CDR-H1 of G4H | artificial | RYAMN |
| 373 | | CDR-H2 of G4H | artificial | RIRSKYNNYATYYADSVKG |
| 374 | | CDR-H3 of G4H | artificial | HGNFGNSYLSYFAY |
| 375 | | VH of G4H | artificial | EVQLVESGGGLVQPGGSLKLSCAASGFTFNRYAMNWVRQAPGKGLE WVARIRSKYNNYATYYADSVKGRFTISRDDSKNTAYLQMNNLKTED TAVYYCVRHGNFGNSYLSYFAYWGQGTLVTVSS |
| 376 | | VL of G4H | artificial | QTVVTQEPSLTVSPGGTVTLTCGSSTGAVTSGYYPNWVQQKPGQAP RGLIGGTKFLAPGTPARFSGSLLGGKAALTLSGVQPEDEAEYYCAL WYSNRWVFGGGTKLTVL |
| 377 | | VH-VL of G4H | artificial | EVQLVESGGGLVQPGGSLKLSCAASGFTFNRYAMNWVRQAPGKGLE WVARIRSKYNNYATYYADSVKGRFTISRDDSKNTAYLQMNNLKTED TAVYYCVRHGNFGNSYLSYFAYWGQGTLVTVSSGGGGSGGGGSGGG GSQTVVTQEPSLTVSPGGTVTLTCGSSTGAVTSGYYPNWVQQKPGQ APRGLIGGTKFLAPGTPARFSGSLLGGKAALTLSGVQPEDEAEYYC ALWYSNRWVFGGGTKLTVL |
| 378 | | CDR-L1 of A2J | artificial | RSSTGAVTSGYYPN |
| 379 | | CDR-L2 of A2J | artificial | ATDMRPS |
| 380 | | CDR-L3 of A2J | artificial | ALWYSNRWV |
| 381 | | CDR-H1 of A2J | artificial | VYAMN |
| 382 | | CDR-H2 of A2J | artificial | RIRSKYNNYATYYADSVKK |
| 383 | | CDR-H3 of A2J | artificial | HGNFGNSYLSWWAY |
| 384 | | VH of A2J | artificial | EVQLVESGGGLVQPGGSLKLSCAASGFTFNVYAMNWVRQAPGKGLE WVARIRSKYNNYATYYADSVKKRFTISRDDSKNTAYLQMNNLKTED |
| | | | | TAVYYCVRHGNFGNSYLSWWAYWGQGTLVTVSS |
| 385 | | VL of A2J | artificial | QTVVTQEPSLTVSPGGTVTLTCRSSTGAVTSGYYPNWVQQKPGQAP RGLIGATDMRPSGTPARFSGSLLGGKAALTLSGVQPEDEAEYYCAL WYSNRWVFGGGTKLTVL |
| 386 | | VH-VL of A2J | artificial | EVQLVESGGGLVQPGGSLKLSCAASGFTFNVYAMNWVRQAPGKGLE WVARIRSKYNNYATYYADSVKKRFTISRDDSKNTAYLQMNNLKTED TAVYYCVRHGNFGNSYLSWWAYWGQGTLVTVSSGGGGSGGGGSGGG GSQTVVTQEPSLTVSPGGTVTLTCRSSTGAVTSGYYPNWVQQKPGQ APRGLIGATDMRPSGTPARFSGSLLGGKAALTLSGVQPEDEAEYYC ALWYSNRWVFGGGTKLTVL |
| 387 | | CDR-L1 of E1L | artificial | GSSTGAVTSGYYPN |
| 388 | | CDR-L2 of E1L | artificial | GTKFLAP |
| 389 | | CDR-L3 of E1L | artificial | ALWYSNRWV |
| 390 | | CDR-H1 of E1L | artificial | KYAMN |
| 391 | | CDR-H2 of E1L | artificial | RIRSKYNNYATYYADSVKS |
| 392 | | CDR-H3 of E1L | artificial | HGNFGNSYTSYYAY |
| 393 | | VH of E1L | artificial | EVQLVESGGGLVQPGGSLKLSCAASGFTFNKYAMNWVRQAPGKGLE WVARIRSKYNNYATYYADSVKSRFTISRDDSKNTAYLQMNNLKTED TAVYYCVRHGNFGNSYTSYYAYWGQGTLVTVSS |
| 394 | | VL of E1L | artificial | QTVVTQEPSLTVSPGGTVTLTCGSSTGAVTSGYYPNWVQQKPGQAP RGLIGGTKFLAPGTPARFSGSLLGGKAALTLSGVQPEDEAEYYCAL WYSNRWVFGGGTKLTVL |
| 395 | | VH-VL of E1L | artificial | EVQLVESGGGLVQPGGSLKLSCAASGFTFNKYAMNWVRQAPGKGLE WVARIRSKYNNYATYYADSVKSRFTISRDDSKNTAYLQMNNLKTED TAVYYCVRHGNFGNSYTSYYAYWGQGTLVTVSSGGGGSGGGGSGGG GSQTVVTQEPSLTVSPGGTVTLTCGSSTGAVTSGYYPNWVQQKPGQ APRGLIGGTKFLAPGTPARFSGSLLGGKAALTLSGVQPEDEAEYYC ALWYSNRWVFGGGTKLTVL |
| 396 | | CDR-L1 of E2M | artificial | RSSTGAVTSGYYPN |
| 397 | | CDR-L2 of E2M | artificial | ATDMRPS |
| 398 | | CDR-L3 of E2M | artificial | ALWYSNRWV |
| 399 | | CDR-H1 of E2M | artificial | GYAMN |
| 400 | | CDR-H2 of E2M | artificial | RIRSKYNNYATYYADSVKE |
| 401 | | CDR-H3 of E2M | artificial | HRNFGNSYLSWFAY |
| 402 | | VH of E2M | artificial | EVQLVESGGGLVQPGGSLKLSCAASGFTFNGYAMNWVRQAPGKGLE WVARIRSKYNNYATYYADSVKERFTISRDDSKNTAYLQMNNLKTED TAVYYCVRHRNFGNSYLSWFAYWGQGTLVTVSS |
| 403 | | VL of E2M | artificial | QTVVTQEPSLTVSPGGTVTLTCRSSTGAVTSGYYPNWVQQKPGQAP RGLIGATDMRPSGTPARFSGSLLGGKAALTLSGVQPEDEAEYYCAL WYSNRWVFGGGTKLTVL |
| 404 | | VH-VL of E2M | artificial | EVQLVESGGGLVQPGGSLKLSCAASGFTFNGYAMNWVRQAPGKGLE WVARIRSKYNNYATYYADSVKERFTISRDDSKNTAYLQMNNLKTED TAVYYCVRHRNFGNSYLSWFAYWGQGTLVTVSSGGGGSGGGGSGGG GSQTVVTQEPSLTVSPGGTVTLTCRSSTGAVTSGYYPNWVQQKPGQ APRGLIGATDMRPSGTPARFSGSLLGGKAALTLSGVQPEDEAEYYC ALWYSNRWVFGGGTKLTVL |
| 405 | | CDR-L1 of F7O | artificial | GSSTGAVTSGYYPN |
| 406 | | CDR-L2 of F7O | artificial | GTKFLAP |
| 407 | | CDR-L3 of F7O | artificial | ALWYSNRWV |
| 408 | | CDR-H1 of F7O | artificial | VYAMN |
| 409 | | CDR-H2 of F7O | artificial | RIRSKYNNYATYYADSVKK |
| 410 | | CDR-H3 of F7O | artificial | HGNFGNSYISWWAY |
| 411 | | VH of F7O | artificial | EVQLVESGGGLVQPGGSLKLSCAASGFTFNVYAMNWVRQAPGKGLE WVARIRSKYNNYATYYADSVKKRFTISRDDSKNTAYLQMNNLKTED TAVYYCVRHGNFGNSYISWWAYWGQGTLVTVSS |
| 412 | | VL of F7O | artificial | QTVVTQEPSLTVSPGGTVTLTCGSSTGAVTSGYYPNWVQQKPGQAP RGLIGGTKFLAPGTPARFSGSLLGGKAALTLSGVQPEDEAEYYCAL WYSNRWVFGGGTKLTVL |
| 413 | | VH-VL of F7O | artificial | EVQLVESGGGLVQPGGSLKLSCAASGFTFNVYAMNWVRQAPGKGLE WVARIRSKYNNYATYYADSVKKRFTISRDDSKNTAYLQMNNLKTED TAVYYCVRHGNFGNSYISWWAYWGQGTLVTVSSGGGGSGGGGSGGG GSQTVVTQEPSLTVSPGGTVTLTCGSSTGAVTSGYYPNWVQQKPGQ APRGLIGGTKFLAPGTPARFSGSLLGGKAALTLSGVQPEDEAEYYC ALWYSNRWVFGGGTKLTVL |
| 414 | | CDR-L1 of F12Q | artificial | GSSTGAVTSGNYPN |
| 415 | | CDR-L2 of F12Q | artificial | GTKFLAP |
| 416 | | CDR-L3 of F12Q | artificial | VLWYSNRWV |
| 417 | | CDR-H1 of F12Q | artificial | SYAMN |
| 418 | | CDR-H2 of F12Q | artificial | RIRSKYNNYATYYADSVKG |
| 419 | | CDR-H3 of F12Q | artificial | HGNFGNSYVSWWAY |
| 420 | | VH of F12Q | artificial | EVQLVESGGGLVQPGGSLKLSCAASGFTFNSYAMNWVRQAPGKGLE WVARIRSKYNNYATYYADSVKGRFTISRDDSKNTAYLQMNNLKTED TAVYYCVRHGNFGNSYVSWWAYWGQGTLVTVSS |
| 421 | | VL of F12Q | artificial | QTVVTQEPSLTVSPGGTVTLTCGSSTGAVTSGNYPNWVQQKPGQAP RGLIGGTKFLAPGTPARFSGSLLGGKAALTLSGVQPEDEAEYYCVL WYSNRWVFGGGTKLTVL |
| 422 | | VH-VL of F12Q | artificial | EVQLVESGGGLVQPGGSLKLSCAASGFTFNSYAMNWVRQAPGKGLE WVARIRSKYNNYATYYADSVKGRFTISRDDSKNTAYLQMNNLKTED TAVYYCVRHGNFGNSYVSWWAYWGQGTLVTVSSGGGGSGGGGSGGG GSQTVVTQEPSLTVSPGGTVTLTCGSSTGAVTSGNYPNWVQQKPGQ APRGLIGGTKFLAPGTPARFSGSLLGGKAALTLSGVQPEDEAEYYC VLWYSNRWVFGGGTKLTVL |
| 423 | | CDR-L1 of I2C | artificial | GSSTGAVTSGNYPN |
| 424 | | CDR-L2 of I2C | artificial | GTKFLAP |
| 425 | | CDR-L3 of I2C | artificial | VLWYSNRWV |
| 426 | | CDR-H1 of I2C | artificial | KYAMN |
| 427 | | CDR-H2 of I2C | artificial | RIRSKYNNYATYYADSVKD |
| 428 | | CDR-H3 of I2C | artificial | HGNFGNSYISYWAY |
| 429 | | VH of I2C | artificial | EVQLVESGGGLVQPGGSLKLSCAASGFTFNKYAMNWVRQAPGKGLE WVARIRSKYNNYATYYADSVKDRFTISRDDSKNTAYLQMNNLKTED TAVYYCVRHGNFGNSYISYWAYWGQGTLVTVSS |
| 430 | | VL of I2C | artificial | QTVVTQEPSLTVSPGGTVTLTCGSSTGAVTSGNYPNWVQQKPGQAP RGLIGGTKFLAPGTPARFSGSLLGGKAALTLSGVQPEDEAEYYCVL WYSNRWVFGGGTKLTVL |
| 431 | | VH-VL of I2C | artificial | EVQLVESGGGLVQPGGSLKLSCAASGFTFNKYAMNWVRQAPGKGLE WVARIRSKYNNYATYYADSVKDRFTISRDDSKNTAYLQMNNLKTED TAVYYCVRHGNFGNSYISYWAYWGQGTLVTVSSGGGGSGGGGSGGG GSQTVVTQEPSLTVSPGGTVTLTCGSSTGAVTSGNYPNWVQQKPGQ APRGLIGGTKFLAPGTPARFSGSLLGGKAALTLSGVQPEDEAEYYC VLWYSNRWVFGGGTKLTVL |
| 432 | | VH of F12q | artificial | EVQLVESGGGLVQPGGSLRLSCAASGFTFNSYAMNWVRQAPGKGLE WVARIRSKYNNYATYYADSVKGRFTISRDDSKNTAYLQMNSLKTED TAVYYCVRHGNFGNSYVSWWAYWGQGTLVTVSS |
| 433 | | VL of F12q | artificial | QTVVTQEPSLTVSPGGTVTLTCGSSTGAVTSGNYPNWVQQKPGQAP RGLIGGTKFLAPGTPARFSGSLLGGKAALTLSGVQPEDEAEYYCVL WYSNRWVFGGGTKLTVL |
| 434 | | VH-VL of F12q | artificial | EVQLVESGGGLVQPGGSLRLSCAASGFTFNSYAMNWVRQAPGKGLE WVARIRSKYNNYATYYADSVKGRFTISRDDSKNTAYLQMNSLKTED TAVYYCVRHGNFGNSYVSWWAYWGQGTLVTVSSGGGGSGGGGSGGG GSQTVVTQEPSLTVSPGGTVTLTCGSSTGAVTSGNYPNWVQQKPGQ APRGLIGGTKFLAPGTPARFSGSLLGGKAALTLSGVQPEDEAEYYC VLWYSNRWVFGGGTKLTVL |
| | | | | |
| 435 | | **DLL3-4-001 (G44C)** | VH | QVQLQESGPGLVKPSETLSLTCTVSGGSISSYYWSWIRQPPGKCLE WIGYVYYSGTTNYNPSLKSRVTISVDTSKNQFSLKLSSVTAADTAV YYCASIAVTGFYFDYWGQGTLVTVSS |
| 436 | | **DLL3-4-001 (G234C)** | VL | EIVLTQSPGTLSLSPGERVTLSCRASQRVNNNYLAWYQQRPGQAPR LLIYGASSRATGIPDRFSGSGSGTDFTLTISRLEPEDFAVYYCQQY DRSPLTFGCGTKLEIK |
| 437 | | **DLL3-4-001 (G44C-G243C)** | scFv | QVQLQESGPGLVKPSETLSLTCTVSGGSISSYYWSWIRQPPGKCLE WIGYVYYSGTTNYNPSLKSRVTISVDTSKNQFSLKLSSVTAADTAV YYCASIAVTGFYFDYWGQGTLVTVSSGGGGSGGGGSGGGGSEIVLT QSPGTLSLSPGERVTLSCRASQRVNNNYLAWYQQRPGQAPRLLIYG ASSRATGIPDRFSGSGSGTDFTLTISRLEPEDFAVYYCQQYDRSPL TFGCGTKLEIK |
| 438 | | **DLL3-4-001 (CC) xI2C** | bispecific molecule | QVQLQESGPGLVKPSETLSLTCTVSGGSISSYYWSWIRQPPGKCLE WIGYVYYSGTTNYNPSLKSRVTISVDTSKNQFSLKLSSVTAADTAV YYCASIAVTGFYFDYWGQGTLVTVSSGGGGSGGGGSGGGGSEIVLT QSPGTLSLSPGERVTLSCRASQRVNNNYLAWYQQRPGQAPRLLIYG ASSRATGIPDRFSGSGSGTDFTLTISRLEPEDFAVYYCQQYDRSPL TFGCGTKLEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVL |
| 439 | | **DLL3-14 - D55E** | VH-CDR2 | IINPSEGSTSYAQKFQG |
| 440 | | **DLL3-14 - G56A** | VH-CDR2 | IINPSDASTSYAQKFQG |
| 441 | | **DLL3-14 - D171E** | VL-CDR1 | RSSQSLVYREGNTYLS |
| 442 | | **DLL3-14 - G172A** | VL-CDR1 | RSSQSLVYRDANTYLS |
| 443 | | **DLL3-14 - N173Q** | VL-CDR1 | RSSQSLVYRDGQTYLS |
| 444 | | **DLL3-14 - T174A** | VL-CDR1 | RSSQSLVYRDGNAYLS |
| | | | | |
| 445 | | **DLL3-14 - L43Q** | VH | QVQLVQSGAEVKKPGASVKVSCKASGYTFTNYYMHWVRQAPGQGLE WMGIINPSDGSTSYAQKFQGRVTMTRDTSTNTVYMDLSSLRSEDTA VYYCARGGNSAFYSYYDMDVWGQGTTVTVSS |
| 446 | | **DLL3-14 - D55E** | VH | QVQLVQSGAEVKKPGASVKVSCKASGYTFTNYYMHWVRQAPGLGLE WMGIINPSEGSTSYAQKFQGRVTMTRDTSTNTVYMDLSSLRSEDTA VYYCARGGNSAFYSYYDMDVWGQGTTVTVSS |
| 447 | | **DLL3-14 - G56A** | VH | QVQLVQSGAEVKKPGASVKVSCKASGYTFTNYYMHWVRQAPGLGLE WMGIINPSDASTSYAQKFQGRVTMTRDTSTNTVYMDLSSLRSEDTA VYYCARGGNSAFYSYYDMDVWGQGTTVTVSS |
| 448 | | **DLL3-14 - L43Q-D55E** | VH | QVQLVQSGAEVKKPGASVKVSCKASGYTFTNYYMHWVRQAPGQGLE WMGIINPSEGSTSYAQKFQGRVTMTRDTSTNTVYMDLSSLRSEDTA VYYCARGGNSAFYSYYDMDVWGQGTTVTVSS |
| 449 | | **DLL3-14** - **L43Q-G56A** | VH | QVQLVQSGAEVKKPGASVKVSCKASGYTFTNYYMHWVRQAPGQGLE WMGIINPSDASTSYAQKFQGRVTMTRDTSTNTVYMDLSSLRSEDTA VYYCARGGNSAFYSYYDMDVWGQGTTVTVS |
| 450 | | **DLL3-14 -** G44C | VH | QVQLVQSGAEVKKPGASVKVSCKASGYTFTNYYMHWVRQAPGLCLE WMGIINPSDGSTSYAQKFQGRVTMTRDTSTNTVYMDLSSLRSEDTA VYYCARGGNSAFYSYYDMDVWGQGTTVTVSS |
| 451 | | **DLL3-14 - L43Q-G44C** | VH | QVQLVQSGAEVKKPGASVKVSCKASGYTFTNYYMHWVRQAPGQCLE WMGIINPSDGSTSYAQKFQGRVTMTRDTSTNTVYMDLSSLRSEDTA VYYCARGGNSAFYSYYDMDVWGQGTTVTVSS |
| 452 | | **DLL3-14 - G44C-D55E** | VH | QVQLVQSGAEVKKPGASVKVSCKASGYTFTNYYMHWVRQAPGLCLE WMGIINPSEGSTSYAQKFQGRVTMTRDTSTNTVYMDLSSLRSEDTA VYYCARGGNSAFYSYYDMDVWGQGTTVTVSS |
| 453 | | **DLL3-14 - G44C-G56A** | VH | QVQLVQSGAEVKKPGASVKVSCKASGYTFTNYYMHWVRQAPGLCLE WMGIINPSDASTSYAQKFQGRVTMTRDTSTNTVYMDLSSLRSEDTA VYYCARGGNSAFYSYYDMDVWGQGTTVTVSS |
| 454 | | **DLL3-14 - L43Q-G44C-D55E** | VH | QVQLVQSGAEVKKPGASVKVSCKASGYTFTNYYMHWVRQAPGQCLE WMGIINPSEGSTSYAQKFQGRVTMTRDTSTNTVYMDLSSLRSEDTA VYYCARGGNSAFYSYYDMDVWGQGTTVTVSS |
| 455 | | **DLL3-14 - L43Q-G44C-G56A** | VH | QVQLVQSGAEVKKPGASVKVSCKASGYTFTNYYMHWVRQAPGQCLE WMGIINPSDASTSYAQKFQGRVTMTRDTSTNTVYMDLSSLRSEDTA VYYCARGGNSAFYSYYDMDVWGQGTTVTVSS |
| | | | | |
| 456 | | **DLL3-14 - D171E** | VL | DVVMTQTPLSLPVTLGQPASISCRSSQSLVYREGNTYLSWFQQRPG QSPRRLIYKVSNWQSGVPDRFSGGGSGTDFTLKISRVEAEDVGVYY CMQGTHWPPTFGQGTKVEIK |
| 457 | | **DLL3-14 - G172A** | VL | DVVMTQTPLSLPVTLGQPASISCRSSQSLVYRDANTYLSWFQQRPG QSPRRLIYKVSNWQSGVPDRFSGGGSGTDFTLKISRVEAEDVGVYY CMQGTHWPPTFGQGTKVEIK |
| 458 | | **DLL3-14 - N173Q** | VL | DVVMTQTPLSLPVTLGQPASISCRSSQSLVYRDGQTYLSWFQQRPG QSPRRLIYKVSNWQSGVPDRFSGGGSGTDFTLKISRVEAEDVGVYY CMQGTHWPPTFGQGTKVEIK |
| 459 | | **DLL3-14 - T174A** | VL | DVVMTQTPLSLPVTLGQPASISCRSSQSLVYRDGNAYLSWFQQRPG QSPRRLIYKVSNWQSGVPDRFSGGGSGTDFTLKISRVEAEDVGVYY CMQGTHWPPTFGQGTKVEIK |
| 460 | | **DLL3-14 - G208S** | VL | DVVMTQTPLSLPVTLGQPASISCRSSQSLVYRDGNTYLSWFQQRPG QSPRRLIYKVSNWQSGVPDRFSGSGSGTDFTLKISRVEAEDVGVYY CMQGTHWPPTFGQGTKVEIK |
| 461 | | **DLL3-14 - D171E-G208S** | VL | DVVMTQTPLSLPVTLGQPASISCRSSQSLVYREGNTYLSWFQQRPG QSPRRLIYKVSNWQSGVPDRFSGSGSGTDFTLKISRVEAEDVGVYY CMQGTHWPPTFGQGTKVEIK |
| 462 | | **DLL3-14 G172A-G208S** | VL | DVVMTQTPLSLPVTLGQPASISCRSSQSLVYRDANTYLSWFQQRPG QSPRRLIYKVSNWQSGVPDRFSGSGSGTDFTLKISRVEAEDVGVYY CMQGTHWPPTFGQGTKVEIK |
| 463 | | **DLL3-14 - Q243C** | VL | DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGCGTKVEIK |
| 464 | | **DLL3-14 - D171E-Q243C** | VL | DVVMTQTPLSLPVTLGQPASISCRSSQSLVYREGNTYLSWFQQRPG QSPRRLIYKVSNWQSGVPDRFSGGGSGTDFTLKISRVEAEDVGVYY CMQGTHWPPTFGCGTKVEIK |
| 465 | | **DLL3-14 - G172A-Q243C** | VL | DVVMTQTPLSLPVTLGQPASISCRSSQSLVYRDANTYLSWFQQRPG QSPRRLIYKVSNWQSGVPDRFSGGGSGTDFTLKISRVEAEDVGVYY CMQGTHWPPTFGCGTKVEIK |
| 466 | | **DLL3-14 - N173Q Q243C** | VL | DVVMTQTPLSLPVTLGQPASISCRSSQSLVYRDGQTYLSWFQQRPG QSPRRLIYKVSNWQSGVPDRFSGGGSGTDFTLKISRVEAEDVGVYY CMQGTHWPPTFGCGTKVEIK |
| 467 | | **DLL3-14 - T174A-Q243C** | VL | DVVMTQTPLSLPVTLGQPASISCRSSQSLVYRDGNAYLSWFQQRPG QSPRRLIYKVSNWQSGVPDRFSGGGSGTDFTLKISRVEAEDVGVYY CMQGTHWPPTFGCGTKVEIK |
| 468 | | **DLL3-14 - G208S-Q243C** | VL | DVVMTQTPLSLPVTLGQPASISCRSSQSLVYRDGNTYLSWFQQRPG QSPRRLIYKVSNWQSGVPDRFSGSGSGTDFTLKISRVEAEDVGVYY CMQGTHWPPTFGCGTKVEIK |
| 469 | | **DLL3-14 - D171E-G208S-Q243C** | VL | DVVMTQTPLSLPVTLGQPASISCRSSQSLVYREGNTYLSWFQQRPG QSPRRLIYKVSNWQSGVPDRFSGSGSGTDFTLKISRVEAEDVGVYY CMQGTHWPPTFGCGTKVEIK |
| 470 | | **DLL3-14 - G172A-G208S-Q243C** | VL | DVVMTQTPLSLPVTLGQPASISCRSSQSLVYRDANTYLSWFQQRPG QSPRRLIYKVSNWQSGVPDRFSGSGSGTDFTLKISRVEAEDVGVYY CMQGTHWPPTFGCGTKVEIK |
| | | | | |
| 471 | | **DLL3-14 - 001** | scFv | QVQLVQSGAEVKKPGASVKVSCKASGYTFTNYYMHWVRQAPGLGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYREGNTYLSWFQQRPG QSPRRLIYKVSNWQSGVPDRFSGGGSGTDFTLKISRVEAEDVGVYY CMQGTHWPPTFGQGTKVEIK |
| 472 | | **DLL3-14 - 002** | scFv | QVQLVQSGAEVKKPGASVKVSCKASGYTFTNYYMHWVRQAPGLGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDANTYLSWFQQRPG QSPRRLIYKVSNWQSGVPDRFSGGGSGTDFTLKISRVEAEDVGVYY CMQGTHWPPTFGQGTKVEIK |
| 473 | | **DLL3-14 - 003** | scFv | QVQLVQSGAEVKKPGASVKVSCKASGYTFTNYYMHWVRQAPGLGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGQTYLSWFQQRPG QSPRRLIYKVSNWQSGVPDRFSGGGSGTDFTLKISRVEAEDVGVYY CMQGTHWPPTFGQGTKVEIK |
| 474 | | **DLL3-14 - 004** | scFv | QVQLVQSGAEVKKPGASVKVSCKASGYTFTNYYMHWVRQAPGLGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNAYLSWFQQRPG QSPRRLIYKVSNWQSGVPDRFSGGGSGTDFTLKISRVEAEDVGVYY CMQGTHWPPTFGQGTKVEIK |
| 475 | | **DLL3-14 - 005** | scFv | QVQLVQSGAEVKKPGASVKVSCKASGYTFTNYYMHWVRQAPGLGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGSGSGTDFTLKISRVEAEDVGVYY CMQGTHWPPTFGQGTKVEIK |
| 476 | | **DLL3-14 - 006** | scFv | QVQLVQSGAEVKKPGASVKVSCKASGYTFTNYYMHWVRQAPGQGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGQGTKVEIK |
| 477 | | **DLL3-14 - 007** | scFv | QVQLVQSGAEVKKPGASVKVSCKASGYTFTNYYMHWVRQAPGLGLE WMGIINPSEGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGQGTKVEIK |
| 478 | | **DLL3-14 - 008** | scFv | QVQLVQSGAEVKKPGASVKVSCKASGYTFTNYYMHWVRQAPGLGLE WMGIINPSDASTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGQGTKVEIK |
| 479 | | **DLL3-14 - 009** | scFv | QVQLVQSGAEVKKPGASVKVSCKASGYTFTNYYMHWVRQAPGQGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG |
| | | | | QSPRRLIYKVSNWQSGVPDRFSGSGSGTDFTLKISRVEAEDVGVYY CMQGTHWPPTFGQGTKVEIK |
| 480 | | **DLL3-14 - 010** | scFv | QVQLVQSGAEVKKPGASVKVSCKASGYTFTNYYMHWVRQAPGQGLE WMGIINPSEGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYREGNTYLSWFQQRPG QSPRRLIYKVSNWQSGVPDRFSGSGSGTDFTLKISRVEAEDVGVYY CMQGTHWPPTFGQGTKVEIK |
| 481 | | **DLL3-14 - 011** | scFv | QVQLVQSGAEVKKPGASVKVSCKASGYTFTNYYMHWVRQAPGQGLE WMGIINPSDASTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDANTYLSWFQQRPG QSPRRLIYKVSNWQSGVPDRFSGSGSGTDFTLKISRVEAEDVGVYY CMQGTHWPPTFGQGTKVEIK |
| 482 | | **DLL3-14-012 (CC)** | scFv | QVQLVQSGAEVKKPGASVKVSCKASGYTFTNYYMHWVRQAPGLCLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGCGTKVEIK |
| 483 | | **DLL3-14 - 013** | scFv | QVQLVQSGAEVKKPGASVKVSCKASGYTFTNYYMHWVRQAPGLCLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYREGNTYLSWFQQRPG QSPRRLIYKVSNWQSGVPDRFSGGGSGTDFTLKISRVEAEDVGVYY CMQGTHWPPTFGCGTKVEIK |
| 484 | | **DLL3-14 - 014** | scFv | QVQLVQSGAEVKKPGASVKVSCKASGYTFTNYYMHWVRQAPGLCLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDANTYLSWFQQRPG QSPRRLIYKVSNWQSGVPDRFSGGGSGTDFTLKISRVEAEDVGVYY CMQGTHWPPTFGCGTKVEIK |
| 485 | | **DLL3-14 - 015** | scFv | QVQLVQSGAEVKKPGASVKVSCKASGYTFTNYYMHWVRQAPGLCLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGQTYLSWFQQRPG QSPRRLIYKVSNWQSGVPDRFSGGGSGTDFTLKISRVEAEDVGVYY CMQGTHWPPTFGCGTKVEIK |
| 486 | | **DLL3-14 - 016** | scFv | QVQLVQSGAEVKKPGASVKVSCKASGYTFTNYYMHWVRQAPGLCLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNAYLSWFQQRPG QSPRRLIYKVSNWQSGVPDRFSGGGSGTDFTLKISRVEAEDVGVYY CMQGTHWPPTFGCGTKVEIK |
| 487 | | **DLL3-14 - 017** | scFv | QVQLVQSGAEVKKPGASVKVSCKASGYTFTNYYMHWVRQAPGLCLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGSGSGTDFTLKISRVEAEDVGVYY CMQGTHWPPTFGCGTKVEIK |
| 488 | | **DLL3-14 - 018** | scFv | QVQLVQSGAEVKKPGASVKVSCKASGYTFTNYYMHWVRQAPGQCLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGCGTKVEIK |
| 489 | | **DLL3-14 - 019** | scFv | QVQLVQSGAEVKKPGASVKVSCKASGYTFTNYYMHWVRQAPGLCLE WMGIINPSEGSTSYAQKFQGRVTMTRDTSTNTVYMDLSSLRSEDTA VYYCARGGNSAFYSYYDMDVWGQGTTVTVSSGGGGSGGGGSGGGGS |
| | | | | DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGCGTKVEIK |
| 490 | | **DLL3-14 - 020** | scFv | QVQLVQSGAEVKKPGASVKVSCKASGYTFTNYYMHWVRQAPGLCLE WMGIINPSDASTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGCGTKVEIK |
| 491 | | **DLL3-14 - 021** | scFv | QVQLVQSGAEVKKPGASVKVSCKASGYTFTNYYMHWVRQAPGQCLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGSGSGTDFTLKISRVEAEDVGVYY CMQGTHWPPTFGCGTKVEIK |
| 492 | | **DLL3-14 - 022** | scFv | QVQLVQSGAEVKKPGASVKVSCKASGYTFTNYYMHWVRQAPGQCLE WMGIINPSEGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYREGNTYLSWFQQRPG QSPRRLIYKVSNWQSGVPDRFSGSGSGTDFTLKISRVEAEDVGVYY CMQGTHWPPTFGCGTKVEIK |
| 493 | | **DLL3-14 - 023** | scFv | QVQLVQSGAEVKKPGASVKVSCKASGYTFTNYYMHWVRQAPGQCLE WMGIINPSDASTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDANTYLSWFQQRPG QSPRRLIYKVSNWQSGVPDRFSGSGSGTDFTLKISRVEAEDVGVYY CMQGTHWPPTFGCGTKVEIK |
| | | | | |
| 494 | | **DLL3-14 - 001 xI2C** | bispecific molecule | QVQLVQSGAEVKKPGASVKVSCKASGYTFTNYYMHWVRQAPGLGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYREGNTYLSWFQQRPG QSPRRLIYKVSNWQSGVPDRFSGGGSGTDFTLKISRVEAEDVGVYY CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 495 | | **DLL3-14 - 002 xI2C** | bispecific molecule | QVQLVQSGAEVKKPGASVKVSCKASGYTFTNYYMHWVRQAPGLGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDANTYLSWFQQRPG QSPRRLIYKVSNWQSGVPDRFSGGGSGTDFTLKISRVEAEDVGVYY CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 496 | | **DLL3-14 - 003 xI2C** | bispecific molecule | QVQLVQSGAEVKKPGASVKVSCKASGYTFTNYYMHWVRQAPGLGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGQTYLSWFQQRPG QSPRRLIYKVSNWQSGVPDRFSGGGSGTDFTLKISRVEAEDVGVYY CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW |
| | | | | AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 497 | | **DLL3-14 - 004 xI2C** | bispecific molecule | QVQLVQSGAEVKKPGASVKVSCKASGYTFTNYYMHWVRQAPGLGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNAYLSWFQQRPG QSPRRLIYKVSNWQSGVPDRFSGGGSGTDFTLKISRVEAEDVGVYY CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 498 | | **DLL3-14 - 005 xI2C** | bispecific molecule | QVQLVQSGAEVKKPGASVKVSCKASGYTFTNYYMHWVRQAPGLGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGSGSGTDFTLKISRVEAEDVGVYY CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 499 | | **DLL3-14 - 006 xI2C** | bispecific molecule | QVQLVQSGAEVKKPGASVKVSCKASGYTFTNYYMHWVRQAPGQGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 500 | | **DLL3-14 - 007 xI2C** | bispecific molecule | QVQLVQSGAEVKKPGASVKVSCKASGYTFTNYYMHWVRQAPGLGLE WMGIINPSEGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 501 | | **DLL3-14 - 008 xI2C** | bispecific molecule | QVQLVQSGAEVKKPGASVKVSCKASGYTFTNYYMHWVRQAPGLGLE WMGIINPSDASTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 502 | | **DLL3-14 - 009 xI2C** | bispecific molecule | QVQLVQSGAEVKKPGASVKVSCKASGYTFTNYYMHWVRQAPGQGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGSGSGTDFTLKISRVEAEDVGVYY CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 503 | | **DLL3-14 - 010 xI2C** | bispecific molecule | QVQLVQSGAEVKKPGASVKVSCKASGYTFTNYYMHWVRQAPGQGLE WMGIINPSEGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYREGNTYLSWFQQRPG QSPRRLIYKVSNWQSGVPDRFSGSGSGTDFTLKISRVEAEDVGVYY CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 504 | | **DLL3-14 - 011 xI2C** | bispecific molecule | QVQLVQSGAEVKKPGASVKVSCKASGYTFTNYYMHWVRQAPGQGLE WMGIINPSDASTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDANTYLSWFQQRPG QSPRRLIYKVSNWQSGVPDRFSGSGSGTDFTLKISRVEAEDVGVYY CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 505 | | **DLL3-14 - 012 (CC) xI2C** | bispecific molecule | QVQLVQSGAEVKKPGASVKVSCKASGYTFTNYYMHWVRQAPGLCLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGCGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 506 | | **DLL3-14 - 013 xI2C** | bispecific molecule | QVQLVQSGAEVKKPGASVKVSCKASGYTFTNYYMHWVRQAPGLCLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYREGNTYLSWFQQRPG QSPRRLIYKVSNWQSGVPDRFSGGGSGTDFTLKISRVEAEDVGVYY CMQGTHWPPTFGCGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 507 | | **DLL3-14 - 014 xI2C** | bispecific molecule | QVQLVQSGAEVKKPGASVKVSCKASGYTFTNYYMHWVRQAPGLCLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDANTYLSWFQQRPG |
| | | | | QSPRRLIYKVSNWQSGVPDRFSGGGSGTDFTLKISRVEAEDVGVYY CMQGTHWPPTFGCGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 508 | | **DLL3-14 - 015 xI2C** | bispecific molecule | QVQLVQSGAEVKKPGASVKVSCKASGYTFTNYYMHWVRQAPGLCLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGQTYLSWFQQRPG QSPRRLIYKVSNWQSGVPDRFSGGGSGTDFTLKISRVEAEDVGVYY CMQGTHWPPTFGCGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 509 | | **DLL3-14 - 016 xI2C** | bispecific molecule | QVQLVQSGAEVKKPGASVKVSCKASGYTFTNYYMHWVRQAPGLCLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNAYLSWFQQRPG QSPRRLIYKVSNWQSGVPDRFSGGGSGTDFTLKISRVEAEDVGVYY CMQGTHWPPTFGCGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 510 | | **DLL3-14 - 017 xI2C** | bispecific molecule | QVQLVQSGAEVKKPGASVKVSCKASGYTFTNYYMHWVRQAPGLCLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGSGSGTDFTLKISRVEAEDVGVYY CMQGTHWPPTFGCGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 511 | | **DLL3-14 - 018 xI2C** | bispecific molecule | QVQLVQSGAEVKKPGASVKVSCKASGYTFTNYYMHWVRQAPGQCLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGCGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 512 | | **DLL3-14 - 019 xI2C** | bispecific molecule | QVQLVQSGAEVKKPGASVKVSCKASGYTFTNYYMHWVRQAPGLCLE WMGIINPSEGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGCGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW |
| | | | | AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 513 | | **DLL3-14 - 020 xI2C** | bispecific molecule | QVQLVQSGAEVKKPGASVKVSCKASGYTFTNYYMHWVRQAPGLCLE WMGIINPSDASTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGCGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 514 | | **DLL3-14 - 021 xI2C** | bispecific molecule | QVQLVQSGAEVKKPGASVKVSCKASGYTFTNYYMHWVRQAPGQCLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGSGSGTDFTLKISRVEAEDVGVYY CMQGTHWPPTFGCGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 515 | | **DLL3-14 - 022 xI2C** | bispecific molecule | QVQLVQSGAEVKKPGASVKVSCKASGYTFTNYYMHWVRQAPGQCLE WMGIINPSEGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYREGNTYLSWFQQRPG QSPRRLIYKVSNWQSGVPDRFSGSGSGTDFTLKISRVEAEDVGVYY CMQGTHWPPTFGCGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 516 | | **DLL3-14 - 023 xI2C** | bispecific molecule | QVQLVQSGAEVKKPGASVKVSCKASGYTFTNYYMHWVRQAPGQCLE WMGIINPSDASTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDANTYLSWFQQRPG QSPRRLIYKVSNWQSGVPDRFSGSGSGTDFTLKISRVEAEDVGVYY CMQGTHWPPTFGCGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 517 | | **DLL3-4 xI2C - scFc** | bispecific HLE molecule | QVQLQESGPGLVKPSETLSLTCTVSGGSISSYYWSWIRQPPGKGLE WIGYVYYSGTTNYNPSLKSRVTISVDTSKNQFSLKLSSVTAADTAV YYCASIAVTGFYFDYWGQGTLVTVSSGGGGSGGGGSGGGGSEIVLT QSPGTLSLSPGERVTLSCRASQRVNNNYLAWYQQRPGQAPRLLIYG ASSRATGIPDRFSGSGSGTDFTLTISRLEPEDFAVYYCQQYDRSPL TFGGGTKLEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA |
| | | | | ALTLSGVQPEDEAEYYCVLWYSNRWVFGGGTKLTVLGGGGDKTHTC PPCPAPELLGGPSVFLFPPKPKDTLMISRTPEVTCVVVDVSHEDPE VKFNWYVDGVEVHNAKTKPCEEQYGSTYRCVSVLTVLHQDWLNGKE YKCKVSNKALPAPIEKTISKAKGQPREPQVYTLPPSREEMTKNQVS LTCLVKGFYPSDIAVEWESNGQPENNYKTTPPVLDSDGSFFLYSKL TVDKSRWQQGNVFSCSVMHEALHNHYTQKSLSLSPGKGGGGSGGGG SGGGGSGGGGSGGGGSGGGGSDKTHTCPPCPAPELLGGPSVFLFPP KPKDTLMISRTPEVTCVVVDVSHEDPEVKFNWYVDGVEVHNAKTKP CEEQYGSTYRCVSVLTVLHQDWLNGKEYKCKVSNKALPAPIEKTIS KAKGQPREPQVYTLPPSREEMTKNQVSLTCLVKGFYPSDIAVEWES NGQPENNYKTTPPVLDSDGSFFLYSKLTVDKSRWQQGNVFSCSVMH EALHNHYTQKSLSLSPGK |
| 518 | | **DLL3-4 xI2C - scFc_d elGK** | bispecific HLE molecule | QVQLQESGPGLVKPSETLSLTCTVSGGSISSYYWSWIRQPPGKGLE WIGYVYYSGTTNYNPSLKSRVTISVDTSKNQFSLKLSSVTAADTAV YYCASIAVTGFYFDYWGQGTLVTVSSGGGGSGGGGSGGGGSEIVLT QSPGTLSLSPGERVTLSCRASQRVNNNYLAWYQQRPGQAPRLLIYG ASSRATGIPDRFSGSGSGTDFTLTISRLEPEDFAVYYCQQYDRSPL TFGGGTKLEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLGGGGDKTHTC PPCPAPELLGGPSVFLFPPKPKDTLMISRTPEVTCVVVDVSHEDPE VKFNWYVDGVEVHNAKTKPCEEQYGSTYRCVSVLTVLHQDWLNGKE YKCKVSNKALPAPIEKTISKAKGQPREPQVYTLPPSREEMTKNQVS LTCLVKGFYPSDIAVEWESNGQPENNYKTTPPVLDSDGSFFLYSKL TVDKSRWQQGNVFSCSVMHEALHNHYTQKSLSLSPGGGGSGGGGSG GGGSGGGGSGGGGSGGGGSDKTHTCPPCPAPELLGGPSVFLFPPKP KDTLMISRTPEVTCVVVDVSHEDPEVKFNWYVDGVEVHNAKTKPCE EQYGSTYRCVSVLTVLHQDWLNGKEYKCKVSNKALPAPIEKTISKA KGQPREPQVYTLPPSREEMTKNQVSLTCLVKGFYPSDIAVEWESNG QPENNYKTTPPVLDSDGSFFLYSKLTVDKSRWQQGNVFSCSVMHEA LHNHYTQKSLSLSPGK |
| 519 | | **DLL3-4-001 (CC) xI2C - scFc** | bispecific HLE molecule | QVQLQESGPGLVKPSETLSLTCTVSGGSISSYYWSWIRQPPGKCLE WIGYVYYSGTTNYNPSLKSRVTISVDTSKNQFSLKLSSVTAADTAV YYCASIAVTGFYFDYWGQGTLVTVSSGGGGSGGGGSGGGGSEIVLT QSPGTLSLSPGERVTLSCRASQRVNNNYLAWYQQRPGQAPRLLIYG ASSRATGIPDRFSGSGSGTDFTLTISRLEPEDFAVYYCQQYDRSPL TFGCGTKLEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLGGGGDKTHTC PPCPAPELLGGPSVFLFPPKPKDTLMISRTPEVTCVVVDVSHEDPE VKFNWYVDGVEVHNAKTKPCEEQYGSTYRCVSVLTVLHQDWLNGKE YKCKVSNKALPAPIEKTISKAKGQPREPQVYTLPPSREEMTKNQVS LTCLVKGFYPSDIAVEWESNGQPENNYKTTPPVLDSDGSFFLYSKL TVDKSRWQQGNVFSCSVMHEALHNHYTQKSLSLSPGKGGGGSGGGG SGGGGSGGGGSGGGGSGGGGSDKTHTCPPCPAPELLGGPSVFLFPP KPKDTLMISRTPEVTCVVVDVSHEDPEVKFNWYVDGVEVHNAKTKP CEEQYGSTYRCVSVLTVLHQDWLNGKEYKCKVSNKALPAPIEKTIS KAKGQPREPQVYTLPPSREEMTKNQVSLTCLVKGFYPSDIAVEWES NGQPENNYKTTPPVLDSDGSFFLYSKLTVDKSRWQQGNVFSCSVMH EALHNHYTQKSLSLSPGK |
| 520 | | **DLL3-4-001 (CC) xI2C - scFc _delGK** | bispecific HLE molecule | QVQLQESGPGLVKPSETLSLTCTVSGGSISSYYWSWIRQPPGKCLE WIGYVYYSGTTNYNPSLKSRVTISVDTSKNQFSLKLSSVTAADTAV YYCASIAVTGFYFDYWGQGTLVTVSSGGGGSGGGGSGGGGSEIVLT |
| | | | | QSPGTLSLSPGERVTLSCRASQRVNNNYLAWYQQRPGQAPRLLIYG ASSRATGIPDRFSGSGSGTDFTLTISRLEPEDFAVYYCQQYDRSPL TFGCGTKLEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLGGGGDKTHTC PPCPAPELLGGPSVFLFPPKPKDTLMISRTPEVTCVVVDVSHEDPE VKFNWYVDGVEVHNAKTKPCEEQYGSTYRCVSVLTVLHQDWLNGKE YKCKVSNKALPAPIEKTISKAKGQPREPQVYTLPPSREEMTKNQVS LTCLVKGFYPSDIAVEWESNGQPENNYKTTPPVLDSDGSFFLYSKL TVDKSRWQQGNVFSCSVMHEALHNHYTQKSLSLSPGGGGSGGGGSG GGGSGGGGSGGGGSGGGGSDKTHTCPPCPAPELLGGPSVFLFPPKP KDTLMISRTPEVTCVVVDVSHEDPEVKFNWYVDGVEVHNAKTKPCE EQYGSTYRCVSVLTVLHQDWLNGKEYKCKVSNKALPAPIEKTISKA KGQPREPQVYTLPPSREEMTKNQVSLTCLVKGFYPSDIAVEWESNG QPENNYKTTPPVLDSDGSFFLYSKLTVDKSRWQQGNVFSCSVMHEA LHNHYTQKSLSLSPGK |
| 521 | | **DLL3-6 xI2C - scFc** | bispecific HLE molecule | QVQLQESGPGLVKPSETLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKVEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLGGGGDKTHTC PPCPAPELLGGPSVFLFPPKPKDTLMISRTPEVTCVVVDVSHEDPE VKFNWYVDGVEVHNAKTKPCEEQYGSTYRCVSVLTVLHQDWLNGKE YKCKVSNKALPAPIEKTISKAKGQPREPQVYTLPPSREEMTKNQVS LTCLVKGFYPSDIAVEWESNGQPENNYKTTPPVLDSDGSFFLYSKL TVDKSRWQQGNVFSCSVMHEALHNHYTQKSLSLSPGKGGGGSGGGG SGGGGSGGGGSGGGGSGGGGSDKTHTCPPCPAPELLGGPSVFLFPP KPKDTLMISRTPEVTCVVVDVSHEDPEVKFNWYVDGVEVHNAKTKP CEEQYGSTYRCVSVLTVLHQDWLNGKEYKCKVSNKALPAPIEKTIS KAKGQPREPQVYTLPPSREEMTKNQVSLTCLVKGFYPSDIAVEWES NGQPENNYKTTPPVLDSDGSFFLYSKLTVDKSRWQQGNVFSCSVMH EALHNHYTQKSLSLSPGK |
| 522 | | **DLL3-6 xI2C - scFc_d elGK** | bispecific HLE molecule | QVQLQESGPGLVKPSETLSLTCTVSGASISSFYWSWIRQPPGKGLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGGGTKVEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLGGGGDKTHTC PPCPAPELLGGPSVFLFPPKPKDTLMISRTPEVTCVVVDVSHEDPE VKFNWYVDGVEVHNAKTKPCEEQYGSTYRCVSVLTVLHQDWLNGKE YKCKVSNKALPAPIEKTISKAKGQPREPQVYTLPPSREEMTKNQVS LTCLVKGFYPSDIAVEWESNGQPENNYKTTPPVLDSDGSFFLYSKL TVDKSRWQQGNVFSCSVMHEALHNHYTQKSLSLSPGGGGSGGGGSG GGGSGGGGSGGGGSGGGGSDKTHTCPPCPAPELLGGPSVFLFPPKP KDTLMISRTPEVTCVVVDVSHEDPEVKFNWYVDGVEVHNAKTKPCE |
| | | | | EQYGSTYRCVSVLTVLHQDWLNGKEYKCKVSNKALPAPIEKTISKA KGQPREPQVYTLPPSREEMTKNQVSLTCLVKGFYPSDIAVEWESNG QPENNYKTTPPVLDSDGSFFLYSKLTVDKSRWQQGNVFSCSVMHEA LHNHYTQKSLSLSPGK |
| 523 | | **DLL3-6-001 (CC) xI2C** - **scFc** | bispecific HLE molecule | QVQLQESGPGLVKPSETLSLTCTVSGASISSFYWSWIRQPPGKCLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGCGTKVEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLGGGGDKTHTC PPCPAPELLGGPSVFLFPPKPKDTLMISRTPEVTCVVVDVSHEDPE VKFNWYVDGVEVHNAKTKPCEEQYGSTYRCVSVLTVLHQDWLNGKE YKCKVSNKALPAPIEKTISKAKGQPREPQVYTLPPSREEMTKNQVS LTCLVKGFYPSDIAVEWESNGQPENNYKTTPPVLDSDGSFFLYSKL TVDKSRWQQGNVFSCSVMHEALHNHYTQKSLSLSPGKGGGGSGGGG SGGGGSGGGGSGGGGSGGGGSDKTHTCPPCPAPELLGGPSVFLFPP KPKDTLMISRTPEVTCVVVDVSHEDPEVKFNWYVDGVEVHNAKTKP CEEQYGSTYRCVSVLTVLHQDWLNGKEYKCKVSNKALPAPIEKTIS KAKGQPREPQVYTLPPSREEMTKNQVSLTCLVKGFYPSDIAVEWES NGQPENNYKTTPPVLDSDGSFFLYSKLTVDKSRWQQGNVFSCSVMH EALHNHYTQKSLSLSPGK |
| 524 | | **DLL3-6-001 (CC) xI2C - scFc_d elGK** | bispecific HLE molecule | QVQLQESGPGLVKPSETLSLTCTVSGASISSFYWSWIRQPPGKCLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGCGTKVEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVLGGGGDKTHTC PPCPAPELLGGPSVFLFPPKPKDTLMISRTPEVTCVVVDVSHEDPE VKFNWYVDGVEVHNAKTKPCEEQYGSTYRCVSVLTVLHQDWLNGKE YKCKVSNKALPAPIEKTISKAKGQPREPQVYTLPPSREEMTKNQVS LTCLVKGFYPSDIAVEWESNGQPENNYKTTPPVLDSDGSFFLYSKL TVDKSRWQQGNVFSCSVMHEALHNHYTQKSLSLSPGGGGSGGGGSG GGGSGGGGSGGGGSGGGGSDKTHTCPPCPAPELLGGPSVFLFPPKP KDTLMISRTPEVTCVVVDVSHEDPEVKFNWYVDGVEVHNAKTKPCE EQYGSTYRCVSVLTVLHQDWLNGKEYKCKVSNKALPAPIEKTISKA KGQPREPQVYTLPPSREEMTKNQVSLTCLVKGFYPSDIAVEWESNG QPENNYKTTPPVLDSDGSFFLYSKLTVDKSRWQQGNVFSCSVMHEA LHNHYTQKSLSLSPGK |
| 525 | | **DLL3-14 xI2C - scFc** | bispecific HLE molecule | QVQLVQSGAEVKKPGASVKVSCKASGYTFTNYYMHWVRQAPGLGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVLG |
| | | | | GGGDKTHTCPPCPAPELLGGPSVFLFPPKPKDTLMISRTPEVTCVV VDVSHEDPEVKFNWYVDGVEVHNAKTKPCEEQYGSTYRCVSVLTVL HQDWLNGKEYKCKVSNKALPAPIEKTISKAKGQPREPQVYTLPPSR EEMTKNQVSLTCLVKGFYPSDIAVEWESNGQPENNYKTTPPVLDSD GSFFLYSKLTVDKSRWQQGNVFSCSVMHEALHNHYTQKSLSLSPGK GGGGSGGGGSGGGGSGGGGSGGGGSGGGGSDKTHTCPPCPAPELLG GPSVFLFPPKPKDTLMISRTPEVTCVVVDVSHEDPEVKFNWYVDGV EVHNAKTKPCEEQYGSTYRCVSVLTVLHQDWLNGKEYKCKVSNKAL PAPIEKTISKAKGQPREPQVYTLPPSREEMTKNQVSLTCLVKGFYP SDIAVEWESNGQPENNYKTTPPVLDSDGSFFLYSKLTVDKSRWQQG NVFSCSVMHEALHNHYTQKSLSLSPGK |
| 526 | | **DLL3-14 xI2C - scFc_d elGK** | bispecific HLE molecule | QVQLVQSGAEVKKPGASVKVSCKASGYTFTNYYMHWVRQAPGLGLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGQGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVLG GGGDKTHTCPPCPAPELLGGPSVFLFPPKPKDTLMISRTPEVTCVV VDVSHEDPEVKFNWYVDGVEVHNAKTKPCEEQYGSTYRCVSVLTVL HQDWLNGKEYKCKVSNKALPAPIEKTISKAKGQPREPQVYTLPPSR EEMTKNQVSLTCLVKGFYPSDIAVEWESNGQPENNYKTTPPVLDSD GSFFLYSKLTVDKSRWQQGNVFSCSVMHEALHNHYTQKSLSLSPGG GGSGGGGSGGGGSGGGGSGGGGSGGGGSDKTHTCPPCPAPELLGGP SVFLFPPKPKDTLMISRTPEVTCVVVDVSHEDPEVKFNWYVDGVEV HNAKTKPCEEQYGSTYRCVSVLTVLHQDWLNGKEYKCKVSNKALPA PIEKTISKAKGQPREPQVYTLPPSREEMTKNQVSLTCLVKGFYPSD IAVEWESNGQPENNYKTTPPVLDSDGSFFLYSKLTVDKSRWQQGNV FSCSVMHEALHNHYTQKSLSLSPGK |
| 527 | | **DLL3-14** - **012 (CC) xI2C - scFc** | bispecific HLE molecule | QVQLVQSGAEVKKPGASVKVSCKASGYTFTNYYMHWVRQAPGLCLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG QSPRRLIYKVSNWQSGVPDRFSGGGSGTDFTLKISRVEAEDVGVYY CMQGTHWPPTFGCGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVLG GGGDKTHTCPPCPAPELLGGPSVFLFPPKPKDTLMISRTPEVTCVV VDVSHEDPEVKFNWYVDGVEVHNAKTKPCEEQYGSTYRCVSVLTVL HQDWLNGKEYKCKVSNKALPAPIEKTISKAKGQPREPQVYTLPPSR EEMTKNQVSLTCLVKGFYPSDIAVEWESNGQPENNYKTTPPVLDSD GSFFLYSKLTVDKSRWQQGNVFSCSVMHEALHNHYTQKSLSLSPGK GGGGSGGGGSGGGGSGGGGSGGGGSGGGGSDKTHTCPPCPAPELLG GPSVFLFPPKPKDTLMISRTPEVTCVVVDVSHEDPEVKFNWYVDGV EVHNAKTKPCEEQYGSTYRCVSVLTVLHQDWLNGKEYKCKVSNKAL PAPIEKTISKAKGQPREPQVYTLPPSREEMTKNQVSLTCLVKGFYP SDIAVEWESNGQPENNYKTTPPVLDSDGSFFLYSKLTVDKSRWQQG NVFSCSVMHEALHNHYTQKSLSLSPGK |
| 528 | | **DLL3-14 - 012 (CC) xI2C-scFc_d elGK** | bispecific HLE molecule | QVQLVQSGAEVKKPGASVKVSCKASGYTFTNYYMHWVRQAPGLCLE WMGIINPSDGSTSYAQKFQGRVTMTRDTSTNTVYMDLSSLRSEDTA VYYCARGGNSAFYSYYDMDVWGQGTTVTVSSGGGGSGGGGSGGGGS DVVMTQTPLSLPVTLGQPASISCRSSQSLVYRDGNTYLSWFQQRPG |
| | | | | QSPRRLIYKVSNWQSGVPDRFSGGGSGTDFTLKISRVEAEDVGVYY CMQGTHWPPTFGCGTKVEIKSGGGGSEVQLVESGGGLVQPGGSLKL SCAASGFTFNKYAMNWVRQAPGKGLEWVARIRSKYNNYATYYADSV KDRFTISRDDSKNTAYLQMNNLKTEDTAVYYCVRHGNFGNSYISYW AYWGQGTLVTVSSGGGGSGGGGSGGGGSQTVVTQEPSLTVSPGGTV TLTCGSSTGAVTSGNYPNWVQQKPGQAPRGLIGGTKFLAPGTPARF SGSLLGGKAALTLSGVQPEDEAEYYCVLWYSNRWVFGGGTKLTVLG GGGDKTHTCPPCPAPELLGGPSVFLFPPKPKDTLMISRTPEVTCVV VDVSHEDPEVKFNWYVDGVEVHNAKTKPCEEQYGSTYRCVSVLTVL HQDWLNGKEYKCKVSNKALPAPIEKTISKAKGQPREPQVYTLPPSR EEMTKNQVSLTCLVKGFYPSDIAVEWESNGQPENNYKTTPPVLDSD GSFFLYSKLTVDKSRWQQGNVFSCSVMHEALHNHYTQKSLSLSPGG GGSGGGGSGGGGSGGGSGGGGSGGGGSDKTHTCPPCPAPELLGGP SVFLFPPKPKDTLMISRTPEVTCVVVDVSHEDPEVKFNWYVDGVEV HNAKTKPCEEQYGSTYRCVSVLTVLHQDWLNGKEYKCKVSNKALPA PIEKTISKAKGQPREPQVYTLPPSREEMTKNQVSLTCLVKGFYPSD IAVEWESNGQPENNYKTTPPVLDSDGSFFLYSKLTVDKSRWQQGNV FSCSVMHEALHNHYTQKSLSLSPGK |
| 529 | | **DLL3-6-001 (CC)** | VH | QVQLQESGPGLVKPSETLSLTCTVSGASISSFYWSWIRQPPGKCLE WIGYIYYSGTTNYNPSLKSRVTISVDTSKNQFSLKLSSVTAADTAV YYCARIAVAGFFFDYWGQGTLVTVSS |
| 530 | | **DLL3-6-001 (CC)** | VL | EIVLTQSPGTLSLSPGERATLSCRASQSVNKNYLAWYQQKPGQAPR LLIYGASSRATGIPDRFSGSGSGTDFTLTISRLEPEDFAVYYCQQY DRSPLTFGCGTKVEIK |
| 531 | | **DLL3-6-001 (CC)** | scFv | QVQLQESGPGLVKPSETLSLTCTVSGASISSFYWSWIRQPPGKCLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGCGTKVEIK |
| 532 | | **DLL3-6-001 (CC) xI2C** | bispecific molecule | QVQLQESGPGLVKPSETLSLTCTVSGASISSFYWSWIRQPPGKCLE WIGYIYYSGTTNYNPSLKSRVTISVDTSKNQFSLKLSSVTAADTAV YYCARIAVAGFFFDYWGQGTLVTVSSGGGGSGGGGSGGGGSEIVLT QSPGTLSLSPGERATLSCRASQSVNKNYLAWYQQKPGQAPRLLIYG ASSRATGIPDRFSGSGSGTDFTLTISRLEPEDFAVYYCQQYDRSPL TFGCGTKVEIKSGGGGSEVQLVESGGGLVQPGGSLKLSCAASGFTF NKYAMNWVRQAPGKGLEWVARIRSKYNNYATYYADSVKDRFTISRD DSKNTAYLQMNNLKTEDTAVYYCVRHGNFGNSYISYWAYWGQGTLV TVSSGGGGSGGGGSGGGGSQTVVTQEPSLTVSPGGTVTLTCGSSTG AVTSGNYPNWVQQKPGQAPRGLIGGTKFLAPGTPARFSGSLLGGKA ALTLSGVQPEDEAEYYCVLWYSNRWVFGGGTKLTVL |
| | | | | |
| 533 | | Fc monomer-1 +c/-g | | DKTHTCPPCPAPELLGGPSVFLFPPKPKDTLMISRTPEVTCVVVDV SHEDPEVKFNWYVDGVEVHNAKTKPCEEQYGSTYRCVSVLTVLHQD WLNGKEYKCKVSNKALPAPIEKTISKAKGQPREPQVYTLPPSREEM TKNQVSLTCLVKGFYPSDIAVEWESNGQPENNYKTTPPVLDSDGSF FLYSKLTVDKSRWQQGNVFSCSVMHEALHNHYTQKSLSLSPGK |
| 534 | | Fc monomer-2 +c/-g/delGK | | DKTHTCPPCPAPELLGGPSVFLFPPKPKDTLMISRTPEVTCVVVDV SHEDPEVKFNWYVDGVEVHNAKTKPCEEQYGSTYRCVSVLTVLHQD WLNGKEYKCKVSNKALPAPIEKTISKAKGQPREPQVYTLPPSREEM TKNQVSLTCLVKGFYPSDIAVEWESNGQPENNYKTTPPVLDSDGSF FLYSKLTVDKSRWQQGNVFSCSVMHEALHNHYTQKSLSLSP |
| 535 | | Fc monomer-3 -c/+g | | DKTHTCPPCPAPELLGGPSVFLFPPKPKDTLMISRTPEVTCVVVDV SHEDPEVKFNWYVDGVEVHNAKTKPREEQYNSTYRVVSVLTVLHQD WLNGKEYKCKVSNKALPAPIEKTISKAKGQPREPQVYTLPPSREEM TKNQVSLTCLVKGFYPSDIAVEWESNGQPENNYKTTPPVLDSDGSF FLYSKLTVDKSRWQQGNVFSCSVMHEALHNHYTQKSLSLSPGK |
| 536 | | Fc monomer-4-c/+g/delGK | | DKTHTCPPCPAPELLGGPSVFLFPPKPKDTLMISRTPEVTCVVVDV SHEDPEVKFNWYVDGVEVHNAKTKPREEQYNSTYRVVSVLTVLHQD WLNGKEYKCKVSNKALPAPIEKTISKAKGQPREPQVYTLPPSREEM TKNQVSLTCLVKGFYPSDIAVEWESNGQPENNYKTTPPVLDSDGSF FLYSKLTVDKSRWQQGNVFSCSVMHEALHNHYTQKSLSLSP |
| 537 | | Fc monomer-5-c/-g | | DKTHTCPPCPAPELLGGPSVFLFPPKPKDTLMISRTPEVTCVVVDV SHEDPEVKFNWYVDGVEVHNAKTKPREEQYGSTYRVVSVLTVLHQD WLNGKEYKCKVSNKALPAPIEKTISKAKGQPREPQVYTLPPSREEM TKNQVSLTCLVKGFYPSDIAVEWESNGQPENNYKTTPPVLDSDGSF FLYSKLTVDKSRWQQGNVFSCSVMHEALHNHYTQKSLSLSPGK |
| 538 | | Fc monomer-6-c/-g/delGK | | DKTHTCPPCPAPELLGGPSVFLFPPKPKDTLMISRTPEVTCVVVDV SHEDPEVKFNWYVDGVEVHNAKTKPREEQYGSTYRVVSVLTVLHQD WLNGKEYKCKVSNKALPAPIEKTISKAKGQPREPQVYTLPPSREEM TKNQVSLTCLVKGFYPSDIAVEWESNGQPENNYKTTPPVLDSDGSF FLYSKLTVDKSRWQQGNVFSCSVMHEALHNHYTQKSLSLSP |
| 539 | | Fc monomer-7+c/+g | | DKTHTCPPCPAPELLGGPSVFLFPPKPKDTLMISRTPEVTCVVVDV SHEDPEVKFNWYVDGVEVHNAKTKPCEEQYNSTYRCVSVLTVLHQD WLNGKEYKCKVSNKALPAPIEKTISKAKGQPREPQVYTLPPSREEM TKNQVSLTCLVKGFYPSDIAVEWESNGQPENNYKTTPPVLDSDGSF FLYSKLTVDKSRWQQGNVFSCSVMHEALHNHYTQKSLSLSPGK |
| 540 | | Fc monomer-8+c/+g/delGK | | DKTHTCPPCPAPELLGGPSVFLFPPKPKDTLMISRTPEVTCVVVDV SHEDPEVKFNWYVDGVEVHNAKTKPCEEQYNSTYRCVSVLTVLHQD WLNGKEYKCKVSNKALPAPIEKTISKAKGQPREPQVYTLPPSREEM TKNQVSLTCLVKGFYPSDIAVEWESNGQPENNYKTTPPVLDSDGSF FLYSKLTVDKSRWQQGNVFSCSVMHEALHNHYTQKSLSLSP |
| 541 | | scFc-1 | | DKTHTCPPCPAPELLGGPSVFLFPPKPKDTLMISRTPEVTCVVVDV SHEDPEVKFNWYVDGVEVHNAKTKPCEEQYGSTYRCVSVLTVLHQD WLNGKEYKCKVSNKALPAPIEKTISKAKGQPREPQVYTLPPSREEM TKNQVSLTCLVKGFYPSDIAVEWESNGQPENNYKTTPPVLDSDGSF FLYSKLTVDKSRWQQGNVFSCSVMHEALHNHYTQKSLSLSPGKGGG GSGGGGSGGGGSGGGGSGGGGSGGGGSDKTHTCPPCPAPELLGGPS VFLFPPKPKDTLMISRTPEVTCVVVDVSHEDPEVKFNWYVDGVEVH NAKTKPCEEQYGSTYRCVSVLTVLHQDWLNGKEYKCKVSNKALPAP IEKTISKAKGQPREPQVYTLPPSREEMTKNQVSLTCLVKGFYPSDI AVEWESNGQPENNYKTTPPVLDSDGSFFLYSKLTVDKSRWQQGNVF SCSVMHEALHNHYTQKSLSLSPGK |
| 542 | | scFc-2 | | DKTHTCPPCPAPELLGGPSVFLFPPKPKDTLMISRTPEVTCVVVDV SHEDPEVKFNWYVDGVEVHNAKTKPCEEQYGSTYRCVSVLTVLHQD WLNGKEYKCKVSNKALPAPIEKTISKAKGQPREPQVYTLPPSREEM TKNQVSLTCLVKGFYPSDIAVEWESNGQPENNYKTTPPVLDSDGSF FLYSKLTVDKSRWQQGNVFSCSVMHEALHNHYTQKSLSLSPGGGGS GGGGSGGGGSGGGGSGGGGSGGGGSDKTHTCPPCPAPELLGGPSVF LFPPKPKDTLMISRTPEVTCVVVDVSHEDPEVKFNWYVDGVEVHNA KTKPCEEQYGSTYRCVSVLTVLHQDWLNGKEYKCKVSNKALPAPIE KTISKAKGQPREPQVYTLPPSREEMTKNQVSLTCLVKGFYPSDIAV EWESNGQPENNYKTTPPVLDSDGSFFLYSKLTVDKSRWQQGNVFSC SVMHEALHNHYTQKSLSLSP |
| 543 | | scFc-3 | | DKTHTCPPCPAPELLGGPSVFLFPPKPKDTLMISRTPEVTCVVVDV SHEDPEVKFNWYVDGVEVHNAKTKPREEQYNSTYRVVSVLTVLHQD WLNGKEYKCKVSNKALPAPIEKTISKAKGQPREPQVYTLPPSREEM TKNQVSLTCLVKGFYPSDIAVEWESNGQPENNYKTTPPVLDSDGSF FLYSKLTVDKSRWQQGNVFSCSVMHEALHNHYTQKSLSLSPGKGGG GSGGGGSGGGGSGGGGSGGGGSGGGGSDKTHTCPPCPAPELLGGPS VFLFPPKPKDTLMISRTPEVTCVVVDVSHEDPEVKFNWYVDGVEVH NAKTKPREEQYNSTYRVVSVLTVLHQDWLNGKEYKCKVSNKALPAP IEKTISKAKGQPREPQVYTLPPSREEMTKNQVSLTCLVKGFYPSDI AVEWESNGQPENNYKTTPPVLDSDGSFFLYSKLTVDKSRWQQGNVF SCSVMHEALHNHYTQKSLSLSPGK |
| 544 | | scFc-4 | | DKTHTCPPCPAPELLGGPSVFLFPPKPKDTLMISRTPEVTCVVVDV SHEDPEVKFNWYVDGVEVHNAKTKPREEQYNSTYRVVSVLTVLHQD WLNGKEYKCKVSNKALPAPIEKTISKAKGQPREPQVYTLPPSREEM TKNQVSLTCLVKGFYPSDIAVEWESNGQPENNYKTTPPVLDSDGSF FLYSKLTVDKSRWQQGNVFSCSVMHEALHNHYTQKSLSLSPGGGGS GGGGSGGGGSGGGGSGGGGSGGGGSDKTHTCPPCPAPELLGGPSVF LFPPKPKDTLMISRTPEVTCVVVDVSHEDPEVKFNWYVDGVEVHNA KTKPREEQYNSTYRVVSVLTVLHQDWLNGKEYKCKVSNKALPAPIE KTISKAKGQPREPQVYTLPPSREEMTKNQVSLTCLVKGFYPSDIAV EWESNGQPENNYKTTPPVLDSDGSFFLYSKLTVDKSRWQQGNVFSC SVMHEALHNHYTQKSLSLSP |
| 545 | | scFc-5 | | DKTHTCPPCPAPELLGGPSVFLFPPKPKDTLMISRTPEVTCVVVDV SHEDPEVKFNWYVDGVEVHNAKTKPREEQYGSTYRVVSVLTVLHQD WLNGKEYKCKVSNKALPAPIEKTISKAKGQPREPQVYTLPPSREEM TKNQVSLTCLVKGFYPSDIAVEWESNGQPENNYKTTPPVLDSDGSF FLYSKLTVDKSRWQQGNVFSCSVMHEALHNHYTQKSLSLSPGKGGG GSGGGGSGGGGSGGGGSGGGGSGGGGSDKTHTCPPCPAPELLGGPS VFLFPPKPKDTLMISRTPEVTCVVVDVSHEDPEVKFNWYVDGVEVH NAKTKPREEQYGSTYRVVSVLTVLHQDWLNGKEYKCKVSNKALPAP IEKTISKAKGQPREPQVYTLPPSREEMTKNQVSLTCLVKGFYPSDI AVEWESNGQPENNYKTTPPVLDSDGSFFLYSKLTVDKSRWQQGNVF SCSVMHEALHNHYTQKSLSLSPGK |
| 546 | | scFc-6 | | DKTHTCPPCPAPELLGGPSVFLFPPKPKDTLMISRTPEVTCVVVDV SHEDPEVKFNWYVDGVEVHNAKTKPREEQYGSTYRVVSVLTVLHQD WLNGKEYKCKVSNKALPAPIEKTISKAKGQPREPQVYTLPPSREEM TKNQVSLTCLVKGFYPSDIAVEWESNGQPENNYKTTPPVLDSDGSF FLYSKLTVDKSRWQQGNVFSCSVMHEALHNHYTQKSLSLSPGGGGS GGGGSGGGGSGGGGSGGGGSGGGGSDKTHTCPPCPAPELLGGPSVF LFPPKPKDTLMISRTPEVTCVVVDVSHEDPEVKFNWYVDGVEVHNA KTKPREEQYGSTYRVVSVLTVLHQDWLNGKEYKCKVSNKALPAPIE KTISKAKGQPREPQVYTLPPSREEMTKNQVSLTCLVKGFYPSDIAV EWESNGQPENNYKTTPPVLDSDGSFFLYSKLTVDKSRWQQGNVFSC SVMHEALHNHYTQKSLSLSP |
| 547 | | scFc-7 | | DKTHTCPPCPAPELLGGPSVFLFPPKPKDTLMISRTPEVTCVVVDV SHEDPEVKFNWYVDGVEVHNAKTKPCEEQYNSTYRCVSVLTVLHQD WLNGKEYKCKVSNKALPAPIEKTISKAKGQPREPQVYTLPPSREEM TKNQVSLTCLVKGFYPSDIAVEWESNGQPENNYKTTPPVLDSDGSF FLYSKLTVDKSRWQQGNVFSCSVMHEALHNHYTQKSLSLSPGKGGG GSGGGGSGGGGSGGGGSGGGGSGGGGSDKTHTCPPCPAPELLGGPS VFLFPPKPKDTLMISRTPEVTCVVVDVSHEDPEVKFNWYVDGVEVH NAKTKPCEEQYNSTYRCVSVLTVLHQDWLNGKEYKCKVSNKALPAP IEKTISKAKGQPREPQVYTLPPSREEMTKNQVSLTCLVKGFYPSDI AVEWESNGQPENNYKTTPPVLDSDGSFFLYSKLTVDKSRWQQGNVF SCSVMHEALHNHYTQKSLSLSPGK |
| 548 | | scFc-8 | | DKTHTCPPCPAPELLGGPSVFLFPPKPKDTLMISRTPEVTCVVVDV SHEDPEVKFNWYVDGVEVHNAKTKPCEEQYNSTYRCVSVLTVLHQD WLNGKEYKCKVSNKALPAPIEKTISKAKGQPREPQVYTLPPSREEM TKNQVSLTCLVKGFYPSDIAVEWESNGQPENNYKTTPPVLDSDGSF FLYSKLTVDKSRWQQGNVFSCSVMHEALHNHYTQKSLSLSPGGGGS GGGGSGGGGSGGGGSGGGGSGGGGSDKTHTCPPCPAPELLGGPSVF LFPPKPKDTLMISRTPEVTCVVVDVSHEDPEVKFNWYVDGVEVHNA KTKPCEEQYNSTYRCVSVLTVLHQDWLNGKEYKCKVSNKALPAPIE KTISKAKGQPREPQVYTLPPSREEMTKNQVSLTCLVKGFYPSDIAV EWESNGQPENNYKTTPPVLDSDGSFFLYSKLTVDKSRWQQGNVFSC SVMHEALHNHYTQKSLSLSP |
| | | | | |
| 549 | | (G₄S)₄ linker | | GGGGSGGGGSGGGGSGGGGS |
| 550 | | (G₄S)₅ linker | | GGGGSGGGGSGGGGSGGGGSGGGGS |
| 551 | | (G₄S)₆ linker | | GGGGSGGGGSGGGGSGGGGSGGGGSGGGGS |
| 552 | | (G₄S)₇ linker | | GGGGSGGGGSGGGGSGGGGSGGGGSGGGGSGGGGS |
| 553 | | (G₄S)₈ linker | | GGGGSGGGGSGGGGSGGGGSGGGGSGGGGSGGGGSGGGGS |
| | | | | |
| 554 | | DLL3-22 | bispecific molecule | QVQLQESGPGLVKPSETLSLTCTVSGDSISSYYWTWIRQPPGKGLE WIGYIYYSGTTNYNPSLKSRVTISVDTSKSQFSLKLSSVTAADTAV YYCASIAVRGFFFDYWGQGTLVTVSSGGGGSGGGGSGGGGSEIVLT QSPGTLSLSPGERATLSCRASQSVSSSYLAWYQQKPGQAPRLLIYG ASTRATGIPDRFSGSGSGTDFTLTISRLEPEDFAVYYCQQYGTSPL TFGGGTKVEIKRSGGGGSEVQLVESGGGLVQPGGSLKLSCAASGFT FNKYAMNWVRQAPGKGLEWVARIRSKYNNYATYYADSVKDRFTISR DDSKNTAYLQMNNLKTEDTAVYYCVRHGNFGNSYISYWAYWGQGTL VTVSSGGGGSGGGGSGGGGSQTVVTQEPSLTVSPGGTVTLTCGSST GAVTSGNYPNWVQQKPGQAPRGLIGGTKFLAPGTPARFSGSLLGGK AALTLSGVQPEDEAEYYCVLWYSNRWVFGGGTKLTVLHHHHHH |

## Claims

1. A method of treating DLL3-positive cancer, comprising administering to a subject in need thereof an anti-DLL3 agent comprising the amino acid sequence of SEQ ID NOs: 437 and 431, wherein the anti-DLL3 agent is administered at a dose of from 0.3 mg to 100 mg once every two weeks.

2. The method of claim 1, wherein the anti-DLL3 agent is administered at a dose of from 1 mg to 30 mg, from 1 mg to 20 mg, from 3 mg to 15 mg, or from 3 mg to 12 mg once every two weeks.

3. The method of claim 1 or 2, wherein the anti-DLL3 agent is administered at day 1 and day 15 of a 28-day cycle.

4. The method of any one of claims 1-3, wherein the anti-DLL3 agent is administered by intravenous (IV) infusion.

5. The method of any one of claims 1-4 further comprising administering an anti-inflammatory agent to the subject.

6. The method of claim 6, wherein the anti-inflammatory agent is a corticosteroid.

7. The method of any one of claims 1-6, wherein the cancer is small cell lung cancer (SCLC).

8. The method of any one of claims 1-6, wherein the cancer is relapsed/refractory SCLC (RR SCLC) or extensive disease SCLC (ED SCLC).

9. The method of any one of claims 1-8, wherein the anti-DLL3 agent comprises the amino acid sequence of SEQ ID NO: 438 or 520.

10. A method of treating DLL3-positive cancer, comprising administering to a subject in need thereof an anti-DLL3 agent comprising the amino acid sequence of SEQ ID NOs: 437 and 431, wherein said anti-DLL3 agent is administered according to the following schedule: a) a first dose of from 0.3 mg to 100 mg on day 1, b) a second dose of from 0.3 mg to 100 mg on day 8, and c) one or more subsequence doses of from 0.3 mg to 100 mg, starting on day 15 and once every two weeks thereafter, and wherein the second and subsequent doses are the same, and are higher than the first dose.

11. The method of claim 10, wherein the second and subsequent doses are at least 1.5-fold, at least 2-fold, at least 3-fold, at least 4-fold, at least 5-fold, at least 6-fold, at least 7-fold, at least 8-fold, at least 9-fold, or at least 10-fold, higher than the first dose.

12. The method of claim 10 or 11, wherein the first dose, the second dose, or the subsequence doses are from 1 mg to 30 mg, from 1 mg to 20 mg, from 3 mg to 15 mg, or from 3 mg to 12 mg.

13. The method of any one of claims 10-12, wherein the anti-DLL3 agent comprises the amino acid sequence of SEQ ID NO: 438 or 520.

14. The method of any one of claims 10-13, wherein the cancer is SCLC.

15. The method of any one of claims 10-14, wherein the cancer is RR SCLC or ED SCLC.

16. The method of any one of claims 10-15, wherein the anti-DLL3 agent is administered by IV infusion.

17. The method of any one of claims 10-16 further comprising administering an anti-inflammatory agent to the subject.

18. The method of any one of claims 1-17, wherein the subject is a human.
